# EUROPEAN PATENT SPECIFICATION

(11) **EP 3 439 702 B1**
(45) Date of publication and mention of the grant of the patent: **20.09.2023**
(21) Application number: 17719409.9
(22) Date of filing: 04.04.2017
(51) Int. Cl.: A61K 47/60, A61K 47/61

(54) **CONJUGATED C1 ESTERASE INHIBITOR AND USES THEREOF**
KONJUGIERTER C1-ESTERASE-INHIBITOR UND VERWENDUNGEN DAVON
INHIBITEUR DE C1 ESTÉRASE CONJUGUÉ ET SES UTILISATIONS

(30) Priority: 04.04.2016 US 201662318003 P
(43) Date of publication of application: 13.02.2019
(73) Proprietor: Takeda Pharmaceutical Company Limited, Osaka-shi, Osaka (JP)
(72) Inventor: HOLMES, Kevin, Lexington MA 02421 (US); NORTON, Angela, W., Lexington MA 02421 (US); PAN, Clark, Lexington MA 02421 (US)
(74) Representative: Carpmaels & Ransford LLP
(86) International application number: PCT/US2017/026001
(87) International publication number: WO 2017/176798

(56) References cited:
- WO-A1-2012/166622
- WO-A1-2013/138694

## Description

### BACKGROUND

C1-inhibitor (C1-INH), also known as C1 esterase inhibitor, is the largest member of the serpin protein superfamily. It is a heavily glycosylated serine proteinase inhibitor having the main function of inhibiting the spontaneous activation of the complement system. C1-INH regulates the complement cascade system, plays a key role in the regulation of the contact (kallikrein-kinin) amplification cascade, and participates in the regulation of the coagulation and fibrinolytic systems. Karnaukhova, E., C1-Esterase Inhibitor: Biological Activities and Therapeutic Applications. J Hematol Thromb Dis, 1: 113 (2013).

Dysfunction and/or deficiency of C1-INH in subjects has been correlated with a variety of autoimmune disease due to the failure of C1-INH to inhibit the activation of the complement system. An example of such a disease is hereditary angioedema (HAE), a rare, but potentially life-threatening disorder characterized by unpredictable and recurrent attacks of inflammation. Symptoms of HAE attacks include swelling of the face, mouth and/or airway that occur spontaneously or are triggered by mild trauma. Such swelling can also occur in any part of the body. In some cases, HAE is associated with low plasma levels of C1-inhibitor, while in other cases the protein circulates in normal or elevated amounts but it is dysfunctional. In addition to the episodes of inflammation, it also can cause more serious or life threatening indications, such as autoimmune diseases or lupus erythematosus.

CINRYZE^{®}, a human plasma derived C1 esterase inhibitor, has been approved for prophylactic use and treatment of acute attacks of HAE. Berinert^{®} (also a plasma-derived human C1-INH, CSL Behring) is indicated for treatment of acute HAE attack. Ruconest^{®} (conestat alfa, Pharming N.V.) is a recombinant C1-INH expressed in engineered rabbits is indicated for IV administration for treatment of acute HAE attack. Ruconest^{®} has the same amino acid sequence as human plasma derived C1-INH, but it is made in transgenic rabbits. Ruconest has an extremely short half-life of about 2.4-2.7 hours. *See* Ruconest^{®} FDA Label and Prescribing Information.
WO 2013/138694 A1 describes *"polymer conjugates containing a C1-inhibitor having at least one substantially non-antigenic polymer covalently attached to the C1-inhibitor via amino group of the C1 inhibitor"* (abstract).

There remains a need for improved C1 esterase inhibitors for the treatment and prophylaxis of various C1 esterase mediated indications.

### SUMMARY

The present invention relates to improved long-acting C1 esterase inhibitor that can be used to effectively treat various complement-mediated disorders including HAE.

The present invention provides a composition comprising a conjugated C1 esterase inhibitor (C1-INH) comprising:
(i) a C1-INH protein comprising at least one polyethylene glycol(PEG) moiety; and wherein the at least one PEG moiety is covalently linked to the C1-INH protein via an oxime linkage, wherein the oxime linkage is between the PEG moiety and a glycan residue of C1-INH, wherein the glycan residue is a sialic acid residue, and wherein the conjugated C1-INH has an extended *in vivo* half-life compared to unconjugated C1-INH; or
(ii) a C1-INH protein comprising at least one glycan residue; and at least one polysialic acid (PSA) moiety,
   wherein the at least one polysialic acid (PSA) moiety is covalently linked to the C1-INH protein via an oxime linkage, wherein the oxime linkage is between the PSA moiety and a glycan residue of C1-INH, wherein the glycan residue is a sialic acid residue, and wherein the conjugated C1-INH has an extended *in vivo* half-life compared to unconjugated C1-INH.

The present invention also provides a method of producing a conjugated C1 esterase inhibitor (C1-INH), said method comprising steps of:
(i) providing a C1-INH protein comprising at least one glycan residue; and providing a PEG moiety under conditions that permit the PEG moiety reacts with the at least one glycan residue to form an oxime linkage, thereby producing the conjugated C1-INH, wherein the at least one glycan residue is a sialic acid residue, and wherein the conjugated C1-INH has an extended *in vivo* half-life compared to unconjugated C1-INH; or
(ii) providing a C1-INH protein comprising at least one glycan residue; and providing a polysialic acid (PSA) moiety under conditions that permit the PSA moiety to react with the at least one glycan residue to form an oxime linkage, thereby producing the conjugated C1-INH,
wherein the at least one glycan residue is a sialic acid residue, and wherein the conjugated C1-INH has an extended *in vivo* half-life compared to unconjugated C1-INH.

The present invention also provides a conjugated C1 esterase inhibitor (C1-INH) produced by said method.

The present invention also provides a pharmaceutical composition comprising a conjugated C1 esterase inhibitor (C1-INH) as described above, and a pharmaceutically acceptable carri er.

The present invention also provides a kit comprising said pharmaceutical composition, and a syringe.

In particular, the present invention provides C1 esterase inhibitor conjugates (also referred to as "conjugated C1 esterase inhibitors") that exhibit comparable or even longer half-life than plasma derived C1-INH, as defined in the claims. The present invention is, in part, based on the surprising discovery that PEGylated and polysialylated C1-INH can have extended serum half-life of, e.g., at least 4 days. It is contemplated that long serum half-life of a conjugated C1-INH leads to superior *in vivo* efficacy and permits a preferable dosing regimen and route of administration. For example, the conjugated C1-INH described herein may be administered subcutaneously or intravenously with reduced frequency compared to currently approved C1-INH therapeutics, while still achieving desired efficacy (e.g., prophylaxis). The conjugated C1 inhibitor proteins described herein may be produced using plasma derived or recombinantly produced C1-INH. Therefore, conjugated C1-INH described herein can be manufactured in a cost-effective manner and not dependent on blood supply. Because they can be recombinantly produced in cultured cells, they offer more consistency in production and final product than those products purified from human blood, human blood components (e.g. plasma), or animal milk. Thus, the present invention provides conjugated C1 esterase inhibitors that are safer, more effective for treatment of HAE and other complement-mediated disorders.

In one aspect, the present invention provides a conjugated C1-INH comprising a C1-INH protein and at least one PEG moiety covalently linked to the C1-INH protein, as defined in the claims. The C1-INH protein comprises at least one glycan residue and the at least one PEG moiety is covalently linked to the at least one glycan residue. The at least one PEG moiety is covalently linked to the C1-INH protein via an oxime linkage.The at least one PEG moiety forms a covalent oxime link to a glycan residue. The glycan residue is a sialic acid residue of C1-INH.

In some embodiments, the C1-INH protein suitable for the present invention is recombinantly produced or plasma derived.

In some embodiments, the C1-INH protein includes a C1-INH domain that has an amino acid sequence at least 50% (e.g., at least 55%, 60%, 65%, 70%, 75%, 80%, 85%, 90%, 95%, 96%, 97%, 98%, 99%, or 100%) identical to SEQ ID NO:1, SEQ ID NO:2, SEQ ID NO:37, or SEQ ID NO:38.

In some embodiments, the C1-INH protein is a fusion protein. In some embodiments, the fusion protein includes an Fc domain directly or indirectly fused to a C1-INH domain. In some embodiments, the Fc domain is derived from IgG1. In some embodiments, the Fc domain comprises amino acid substitutions corresponding to L234A and L235A according to EU numbering. In some embodiments, the Fc domain comprises one or more amino acid substitutions at positions corresponding to Thr250, Met252, Ser254, Thr256, Thr307, Glu380, Met428, His433, and/or Asn434 of IgG1 according to EU numbering.

In some embodiments, the fusion protein includes an albumin domain directly or indirectly fused to a C1-INH domain.

In some embodiments, the present invention provides a C1-INH protein that has a glycosylation profile comprising no more than about 50% (e.g., no more than 45%, 40%, 35 %, 30%, 25%, 20%, 15 %, 10%, or 5%) neutral glycan species.

In some embodiments, the present invention provides a C1-INH protein that has a glycosylation profile comprising between about 5% and about 25% neutral glycan species.

In some embodiments, the present invention provides a C1-INH protein that comprises, on average, at least about 30% (e.g., at least 35%, 40%, 45%, 50%, 55%, 60%, 65%, 70%, 75%, 80%, 85%, 90%, 95%, 96%, 97%, 98%, 99%, or 100%) charged glycans per molecule.

In some embodiments, the C1-INH protein contains less than about 20% (e.g., less than 15%, 10%, or 5%) of one or more of mannose, α-galactose, NGNA, or oligomannose-type glycosylation.

In some embodiments, the C1-INH protein has a glycosylation profile comprising one or more of the following: between about 5% and about 30% neutral glycan species; between about 10% and about 30% mono-sialylated glycan species; between about 30% and about 50% di-sialylated glycan species; between about 15% and about 35% tri-sialylated glycan species; and/or between about 5% and about 15% tetra-sialylated glycan species.

In some embodiments, the C1-INH protein has a glycosylation profile comprising: no more than 30% neutral glycan species; between about 20% and about 30% mono-sialylated glycan species; between about 30% and about 40% di-sialylated glycan species; between about 10% and about 20% tri-sialylated glycan species; and, between about 5% and about 10% tetra-sialylated glycan species.

In some embodiments, the C1-INH protein comprises, on average, at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 sialylated glycan residues per molecule.

In some embodiments, the C1-inhibitor polypeptide comprises, on average, at least about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 mole sialic acid per mole of protein.

In some embodiments, a C1-INH protein with a glycosylation profile described herein is a fusion protein. In certain embodiments, a C1-INH protein with a glycosylation profile described herein is an unconjugated protein.

In some embodiments, a PEG conjugated to a C1-INH protein has a molecular weight between about 1 KDa and 50 KDa, between about 1 KDa and 40 KDa, between about 5 KDa and 40 KDa, between about 1 KDa and 30 KDa, between about 1 KDa and 25 KDa, between about 1 KDa and 20 KDa, between about 1 KDa and 15 KDa, between about 1 KDa and 10 KDa, or between about 1 KDa and 5 KDa. In some embodiments, a PEG conjugated to a C1-INH protein has a molecular weight of or greater than about 1 KDa, 2 KDa, 3 KDa, 4 KDa, 5 KDa, 10 KDa, 15 KDa, 20 KDa, 25 KDa, 30 KDa, 35 KDa, 40 KDa, 45 KDa, or 50 KDa. In some embodiments, a PEG conjugated to a C1-INH protein has linear or branched structures. In some embodiments, the branched PEG moiety can have 2, 3, 4, or 5 arm branches.

In some embodiments, the conjugated C1-INH has a PEG/C1-INH ration between about 1 to about 25, between about 1 to about 20, between about 1 to about 15, between 1 to about 10, or between about 1 to about 5.

In some embodiments, the conjugated C1-INH has a half-life comparable to or greater than a plasma-derived human C1-INH protein. In some embodiments, the half-life of the conjugated C1-INH is in the range of 100%-500% of the half-life of the plasma-derived C1-INH protein. In some embodiments, the conjugated C1-INH protein has a half-life of at least about 70, 75, 80, 85, 90, 95, 100, 105, 110, 115, 120, 125, 130, 135, 140, 145, 150, 155, 160, 165, or 170 hours.

In some embodiments, the conjugated C1-INH has a half-life of at least about 3, 4, 5, 6, 7, 8, 9, 10, 11, 12, 13, or 14 days.

In some embodiments, the conjugated C1-INH has a specific activity in the range of 50%-150% of the specific activity of plasma-derived human C1-INH protein.

In another aspect, the present invention provides a method of producing a conjugated C1 esterase inhibitor (C1-INH), comprising steps of providing a C1-INH protein comprising at least one glycan residue, and providing a PEG moiety under conditions that permit the PEG moiety to react with the at least one glycan residue to form a linkage, thereby producing the conjugated C1-INH, as defined in the claims.

In some embodiments, the PEG moiety comprises PEG-CH₂-O-NH₂. The at least one glycan residue is a sialic acid residue.

In some embodiments, the method described herein further includes a step of oxidizing the at least one sialic acid residue prior to reacting with the PEG moiety. In some embodiments, the oxidizing step comprises use of periodate oxidation. In some embodiments, the periodate oxidation is carried out with a molar ratio of periodate to C1-INH at between about 20:1 to about 50:1. In some embodiments, the molar ratio of periodate to PEG is between about 2.5 to about 40. In some embodiments, the molar ratio of PEG to C1-INH is between 25:1 and 100:1.

In further embodiments, the present method further comprises a step of purifying the conjugated C1-INH. In some embodiments, the purifying step includes one or more of anion exchange, tangential flow filtration, diafiltration, and dialysis.

In a further aspect, the present invention provides a pharmaceutical composition comprising a conjugated C1 esterase inhibitor (C1-INH), and a pharmaceutically acceptable carrier, as defined in the claims.

In some embodiments, the pharmaceutical composition comprising a conjugated C1-INH is liquid. In other embodiments, the pharmaceutical composition comprising a conjugated C1-INH is lyophilized.

In yet another aspect, the present invention provides a kit comprising a pharmaceutical composition comprising conjugated C1-INH (e.g., in a liquid and lyophilized form), as defined in the claims. The kit contains a syringe. In some embodiments, the syringe is preloaded with the pharmaceutical composition comprising conjugated C1-INH.

In some embodiments, wherein the pharmaceutical composition is lyophilized, the kit further comprises a reconstitution buffer.

In still another aspect, the present invention provides a pharmaceutical composition or kit for use in a method of treating a complement-mediated disorder, as defined in the claims.

In some embodiments, the complement-mediated disorder is selected from hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis, and/or multifocal motor neuropathy.

In another aspect, the present invention provides a composition comprising a conjugated C1 esterase inhibitor (C1-INH) comprising a C1-INH protein comprising at least one glycan residue; and at least one polysialic acid (PSA) moiety, as defined in the claims. The at least one polysialic acid (PSA) moiety is covalently linked to the C1-INH protein via an oxime linkage. The oxime linkage is between the PSA moiety and a glycan residue of C1-INH.The glycan residue is a sialic acid residue.

In some embodiments, the C1-INH protein is recombinantly produced or plasma derived.

In some embodiments, the C1-INH protein comprises a C1-INH domain having an amino acid sequence at least about 50%, 55%, 60%, 65%, 70%, 75%, 80%, 85%, 90%, 95%, 96%, 97%, 98%, 99%, or 100% identical to SEQ ID NO:1, SEQ ID NO:2, SEQ ID NO:37, or SEQ ID NO:38.

In some embodiments, the C1-INH protein is a fusion protein. In some embodiments, the fusion protein may comprise an Fc domain directly or indirectly fused to a C1-INH domain. In some embodiments, the Fc domain may be derived from IgG1. In some embodiments, the Fc domain may comprise amino acid substitutions corresponding to L234A and L235A according to EU numbering. In some embodiments, the fusion protein may comprise an albumin domain directly or indirectly fused to a C1-INH domain.

In some embodiments, the C1-INH protein has a glycosylation profile comprising no more than about 50%, 45%, 40%, 35 %, 30%, 25%, 20%, 15 %, 10%, or 5% neutral glycan species, prior to PEGylation.

In some embodiments, the C1-INH protein has a glycosylation profile comprising between about 5% and about 25% neutral glycan species, prior to PEGylation.

In some embodiments, the C1-INH protein comprises, on average, at least about 30%, 35%, 40%, 45%, 50%, 55%, 60%, 65%, 70%, 75%, 80%, 85%, 90%, 95%, 96%, 97%, 98%, 99%, or 100% charged glycans per molecule.

In some embodiments, the C1-INH protein contains less than about 20%, 15%, 10%, or 5% of one or more of mannose, α-galactose, NGNA, or oligomannose-type glycosylation, prior to conjugation with PSA.

In some embodiments, prior to conjugation with PSA, the C1-INH protein has a glycosylation profile comprising one or more of the following: between about 5% and about 30% neutral glycan species; between about 10% and about 30% mono-sialylated glycan species; between about 30% and about 50% di-sialylated glycan species; between about 15% and about 35% tri-sialylated glycan species; or between about 5% and about 15% tetra-sialylated glycan species.

In some embodiments, prior to conjugation with PSA, the C1-INH protein has a glycosylation profile comprising: no more than 30% neutral glycan species; between about 20% and about 30% mono-sialylated glycan species; between about 30% and about 40% di-sialylated glycan species; between about 10% and about 20% tri-sialylated glycan species; and between about 5% and about 10% tetra-sialylated glycan species.

In some embodiments, the C1-INH protein comprises, on average, at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 sialylated glycan residues per molecule.

In some embodiments, the C1-INH protein comprises, on average, at least about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 mole sialic acid per mole of protein.

In some embodiments, the PSA has a molecular weight between about 1 KDa and 50 KDa, between about 1 KDa and 40 KDa, between about 5 KDa and 40 KDa, between about 1 KDa and 30 KDa, between about 1 KDa and 25 KDa, between about 1 KDa and 20 KDa, between about 1 KDa and 15 KDa, between about 1 KDa and 10 KDa, or between about 1 KDa and 5 KDa.

In some embodiments, the PSA has a molecular weight of about 1 KDa, 5 KDa, 10 KDa, 15 KDa, 20 KDa, 25 KDa, 30 KDa, 35 KDa, 40 KDa, 45 KDa, or 50 KDa.

In some embodiments, the conjugated C1-INH has a PSA/C1-INH ratio of between about 1 to about 25, between about 1 to about 20, between about 1 to about 15, between about 1 to about 10, or between about 1 to about 5.

In some embodiments, the conjugated C1-INH has a half-life comparable or greater that than a plasma derived human C1-INH.

In some embodiments, the conjugated C1-INH has a half-life in the range of 100%-500% of the half-life of the plasma derived C1-INH.

In some embodiments, the conjugated C1-INH has a half-life of at least about 70, 75, 80, 85, 90, 95, 100, 105, 110, 115, 120, 125, 130, 135, 140, 145, 150, 155, 160, 165, or 170 hours.

In some embodiments, the conjugated C1-INH has a half-life of at least about 3, 4, 5, 6, 7, 8, 9, 10, 11, 12, 13, or 14 days.

In some embodiments, the conjugated C1-INH has a specific activity in the range of 50%-150% of the specific activity of plasma derived human C1-INH.

In a further aspect, the present invention provides a method of producing a conjugated C1 esterase inhibitor (C1-INH), the method comprising the steps of: providing a C1-INH protein comprising at least one glycan residue; and providing a polysialic acid (PSA) moiety under conditions that permit the PSA moiety to react with the at least one glycan residue to form a linkage, thereby producing the conjugated C1-INH, as defined in the claims. The at least one glycan residue is a sialic acid residue.

In some embodiments, the method further comprises a step of oxidizing the at least one sialic acid residue prior to reacting with the PSA moiety. In some embodiments, the oxidizing step comprises periodate oxidation. In some embodiments, the periodate oxidation may be carried out with a molar ratio of periodate to C1-INH at between about 20:1 to about 50:1. In some embodiments, the molar ratio of periodate to PSA may be between about 2.5 to about 40.

In some embodiments, the molar ratio of PSA to C1-INH is between about 25:1 and 100:1.

In some embodiments, the method further comprises a step of purifying the conjugated C1-INH.

In some embodiments, the purifying step comprises one or more of anion exchange, tangential flow filtration diafiltration, and dialysis.

In yet another aspect, the present invention provides conjugated C1 esterase inhibitor (C1-INH) produced by a method of an above aspect or embodiment.

In still another aspect, the present invention provides a pharmaceutical composition comprising a conjugated C1 esterase inhibitor (C1-INH) of an above aspect or embodiment and a pharmaceutically acceptable carrier, as defined in the claims. In some embodiments, the composition of the pharmaceutical composition is liquid. In some embodiments, the composition of the pharmaceutical composition is lyophilized.

In one aspect, the present invention provides a kit comprising a pharmaceutical composition of an above aspect or embodiment and a syringe, as deined in the claims. In some embodiments, the syringe is preloaded with the pharmaceutical composition. In some embodiments, the pharmaceutical composition is lyophilized and the kit further comprises a reconstitution buffer.

In another aspect, the present invention provides a pharmaceutical composition or kit for use in a method of treating a complement-mediated disorder comprising administering to a subject in need of treatment a pharmaceutical composition of an above aspect or embodiment, as defined in the claims. In some embodiments, the complement-mediated disorder is selected from hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis, multifocal motor neuropathy.

Other features, objects, and advantages of the present invention are apparent in the detailed description that follows. Various changes and modifications within the scope of the invention will become apparent to those skilled in the art from the detailed description.

### BRIEF DESCRIPTION OF THE DRAWINGS

The drawings are for illustration purposes only.
Figure 1 is a schematic representation of C1-INH. From right to left the three domains are the signal peptide, the N-terminus, also referred to as N-terminal domain, and the serpin domain. N-linked glycans are shown as long vertical lines with diamond heads and O-linked glycans are shown as short vertical lines.
Figure 2 depicts the mature C1-INH amino acid sequence and potential sites for PEGylation.
Figure 3 depicts a schematic of a chemical equation depicting an exemplary amine mediated PEGylation.
Figure 4A is a schematic of a chemical equation depicting an exemplary glycan mediated aminoxy PEGylation. Figure 4B is a schematic of a chemical equation depicting an exemplary sialic acid mediated (SAM) aminoxy PEGylation.
Figure 5 depicts a schematic of a chemical equation depicting an exemplary galactose mediated (GAM) PEGylation.
Figure 6, panels A and B, depicts the results of a preliminary rat study of C1-INH PEGylated (either 5 KDa or 40 KDa) via amino groups compared with sialic acid. rhC1-INH and Cinryze are provided as a comparator. Panel C depicts an SDS-PAGE gel of C1-INH PEGylated with either 5 KDa or 40 KDa PEG.
Figure 7 depicts a schematic of exemplary PEGylation process A.
Figure 8 depicts a schematic of exemplary PEGylation process B.
Figures 9A-E depict schematics summarizing several exemplary PEGylation protocols suitable for PEGylating C1-INH.
Figure 10A depicts the C1-INH-PEG IC50 of 5KSAM KHR5 octyl load samples. Figure 10B depicts the C1-INH-PEG IC50 before and after removal of free PEG by TFF.
Figure 11 depicts the chromatography results of an exemplary 40 KDa PEGylated C1-INH purification from free PEG and other contaminants.
Figure 12 depicts the chromatography results of an exemplary 20 KDa PEGylated C1-INH purification from free PEG and other contaminants.
Figure 13 depicts the chromatography results of an exemplary 5 KDa PEGylated C1-INH purification from free PEG and other contaminants.
Figure 14 depicts the results of a Non-Human Primate (NHP) PK Study of IV Administered PEGylated rhC 1 INH v. rhC 1 INH.
Figure 15 depicts the results of a NHP PK study in which varied C1-INH-PEG loads were administered to the NHP.
Figure 16 depicts the results of an IV v. SC NHP study of PEGylated rhC1-INH.
Figure 17 depicts the results of a rat PK titer analysis on C1-INH-PEG samples with varied 5KPEG loading.
Figure 18, panels A-E, depicts a series of gels and graphs that depict the purity of C1-INH-PEG. Panels A and B are barium-iodine stained SDS-PAGE gels used to detect free PEG in C1-INH-PEG samples. Panels C and D are RP-HPLC graphs that were used to detect free PEG 1K and 2K in C1-INH-PEG samples. Panel E depicts two SDS-PAGE gels loaded with C1-INH samples.
Figure 19, panels A-C, depicts a series of graphs and gels that depict purity, IC50, and PK data of C1-INH-PEG samples conjugated with SAM process. Panel A is an IC50 graph of various C1-INH samples. Panel B is an SDS-PAGE gel that depicts C1-INH sample purity and associated C1-INH sample IC50 values. Panel C is a graph that depicts PK values from a rat study in which the rats received intravenous C1-INH-PEG and non-PEGylated C1-INH.
Figure 20, panels A, B, and C, depicts a series of graphs that depict C1-INH IC50 values.
Figure 21 depicts a schematic for an exemplary amine coupling PEGylation process for C1-INH.
Figure 22, panels A-D, depicts a series of gels and graphs that depict the purity of C1-INH-PEG. Panel A depicts a barium iodine stained SDS-PAGE gel used to detect free PEG in C1-INH-PEG samples. Panel B depicts an RP-HPLC graph for the detection of free PEG 1K and 2K. Panels C and D depict purification chromatograms for free NHS-PEG20K (Panel C) and NHS-PEG40K (Panel D).
Figure 23, panels A-C, depicts a series of graphs and gels that depict purity, IC50, and PK data of CL-INH samples. Panel A is an IC50 graph of various C1-INH samples. Panel B is a graph that depicts PK values from a rat study in which the rats received intravenous C1-INH-PEG and non-PEGylated C1-INH. Panel C is an SDS-PAGE gel that depicts C1-INH sample purity and associated C1-INH sample IC50 values.
Figure 24, panels A and B, depicts a gel and a graph that depicts the purity of C1-INH-PSA produced with the sialic acid mediated (SAM) process. Panel A is an SDS gel, and Panel B is an IC50 graph of C1-INH-PSA.
Figure 25, panels A, B, and C, depicts a series of graphs that show PK values from a rat study in which the rats received intravenous C1-INH-PEG, C1-INH-PSA, Cinryze-PEG, C1-INH, or Cinryze.

### DEFINITIONS

In order for the present invention to be more readily understood, certain terms are first defined below. Additional definitions for the following terms and other terms are set forth throughout the specification.

*Animal*: As used herein, the term "animal" refers to any member of the animal kingdom. "Animal" may refer to humans, at any stage of development. "Animal" may refer to non-human animals, at any stage of development. The non-human animal can be a mammal (*e.g*., a rodent, a mouse, a rat, a rabbit, a monkey, a dog, a cat, a sheep, cattle, a primate, and/or a pig). Animals include mammals, birds, reptiles, amphibians, fish, insects, and/or worms. An animal may be a transgenic animal, genetically-engineered animal, and/or a clone.

*Approximately or about*: As used in this application, the terms "about" and "approximately" are used as equivalents. Any numerals used in this application with or without about/approximately are meant to cover any normal fluctuations appreciated by one of ordinary skill in the relevant art. As used herein, the term "approximately" or "about," as applied to one or more values of interest, refers to a value that is similar to a stated reference value. The term "approximately" or "about" may refer to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Bioavailability*: As used herein, the term "bioavailability" generally refers to the percentage of the administered dose that reaches the blood stream of a subject.

*Biologically active*: As used herein, the phrase "biologically active" refers to a characteristic of any agent that has activity in a biological system, and particularly in an organism. For instance, an agent that, when administered to an organism, has a biological effect on that organism, is considered to be biologically active. Where a peptide is biologically active, a portion of that peptide that shares at least one biological activity of the peptide may be typically referred to as a "biologically active" portion.

*Carrier or diluent*: As used herein, the terms "carrier" or "diluent" refers to a pharmaceutically acceptable (e.g., safe and non-toxic for administration to a human) carrier or diluting substance useful for the preparation of a pharmaceutical formulation. Exemplary diluents include sterile water, bacteriostatic water for injection (BWFI), a pH buffered solution (e.g. phosphate-buffered saline), sterile saline solution, Ringer's solution or dextrose solution.

*C1-inhibitor or C1 esterase inhibitor or C1-INH*: As used herein, the term "C1-inhibitor" or "C1 esterase inhibitor" or "C1-INH" can all be used interchangeably and refer to any wild-type, native, naturally occurring, recombinant produced, and/or modified C1-INH proteins (e.g., C1-INH proteins with one or more amino acid mutations, deletions, truncations, insertions, and/or fusion proteins) that retain substantial C1-INH biological activity unless otherwise specified. A "C1-inhibitor" or "C1 esterase inhibitor" or "C1-INH" may be a fusion protein. A C1-INH fusion protein may comprise a C1-INH polypeptide or domain and an Fc domain. A C1-INH fusion protein may comprise a C1-INH polypeptide or domain and an albumin domain. The fusion protein may further comprise a linker. A C1-INH protein may be recombinantly expressed in recombinant cells. The C1-INH may be expressed in mammalian cells, preferably CHO cells, or human cells, preferably HT1080 or HEK cells.

*Conjugate*: As used herein, the term "conjugate" may refer to a moiety covalently attached to a protein directly or indirectly. Typically, where a protein is attached to a conjugate, it may be referred to as a conjugated protein or protein conjugate. A conjugate described herein may be polyethylene glycol (PEG). Where a protein is attached to a PEG moiety, it may be referred to as a PEGylated protein.

*Functional equivalent or derivative*: As used herein, the term "functional equivalent" or "functional derivative" denotes, in the context of a functional derivative of an amino acid sequence, a molecule that retains a biological activity (either function or structural) that is substantially similar to that of the original sequence. A functional derivative or equivalent may be a natural derivative or is prepared synthetically. Exemplary functional derivatives include amino acid sequences having substitutions, deletions, or additions of one or more amino acids, provided that the biological activity of the protein is conserved. The substituting amino acid desirably has chemico-physical properties which are similar to that of the substituted amino acid. Desirable similar chemico-physical properties include, similarities in charge, bulkiness, hydrophobicity, hydrophilicity, and the like.

*Fusion protein*: As used herein, the term "fusion protein" or "chimeric protein" refers to a protein created through the joining of two or more originally separate proteins, or portions thereof. A linker or spacer may be present between each protein.

*Half Life:* As used herein, the term "half-life" is the time required for a quantity such as protein concentration or activity to fall to half of its value as measured at the beginning of a time period.

*Hereditary angioedema or HAE*: As used herein, the term "hereditary angioedema" or "HAE" refers to a blood disorder characterized by unpredictable and recurrent attacks of inflammation. HAE is typically associated with C1-INH deficiency, which may be the result of low levels of C1-INH or C1-INH with impaired or decreased activity. Symptoms include swelling that can occur in any part of the body, such as the face, extremities, genitals, gastrointestinal tract and upper airways.

*Improve, increase, or reduce*: As used herein, the terms "improve," "increase" or "reduce," or grammatical equivalents, indicate values that are relative to a baseline measurement, such as a measurement in the same individual prior to initiation of the treatment described herein, or a measurement in a control subject (or multiple control subject) in the absence of the treatment described herein. A "control subject" is a subject afflicted with the same form of disease as the subject being treated, who is about the same age as the subject being treated.

*In Vitro*: As used herein, the term *"in vitro"* refers to events that occur in an artificial environment, *e.g.,* in a test tube or reaction vessel, in cell culture, *etc.,* rather than within a multi-cellular organism.

*In Vivo*: As used herein, the term *"in vivo"* refers to events that occur within a multi-cellular organism, such as a human and a non-human animal. In the context of cell-based systems, the term may be used to refer to events that occur within a living cell (as opposed to, for example, *in vitro* systems).

*Linker*: As used herein, the term "linker" refers to, in a fusion protein, an amino acid sequence other than that appearing at a particular position in the natural protein and is generally designed to be flexible or to interpose a structure, such as an α-helix, between two protein moieties. A linker is also referred to as a spacer. A linker or a spacer typically does not have biological function on its own.

*Polypeptide*: The term "polypeptide" as used herein refers to a sequential chain of amino acids linked together via peptide bonds. As is known to those skilled in the art, polypeptides may be processed and/or modified. As used herein, the terms "polypeptide" and "peptide" are used inter-changeably.

*Prevent:* As used herein, the term "prevent" or "prevention", when used in connection with the occurrence of a disease, disorder, and/or condition, refers to reducing the risk of developing the disease, disorder and/or condition. See the definition of "risk."

*Protein*: The term "protein" as used herein refers to one or more polypeptides that function as a discrete unit. If a single polypeptide is the discrete functioning unit and does not require permanent or temporary physical association with other polypeptides in order to form the discrete functioning unit, the terms "polypeptide" and "protein" may be used interchangeably. If the discrete functional unit is comprised of more than one polypeptide that physically associate with one another, the term "protein" refers to the multiple polypeptides that are physically coupled and function together as the discrete unit.

*Risk*: As will be understood from context, a "risk" of a disease, disorder, and/or condition comprises a likelihood that a particular individual will develop a disease, disorder, and/or condition (e.g., muscular dystrophy). Risk may be expressed as a percentage. Risk may be from 0,1, 2, 3, 4, 5, 6, 7, 8, 9, 10, 20, 30, 40, 50, 60, 70, 80, 90 up to 100%. Risk may be expressed as a risk relative to a risk associated with a reference sample or group of reference samples. A reference sample or group of reference samples may have a known risk of a disease, disorder, condition and/or event (e.g., muscular dystrophy). A reference sample or group of reference samples maybe from individuals comparable to a particular individual. Relative risk may be 0,1, 2, 3, 4, 5, 6, 7, 8, 9, 10, or more.

*Subject*: As used herein, the term "subject" refers to a human or any non-human animal (e.g., mouse, rat, rabbit, dog, cat, cattle, swine, sheep, horse or primate). A human includes pre- and post-natal forms. A subject may be a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

*Substantially*: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Substantial homology*: The phrase "substantial homology" is used herein to refer to a comparison between amino acid or nucleic acid sequences. As will be appreciated by those of ordinary skill in the art, two sequences are generally considered to be "substantially homologous" if they contain homologous residues in corresponding positions. Homologous residues may be identical residues. Alternatively, homologous residues may be non-identical residues will appropriately similar structural and/or functional characteristics. For example, as is well known by those of ordinary skill in the art, certain amino acids are typically classified as "hydrophobic" or "hydrophilic" amino acids, and/or as having "polar" or "non-polar" side chains Substitution of one amino acid for another of the same type may often be considered a "homologous" substitution.

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology; Altschul, et al., "Gapped BLAST and PSI-BLAST: a new generation of protein database search programs", Nucleic Acids Res. 25:3389-3402, 1997; Baxevanis, et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying homologous sequences, the programs mentioned above typically provide an indication of the degree of homology. Two sequences may be considered to be substantially homologous if at least 50%, 55%, 60%, 65%, 70%, 75%, 80%, 85%, 90%, 91%, 92%, 93%, 94%, 95%, 96%, 97%, 98%, 99% or more of their corresponding residues are homologous over a relevant stretch of residues. The relevant stretch may be a complete sequence. The relevant stretch may be at least 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 or more residues.

*Substantial identity:* The phrase "substantial identity" is used herein to refer to a comparison between amino acid or nucleic acid sequences. As will be appreciated by those of ordinary skill in the art, two sequences are generally considered to be "substantially identical" if they contain identical residues in corresponding positions. As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology*;* Altschul et al., Nucleic Acids Res. 25:3389-3402, 1997; Baxevanis et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying identical sequences, the programs mentioned above typically provide an indication of the degree of identity. Two sequences may be considered to be substantially identical if at least 50%, 55%, 60%, 65%, 70%, 75%, 80%, 85%, 90%, 91%, 92%, 93%, 94%, 95%, 96%, 97%, 98%, 99% or more of their corresponding residues are identical over a relevant stretch of residues. The relevant stretch may be a complete sequence. The relevant stretch may be at least 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 or more residues.

*Suffering from:* An individual who is "suffering from" a disease, disorder, and/or condition has been diagnosed with or displays one or more symptoms of the disease, disorder, and/or condition.

*Susceptible to*: An individual who is "susceptible to" a disease, disorder, and/or condition has not been diagnosed with the disease, disorder, and/or condition. An individual who is susceptible to a disease, disorder, and/or condition may not exhibit symptoms of the disease, disorder, and/or condition. An individual who is susceptible to a disease, disorder, condition, or event (for example, DMD) may be characterized by one or more of the following: (1) a genetic mutation associated with development of the disease, disorder, and/or condition; (2) a genetic polymorphism associated with development of the disease, disorder, and/or condition; (3) increased and/or decreased expression and/or activity of a protein associated with the disease, disorder, and/or condition; (4) habits and/or lifestyles associated with development of the disease, disorder, condition, and/or event (5) having undergone, planning to undergo, or requiring a transplant. An individual who is susceptible to a disease, disorder, and/or condition may develop the disease, disorder, and/or condition. An individual who is susceptible to a disease, disorder, and/or condition may not develop the disease, disorder, and/or condition.

*Therapeutically effective amount:* As used herein, the term "therapeutically effective amount" of a therapeutic agent means an amount that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by those of ordinary skill in the art that a therapeutically effective amount is typically administered via a dosing regimen comprising at least one unit dose.

*Treating*: As used herein, the term "treat," "treatment," or "treating" refers to any method used to partially or completely alleviate, ameliorate, relieve, inhibit, prevent, delay onset of, reduce severity of and/or reduce incidence of one or more symptoms or features of a particular disease, disorder, and/or condition. Treatment may be administered to a subject who does not exhibit signs of a disease and/or exhibits only early signs of the disease for the purpose of decreasing the risk of developing pathology associated with the disease.

### DETAILED DESCRIPTION OF CERTAIN EMBODIMENTS

The present invention provides, among other things, a conjugated C1-INH for improved treatment of complement-mediated disorders, including hereditary angioedema (HAE), as defined in the claims.

It is contemplated that a conjugated C1-INH (e.g., a PEGylated C1-INH, or a polysialic acid (PSA) conjugated C1-INH) has extended half-life compared to unconjugated (e.g., un-PEGylated) but otherwise identical C1-INH. According to the present invention, any C1-INH proteins may be conjugated (e.g., PEGylated, or PSA conjugated) including, plasma-derived or recombinantly expressed C1-INH proteins. In some embodiments, a C1-INH protein that may be conjugated (e.g., PEGylated, or PSA conjugated) is a fusion protein. As described below, the result of conjugation (e.g., PEGylation, or PSA conjugated) according to the present invention extends *in vivo* half-life while retaining unexpectedly good bioavailability and/or bioactivity of the C1-INH protein. Therefore, conjugated (e.g., PEGylated, or PSA conjugated) C1-INH provided herein permits improved treatment of HAE and other complement-mediated diseases, disorders or conditions by, e.g., reducing dosing frequency and increasing prophylactic efficacy.

Various aspects of the invention are described in detail in the following sections. In this application, the use of "or" means "and/or" unless stated otherwise.

### C1-INH Proteins

The present invention may be used to conjugate any C1-INH proteins. Human C1-INH is an important anti-inflammatory plasma protein with a wide range of inhibitory and non-inhibitory biological activities. By sequence homology, structure of its C-terminal domain, and mechanism of protease inhibition, it belongs to the serpin superfamily, the largest class of plasma protease inhibitors, which also includes antithrombin, α1-proteinase inhibitor, plasminogen activator inhibitor, and many other structurally similar proteins that regulate diverse physiological systems. C1-INH is an inhibitor of proteases in the complement system, the contact system of kinin generation, and the intrinsic coagulation pathway. Cai, S. & Davis, A. E., Complement Regulatory Protein C1 Inhibitor Binds to Selectins and Interferes with Endothelial-Leukocyte Adhesion, J Immunol, 171:4786-4791 (2003). Specifically, C1-INH has been shown to inhibit C1r and C1s of the complement system. C1-INH is also a major regulator of coagulation factors XI and XII, as well as kallikrein and other serine proteases of the coagulation and fibrinolytic systems including tissue type plasminogen activator and plasmin.

Low plasma content of C1-INH or its dysfunction result in the activation of both complement and contact plasma cascades, and may affect other systems as well. A decrease in C1-INH plasma content to levels lower than 55 µg/mL (~25% of normal) has been shown to induce spontaneous activation of C1.

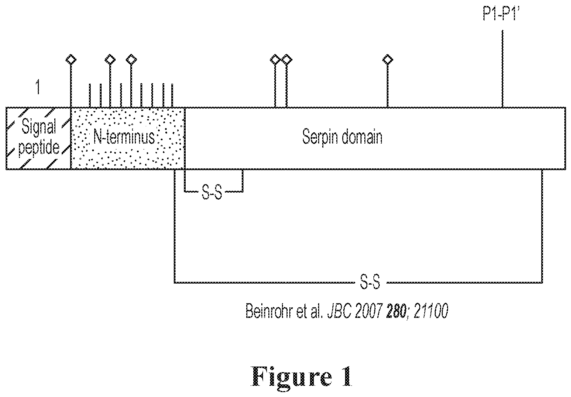

A schematic depicting the structure of C1-INH is provided in Figure 1. The signal peptide, N-terminal domain, and serpin domain are shown. C1-INH is The 22 amino acid signal peptide is required for secretion and cleaved from the rest of the C1-INH protein. C1-INH has two domains: a C-terminal domain having 365 amino acids, which is a typical serpin domain, and an N-terminal domain having 113 amino acids. The protein is stabilized by two disulfide bridges which connect the domains. These disulfide bridges are formed by Cys101 of the N-terminal domain which forms a disulfide bond with Cys406 of the C-terminal (serpin) domain and Cys108 of the N-terminal domain which forms a disulfide bond with Cys183 of C-terminal domain. The serpin domain is responsible for the protease activity of C1-INH. P1-P1' denotes the Arg444-Thr445 scissile bond.

More than 26% of the weight of the glycosylated protein is carbohydrate. The glycans are unevenly distributed over human C1-INH. The N-terminus is heavily glycosylated, having three N-linked (shown as long vertical lines with diamond heads) and at least seven O-linked (shown as short vertical lines) carbohydrate groups. Three N-attached glycans are attached to asparagine residues Asn216, Asn231, and Asn330 in the serpin domain (shown as long vertical lines with diamond heads). Although the functional role of the exceptionally long and heavily glycosylated N-terminal domain is still unclear, it may be essential for the protein's conformational stability, recognition, affinity to endotoxins and selectins, and clearance. The intrinsic heterogeneity of the carbohydrate moiety greatly contributes to the heterogeneity of the whole C1-INH, one of the reasons why production of a recombinant C1-INH mimicking the properties of plasma-derived C1-INH is difficult.

As used herein, C1-INH proteins suitable for conjugation and use according to the present invention comprise a C1-INH polypeptide or domain with wild-type or modified amino acid sequences (e.g., C1-INH proteins with amino acid mutations, deletions, truncations, and/or insertions) that retain substantial C1-INH biological activity. Typically, a C1-INH protein is produced using recombinant technology, but may also be plasma-derived.

In some embodiments, a C1-INH polypeptide or domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical or homologous to the wild-type human C1-INH protein (amino acids 1-478) (amino acids 1-97 are underlined):

NPNATSSSSQDPESLQDRGEGKVATTVISKMLFVEPILEVSSLPTTNSTTNSATKITAN
TTDEPTTQPTTEPTTQPTIQPTQPTTQLPTDSPTQPTTGSFCPGPVTLCSDLESHSTEAV
LGDALVDFSLKLYHAFSAMKKVETNMAFSPFSIASLLTQVLLGAGENTKTNLESILSY
PKDFTCVHQALKGFTTKGVTSVSQIFHSPDLAIRDTFVNASRTLYSSSPRVLSNNSDA
NLELINTWVAKNTNNKISRLLDSLPSDTRLVLLNAIYLSAKWKTTFDPKKTRMEPFHF
KNSVIKVPMMNSKKYPVAHFIDQTLKAKVGQLQLSHNLSLVILVPQNLKHRLEDME
QALSPSVFKAIMEKLEMSKFQPTLLTLPRIKVTTSQDMLSIMEKLEFFDFSYDLNLCG
LTEDPDLQVSAMQHQTVLELTETGVEAAAASAISVARTLLVFEVQQPFLFVLWDQQ
HKFPVFMGRVYDPRA (SEQ ID NO:1).

In some embodiments, a C1-INH polypeptide or domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical or homologous to the mature wild-type human C1-INH protein (amino acids 98-478):

GSFCPGPVTLCSDLESHSTEAVLGDALVDFSLKLYHAFSAMKKVETNMAFSPFSIASL
LTQVLLGAGENTKTNLESILSYPKDFTCVHQALKGFTTKGVTSVSQIFHSPDLAIRDTF
VNASRTLYSSSPRVLSNNSDANLELINTWVAKNTNNKISRLLDSLPSDTRLVLLNAIY
LSAKWKTTFDPKKTRMEPFHFKNSVIKVPMMNSKKYPVAHFIDQTLKAKVGQLQLS
HNLSLVILVPQNLKHRLEDMEQALSPSVFKAIMEKLEMSKFQPTLLTLPRIKVTTSQD
MLSIMEKLEFFDFSYDLNLCGLTEDPDLQVSAMQHQTVLELTETGVEAAAASAISVA
RTLLVFEVQQPFLFVLWDQQHKFPVFMGRVYDPRA (SEQ ID NO:2).

In some embodiments, a C1-INH polypeptide or domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical or homologous to a human C1-INH protein (amino acids 1-478) having an E165Q mutation (mutated amino acid bolded and underlined):

NPNATSSSSQDPESLQDRGEGKVATTVISKMLFVEPILEVSSLPTTNSTTNSATKITAN
TTDEPTTQPTTEPTTQPTIQPTQPTTQLPTDSPTQPTTGSFCPGPVTLCSDLESHSTEAV
LGDALVDFSLKLYHAFSAMKKVETNMAFSPFSIASLLTQVLLGAGENTKTNLESILS
YPKDFTCVHQALKGFTTKGVTSVSQIFHSPDLAIRDTFVNASRTLYSSSPRVLSNNSD
ANLELINTWVAKNTNNKISRLLDSLPSDTRLVLLNAIYLSAKWKTTFDPKKTRMEPF
HFKNSVIKVPMMNSKKYPVAHFIDQTLKAKVGQLQLSHNLSLVILVPQNLKHRLED
MEQALSPSVFKAIMEKLEMSKFQPTLLTLPRIKVTTSQDMLSIMEKLEFFDFSYDLNL
CGLTEDPDLQVSAMQHQTVLELTETGVEAAAASAISVARTLLVFEVQQPFLFVLWD
QQHKFPVFMGRVYDPRA (SEQ ID NO:37).

In some embodiments, a C1-INH polypeptide or domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical or homologous to a mature human C1-INH protein (amino acids 98-478) having an E165Q mutation (mutated amino acid bolded and underlined):

GSFCPGPVTLCSDLESHSTEAVLGDALVDFSLKLYHAFSAMKKVETNMAFSPFSIASL
LTQVLLGAGENTKTNLESILSYPKDFTCVHQALKGFTTKGVTSVSQIFHSPDLAIRDT
FVNASRTLYSSSPRVLSNNSDANLELINTWVAKNTNNKISRLLDSLPSDTRLVLLNAI
YLSAKWKTTFDPKKTRMEPFHFKNSVIKVPMMNSKKYPVAHFIDQTLKAKVGQLQL
SHNLSLVILVPQNLKHRLEDMEQALSPSVFKAIMEKLEMSKFQPTLLTLPRIKVTTSQ
DMLSIMEKLEFFDFSYDLNLCGLTEDPDLQVSAMQHQTVLELTETGVEAAAASAISV
ARTLLVFEVQQPFLFVLWDQQHKFPVFMGRVYDPRA (SEQ ID NO:38).

Homologues or analogues of human C1-INH proteins can be prepared according to methods for altering polypeptide sequence known to one of ordinary skill in the art such as are found in references that compile such methods. As will be appreciated by those of ordinary skill in the art, two sequences are generally considered to be "substantially homologous" if they contain homologous residues in corresponding positions. Homologous residues may be identical residues. Alternatively, homologous residues may be non-identical residues will appropriately similar structural and/or functional characteristics. For example, as is well known by those of ordinary skill in the art, certain amino acids are typically classified as "hydrophobic" or "hydrophilic" amino acids, and/or as having "polar" or "non-polar" side chains. Substitution of one amino acid for another of the same type may often be considered a "homologous" substitution. Conservative substitutions of amino acids include substitutions made among amino acids within the following groups: (a) M, I, L, V; (b) F, Y, W; (c) K, R, H; (d) A, G; (e) S, T; (f) Q, N; and (g) E, D. A "conservative amino acid substitution" may refer to an amino acid substitution that does not alter the relative charge or size characteristics of the protein in which the amino acid substitution is made.

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology*;* Altschul, et al., "Gapped BLAST and PSI-BLAST: a new generation of protein database search programs", Nucleic Acids Res. 25:3389-3402, 1997; Baxevanis, et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying homologous sequences, the programs mentioned above typically provide an indication of the degree of homology.

In some embodiments, a C1-INH polypeptide or domain suitable for the present invention may be a truncated C1-INH protein. For example, a C1-INH polypeptide or domain suitable for the present invention includes a portion or a fragment of any of SEQ ID NO:1, SEQ ID NO:2, SEQ ID NO:37 or SEQ ID NO:38..

### C1-INH Fusion Proteins

In some embodiments, C1-INH proteins that can be conjugated according to the present invention include C1-INH fusion proteins, as defined in the claims. A C1-INH fusion protein may include a C1-INH domain (also referred to as a C1-INH polypeptide) and another domain or moiety that typically can facilitate a therapeutic effect of C1-INH by, for example, enhancing or increasing half-life, stability, potency, and/or delivery of C1-INH protein, or reducing or eliminating immunogenicity, clearance, or toxicity. Such suitable domains or moieties for a C1-INH fusion protein include Fc domains and albumin domains. A suitable fusion domain or moiety (e.g., a Fc or albumin domain) may be directly or indirectly linked, fused or attached to the N-terminus, C-terminus or internally to a C1-INH protein. The following sections describe exemplary C1-INH fusion proteins that may be conjugated.

### Fc Domains

In some embodiments, a suitable C1-INH fusion protein contains an Fc domain or a portion thereof that binds to the FcRn receptor. A suitable Fc domain may be derived from an immunoglobulin subclass such as IgG. In some embodiments, a suitable Fc domain is derived from IgG1, IgG2, IgG3, or IgG4. In some embodiments, a suitable Fc domain is derived from IgM, IgA, IgD, or IgE. Particularly suitable Fc domains include those derived from human or humanized antibodies. In some embodiments, a suitable Fc domain is a modified Fc portion, such as a modified human Fc portion.

C1-inhibitor Fc fusion proteins may exist as dimers, as shown in FIG. 1.

In some embodiments, an Fc domain suitable for the present invention may include an amino acid sequence at least 50% (e.g., at least 55%, 60%, 65%, 70%, 75%, 80%, 85%, 90%, 95%, 96%, 97%, 98%, 99%, or 100%) identical to the wild-type human IgG1 Fc domain:

DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPG
K (SEQ ID NO:3).

In some embodiments, a suitable Fc domain may include one or more mutations that reduce or eliminate complement activation and/or antibody-dependent cell-mediated cytotoxicity (ADCC) activity (also referred to as "effector function"). For example, suitable Fc domains may include mutations corresponding to L234A and L235A (LALA) of IgG1, according to EU numbering. An exemplary human IgG1 Fc domain having a LALA mutation (mutated residues underlined) is shown below: 
DKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSP
GK (SEQ ID NO:4).
 In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to SEQ ID NO:4 while maintaining mutations corresponding to L234A and L235A (LALA) of IgG1, according to EU numbering.

It is contemplated that improved binding between Fc domain and the FcRn receptor results in prolonged serum half-life. Thus, in some embodiments, a suitable Fc domain comprises one or more amino acid mutations that lead to improved binding to FcRn. Various mutations within the Fc domain that effect improved binding to FcRn are known in the art and can be adapted to practice the present invention. In some embodiments, a suitable Fc domain comprises one or more mutations at one or more positions corresponding to Thr 250, Met 252, Ser 254, Thr 256, Thr 307, Glu 380, Met 428, His 433, and/or Asn 434 of human IgG1, according to EU numbering.

For example, a suitable Fc domain may contain mutations of H433K (His433Lys) and/or N434F (Asn434Phe). A suitable Fc domain may contain mutations H433K (His433Lys) and N434F (Asn434Phe). Additional amino acid substitutions that can be included in a Fc domain include those described in, e.g., U.S. Patent Nos. 6,277,375; 8,012,476; and 8,163,881.

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain while maintaining one or more mutations corresponding to Thr 250, Met 252, Ser 254, Thr 256, Thr 307, Glu 380, Met 428, His 433, and/or Asn 434 of human IgG1, according to EU numbering (underlined below):

DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALKFHYTQKSLSLSPGK
(SEQ ID NO:5).

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain while maintaining mutations corresponding to L234A and L235A (LALA) of IgG1, and one or more mutations corresponding to Thr 250, Met 252, Ser 254, Thr 256, Thr 307, Glu 380, Met 428, His 433, and/or Asn 434 of human IgG1, according to EU numbering (mutated residues underlined):

DKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALKFHYTQKSLSLSPG
K (SEQ ID NO:6).

In some embodiments, an Fc domain derived from IgG4 is used for the present invention. Without wishing to be bound by any theory, IgG4 is reported to have lower complement activation than WT IgG1. Thus, in some embodiments, a wild-type human IgG4 Fc domain is used in the present invention. In some embodiments, an Fc domain suitable for the present invention is derived from human IgG4 with a mutation corresponding to an S228P substitution in the core hinge region sequence according to the EU index. This substitution has also been referred to as S241P according to Kabat et al (1987 Sequences of proteins of immunological interest. United States Department of Health and Human Services, Washington DC.). Without wishing to be bound by any theory, it is contemplated that this substitution has the effect of making the sequence of the core of the hinge region the same as that of a Wild-type IgGl or IgG2 isotype antibody and results in the production of the homogenous form of the IgG4 antibody and hence abrogates the dissociation and reassociation of the heavy chains which often leads to the production of heterodimeric IgG4 antibodies. In addition, IgG4 derived Fc domains may be used for stability at high concentrations.

Thus, in some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the wild-type human IgG4 Fc domain:

ESKYGPPCPSCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSS
IEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSL
GK (SEQ ID NO:9).

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the human IgG4 Fc domain while maintaining a mutation corresponding to an S241P substitution according to EU numbering (mutated residue underlined):

ESKYGPPCPPCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSS
IEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSL
GK (SEQ ID NO: 10).

In some embodiments, an Fc domain described herein may include a signal peptide. An exemplary signal peptide suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to METPAQLLFLLLLWLPDTTG.

For example, a suitable Fc domain may have an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain with a signal peptide, and having mutations that enhance the binding to the FcRn receptor (signal peptide and mutated residues underlined):

METPAQLLFLLLLWLPDTTGDKTHTCPPCPAPELLGGPSVFLFPPKPKDTLMISRTPE
VTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVFSCS
VMHEALKFHYTQKSLSLSPGK (SEQ ID NO:7).

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain with a signal peptide, and having both LALA and mutations that enhance the binding to the FcRn receptor (mutated residues underlined):

METPAQLLFLLLLWLPDTTGDKTHTCPPCPAPEAAGGPSVFLFPPKPKDTLMISRTPE
VTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVFSCS
VMHEALKFHYTQKSLSLSPGK (SEQ ID NO:8).

### Exemplary C1-INH-Fc Fusion Proteins

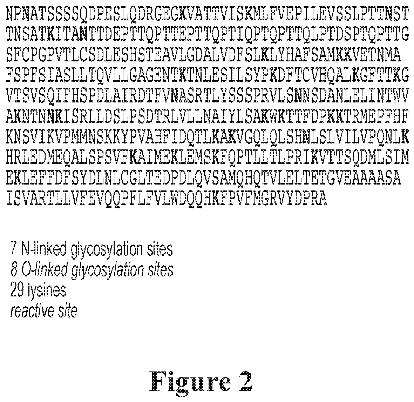

In particular embodiments, a suitable C1-INH fusion protein includes a C1-INH polypeptide or domain and an Fc domain. In some embodiments, a suitable C1-INH fusion protein includes a linker that associates the C1-INH polypeptide or domain with the Fc domain. In certain embodiments, as shown in FIG. 2, Fc moieties may be directly fused to the N-terminal region of the full length (1-478 aa) as well as mature (98-478) C1-inhibitor. Suitable C1-INH Fc fusion proteins may have an amino acid sequence shown below: 
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPG
KNPNATSSSSQDPESLQDRGEGKVATTVISKMLFVEPILEVSSLPTTNSTTNSATKITA
NTTDEPTTQPTTEPTTQPTIQPTQPTTQLPTDSPTQPTTGSFCPGPVTLCSDLESHSTEA
VLGDALVDFSLKLYHAFSAMKKVETNMAFSPFSIASLLTQVLLGAGENTKTNLESILS
YPKDFTCVHQALKGFTTKGVTSVSQIFHSPDLAIRDTFVNASRTLYSSSPRVLSNNSD
ANLELINTWVAKNTNNKISRLLDSLPSDTRLVLLNAIYLSAKWKTTFDPKKTRMEPF
HFKNSVIKVPMMNSKKYPVAHFIDQTLKAKVGQLQLSHNLSLVILVPQNLKHRLED
MEQALSPSVFKAIMEKLEMSKFQPTLLTLPRIKVTTSQDMLSIMEKLEFFDFSYDLNL
CGLTEDPDLQVSAMQHQTVLELTETGVEAAAASAISVARTLLVFEVQQPFLFVLWD
QQHKFPVFMGRVYDPRA (SEQ ID NO:11)
 or 
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPG
KGSFCPGPVTLCSDLESHSTEAVLGDALVDFSLKLYHAFSAMKKVETNMAFSPFSIAS
LLTQVLLGAGENTKTNLESILSYPKDFTCVHQALKGFTTKGVTSVSQIFHSPDLAIRD
TFVNASRTLYSSSPRVLSNNSDANLELINTWVAKNTNNKISRLLDSLPSDTRLVLLNA
IYLSAKWKTTFDPKKTRMEPFHFKNSVIKVPMMNSKKYPVAHFIDQTLKAKVGQLQ
LSHNLSLVILVPQNLKHRLEDMEQALSPSVFKAIMEKLEMSKFQPTLLTLPRIKVTTS
QDMLSIMEKLEFFDFSYDLNLCGLTEDPDLQVSAMQHQTVLELTETGVEAAAASAIS
VARTLLVFEVQQPFLFVLWDQQHKFPVFMGRVYDPRA (SEQ ID NO:12)
 or 
DKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSP
GKNPNATSSSSQDPESLQDRGEGKVATTVISKMLFVEPILEVSSLPTTNSTTNSATKIT
ANTTDEPTTQPTTEPTTQPTIQPTQPTTQLPTDSPTQPTTGSFCPGPVTLCSDLESHSTE
AVLGDALVDFSLKLYHAFSAMKKVETNMAFSPFSIASLLTQVLLGAGENTKTNLESI
LSYPKDFTCVHQALKGFTTKGVTSVSQIFHSPDLAIRDTFVNASRTLYSSSPRVLSNNS
DANLELINTWVAKNTNNKISRLLDSLPSDTRLVLLNAIYLSAKWKTTFDPKKTRMEP
FHFKNSVIKVPMMNSKKYPVAHFIDQTLKAKVGQLQLSHNLSLVILVPQNLKHRLED
MEQALSPSVFKAIMEKLEMSKFQPTLLTLPRIKVTTSQDMLSIMEKLEFFDFSYDLNL
CGLTEDPDLQVSAMQHQTVLELTETGVEAAAASAISVARTLLVFEVQQPFLFVLWD
QQHKFPVFMGRVYDPRA (SEQ ID NO:13)
 or 
DKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSP
GKGSFCPGPVTLCSDLESHSTEAVLGDALVDFSLKLYHAFSAMKKVETNMAFSPFSI
ASLLTQVLLGAGENTKTNLESILSYPKDFTCVHQALKGFTTKGVTSVSQIFHSPDLAIR
DTFVNASRTLYSSSPRVLSNNSDANLELINTWVAKNTNNKISRLLDSLPSDTRLVLLN
AIYLSAKWKTTFDPKKTRMEPFHFKNSVIKVPMMNSKKYPVAHFIDQTLKAKVGQL
QLSHNLSLVILVPQNLKHRLEDMEQALSPSVFKAIMEKLEMSKFQPTLLTLPRIKVTT
SQDMLSIMEKLEFFDFSYDLNLCGLTEDPDLQVSAMQHQTVLELTETGVEAAAASAI
SVARTLLVFEVQQPFLFVLWDQQHKFPVFMGRVYDPRA (SEQ ID NO:14)
 or 
ESKYGPPCPSCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSS
IEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSL
GKNPNATSSSSQDPESLQDRGEGKVATTVISKMLFVEPILEVSSLPTTNSTTNSATKIT
ANTTDEPTTQPTTEPTTQPTIQPTQPTTQLPTDSPTQPTTGSFCPGPVTLCSDLESHSTE
AVLGDALVDFSLKLYHAFSAMKKVETNMAFSPFSIASLLTQVLLGAGENTKTNLESI
LSYPKDFTCVHQALKGFTTKGVTSVSQIFHSPDLAIRDTFVNASRTLYSSSPRVLSNNS
DANLELINTWVAKNTNNKISRLLDSLPSDTRLVLLNAIYLSAKWKTTFDPKKTRMEP
FHFKNSVIKVPMMNSKKYPVAHFIDQTLKAKVGQLQLSHNLSLVILVPQNLKHRLED
MEQALSPSVFKAIMEKLEMSKFQPTLLTLPRIKVTTSQDMLSIMEKLEFFDFSYDLNL
CGLTEDPDLQVSAMQHQTVLELTETGVEAAAASAISVARTLLVFEVQQPFLFVLWD
QQHKFPVFMGRVYDPRA (SEQ ID NO:32)
 or 
ESKYGPPCPSCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSS
IEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSL
GKGSFCPGPVTLCSDLESHSTEAVLGDALVDFSLKLYHAFSAMKKVETNMAFSPFSI
ASLLTQVLLGAGENTKTNLESILSYPKDFTCVHQALKGFTTKGVTSVSQIFHSPDLAIR
DTFVNASRTLYSSSPRVLSNNSDANLELINTWVAKNTNNKISRLLDSLPSDTRLVLLN
AIYLSAKWKTTFDPKKTRMEPFHFKNSVIKVPMMNSKKYPVAHFIDQTLKAKVGQL
QLSHNLSLVILVPQNLKHRLEDMEQALSPSVFKAIMEKLEMSKFQPTLLTLPRIKVTT
SQDMLSIMEKLEFFDFSYDLNLCGLTEDPDLQVSAMQHQTVLELTETGVEAAAASAI
SVARTLLVFEVQQPFLFVLWDQQHKFPVFMGRVYDPRA (SEQ ID NO:33)
 or 
ESKYGPPCPPCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSS
IEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSL
GKNPNATSSSSQDPESLQDRGEGKVATTVISKMLFVEPILEVSSLPTTNSTTNSATKIT
ANTTDEPTTQPTTEPTTQPTIQPTQPTTQLPTDSPTQPTTGSFCPGPVTLCSDLESHSTE
AVLGDALVDFSLKLYHAFSAMKKVETNMAFSPFSIASLLTQVLLGAGENTKTNLESI
LSYPKDFTCVHQALKGFTTKGVTSVSQIFHSPDLAIRDTFVNASRTLYSSSPRVLSNNS
 
DANLELINTWVAKNTNNKISRLLDSLPSDTRLVLLNAIYLSAKWKTTFDPKKTRMEP
FHFKNSVIKVPMMNSKKYPVAHFIDQTLKAKVGQLQLSHNLSLVILVPQNLKHRLED
MEQALSPSVFKAIMEKLEMSKFQPTLLTLPRIKVTTSQDMLSIMEKLEFFDFSYDLNL
CGLTEDPDLQVSAMQHQTVLELTETGVEAAAASAISVARTLLVFEVQQPFLFVLWD
QQHKFPVFMGRVYDPRA (SEQ ID NO:15)
 or

ESKYGPPCPPCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSS
IEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSL
GKGSFCPGPVTLCSDLESHSTEAVLGDALVDFSLKLYHAFSAMKKVETNMAFSPFSI
ASLLTQVLLGAGENTKTNLESILSYPKDFTCVHQALKGFTTKGVTSVSQIFHSPDLAIR
DTFVNASRTLYSSSPRVLSNNSDANLELINTWVAKNTNNKISRLLDSLPSDTRLVLLN
AIYLSAKWKTTFDPKKTRMEPFHFKNSVIKVPMMNSKKYPVAHFIDQTLKAKVGQL
QLSHNLSLVILVPQNLKHRLEDMEQALSPSVFKAIMEKLEMSKFQPTLLTLPRIKVTT
SQDMLSIMEKLEFFDFSYDLNLCGLTEDPDLQVSAMQHQTVLELTETGVEAAAASAI
SVARTLLVFEVQQPFLFVLWDQQHKFPVFMGRVYDPRA (SEQ ID NO:16).

In some embodiments, a suitable C1-INH Fc fusion protein has an amino acid sequence at least 50%, 55%, 60%, 65%, 70%, 75%, 80%, 85%, 90%, 91%, 92%, 93%, 94%, 95%, 96%, 97%, 98%, 99% or more homologous or identical to SEQ ID NO:11, SEQ ID NO:12, SEQ ID NO:13, SEQ ID NO:14, SEQ ID NO:15, SEQ ID NO:16, SEQ ID NO:32, or SEQ ID NO:33.

It is contemplated that a C1-INH-Fc fusion protein may be provided in various configurations including homodimeric or monomeric configurations. For example, a suitable homodimeric configuration may be designed to have the C-terminal end of fusion partner (e.g., a C1-INH polypeptide plus linker) attached to the N-terminal end of both Fc polypeptide strands. A suitable monomeric configuration may be designed to have the C-terminal end of fusion partner (e.g., a C1-INH polypeptide plus linker) fused to one Fc dimer.

Monomeric, also referred to herein as monovalent, forms may be used for certain applications and routes of administration, e.g., subcutaneous administration. A monomeric configuration may decrease steric hindrance, increase half-life, and/or may increase bioavailability.

Without wishing to be bound by any theory, it is contemplated that monovalent forms may be particularly useful for C1-INH-Fc fusion constructs because C1-INH is a suicide inhibitor. Since it is a suicide inhibitor, the binding of one C1-INH "arm" of a dimer Fc fusion will result in increased rate of clearance of the bound C1-INH fusion protein, even in the event that a second arm remain unbound.

An advantage of the Fc fusion proteins, both monomeric and dimeric, is that Fc expression was found to occur at higher levels than expression of C1-INH alone. Activity assays comparing the dimeric C1-INH-Fc constructs with C1-INH without the Fc fusion have been shown to have similar C1q binding activity. The inclusion of a linker was also tested and found not to affect the ability of C1-INH-Fc fusion protein to bind its target.

Methods of making monomeric antibody fusion proteins include those described in, e.g., PCT Publication Nos. WO2011/063348; WO2012/020096; WO2013/138643; WO2014087299; Dumont, J. et al., Monomeric Fc Fusions: Impact on Pharmacokinetic and Biological Activity of Protein Therapeutics, Biodrugs, 20(3): 151-160 (2006); Ishino, T. et al, Protein Structure and Folding: Half-life Extension of Biotherapeutics Modality by N-Glycosylation for the Engineering a Monomeric Fc Domain, J. Biol. Chem., 288:16529-16537 (2013).

Monovalent C1-inhibitor can be made by using a plasmid containing the Fc-C1 co transfected with a plasmid expressing Fc alone. In addition, it could be made by using a dual promoter plasmid with one promoter generating Fc-C1 and the other promoter generating Fc alone. Monovalent Fc could also be made using bispecific technology where specific amino acids in the hinge region of the Fc are mutated to impart stability of the Fc region (e.g. Knob and hole technology or other stabilizing mutations which drive formation of the monovalent C1).

### Albumin Domains

In some embodiments, a suitable C1-INH fusion protein contains an albumin domain. Albumin is a soluble, monomeric protein which comprises about one-half of the blood serum protein. Albumin functions primarily as a carrier protein for steroids, fatty acids, and thyroid hormones and plays a role in stabilizing extracellular fluid volume. Albumin has a globular unglycosylated serum protein of molecular weight 66,500. Albumin is synthesized in the liver as preproalbumin which has an N-terminal peptide that is removed before the nascent protein is released from the rough endoplasmic reticulum. The product, proalbumin, is in turn cleaved in the Golgi vesicles to produce the secreted albumin.

Albumin is made up of three homologous domains (I-III), and each of these is comprised of two subdomains (A and B). The principal regions of ligand binding to human serum albumin are located in cavities in subdomains IIA and IIIA, which are formed mostly of hydrophobic and positively charged residues and exhibit similar chemistry. Human serum albumin has 585 amino acids and a molecular mass of 66,500 Da. The amino acids include 35 cysteines, all but one of which are involved in the formation of 17 stabilizing disulfide bonds.

Typically, Albumin has a prolonged serum half-life of 19 days. FcRn controls the long serum half-life of albumin. FcRn is a dual binding receptor that, in addition to albumin, binds IgG, and protects both proteins from intracellular degradation. The C-terminal domain of the albumin molecule has been shown to be important for binding to FcRn. In particular, domain IIIB is shown to be important for binding to FcRn. In some embodiments, lack of domain IIIB or mutations of 464His, 510His, and 535His abolishes FcRn binding.

Typically, Albumin fusion proteins of the invention are monomeric. In some embodiments, this feature may be an advantage over the dimeric Fc fusion embodiments for the reasons described above with regard to monomeric Fc fusion embodiments.

In some embodiments, an albumin polypeptide suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the wild-type human serum albumin:











 (SEQ ID NO:17).

In some embodiments, an albumin polypeptide suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the D3 domain of wild-type human serum albumin:

METPAQLLFLLLLWLPDTTGVEEPQNLIKQNCELFEQLGEYKFQNALLVRYTKKVPQ
VSTPTLVEVSRNLGKVGSKCCKHPEAKRMPCAEDYLSVVLNQLCVLHEKTPVSDRV
TKCCTESLVNRRPCFSALEVDETYVPKEFNAETFTFHADICTLSEKERQIKKQTALVE

LVKHKPKATKEQLKAVMDDFAAFVEKCCKADDKETCFAEEGKKLVAASRAALGL
(SEQ ID NO:20).

### Linker or Spacer

A C1-INH polypeptide or domain may be directly or indirectly linked to an Fc domain or an albumin domain. In some embodiments, a suitable C1-INH fusion protein contains a linker or spacer that joins a C1-INH polypeptide or domain and an Fc or albumin domain. An amino acid linker or spacer is generally designed to be flexible or to interpose a structure, such as an alpha-helix, between the two protein moieties. A linker or spacer can be relatively short, or can be longer. Typically, a linker or spacer contains for example 3-100 (e.g., 5-100, 10-100, 20-100 30-100, 40-100, 50-100, 60-100, 70-100, 80-100, 90-100, 5-55, 10-50, 10-45, 10-40, 10-35, 10-30, 10-25, 10-20) amino acids in length. In some embodiments, a linker or spacer is equal to or longer than 2, 3, 4, 5, 6, 7, 8, 9, 10, 15, 20, 25, 30, 35, 40, 45, 50, 55, 60, 65, 70, 75, 80, 85, 90, 95, or 100 amino acids in length. Typically, a longer linker may decrease steric hindrance. In some embodiments, a linker will comprise a mixture of glycine and serine residues. In some embodiments, the linker may additionally comprise threonine, proline, and/or alanine residues. Thus, in some embodiments, the linker comprises between 10-100, 10-90, 10-80, 10-70, 10-60, 10-50, 10-40, 10-30, 10-20, 10-15 amino acids. In some embodiments, the linker comprises at least 10, 15, 20, 25, 30, 35, 40, 45, 50, 55, 60, 65, 70, 75, 80, 85, 90, or 95 amino acids. In some embodiments, the linker is not a linker consisting of ALEVLFQGP (SEQ ID NO: 37).

Linkers or spacers suitable for the present invention include GGG linker and GGGGSGGGGS ((GGGGS)2 linker SEQ ID NO:27). In some embodiments, the linker comprises the sequence GGG and/or the sequence of SEQ ID NO:27.

Other suitable linkers include GAPGGGGGAAAAAGGGGGGAP (GAG linker, SEQ ID NO:34);
GAPGGGGGAAAAAGGGGGGAPGGGGGAAAAAGGGGGGAP (GAG2 linker, SEQ ID NO:35); and

GAPGGGGGAAAAAGGGGGGAPGGGGGAAAAAGGGGGGAPGGGGGAAAAAGGG
GGGAP (GAG3 linker, SEQ ID NO:36).

Suitable linkers or spacers also include those having an amino acid sequence at least 50%, 55%, 60%, 65%, 70%, 75%, 80%, 85%, 90%, 91%, 92%, 93%, 94%, 95%, 96%, 97%, 98%, 99% or more homologous or identical to the above exemplary linkers, e.g., GGG linker, GGGGSGGGGS ((GGGGS)2 linker SEQ ID NO:27), GAG linker (SEQ ID NO:34), GAG2 linker (SEQ ID NO:35), or GAG3 linker (SEQ ID NO:36). Additional linkers suitable for use with some embodiments may be found in US2012/0232021, filed on March 2, 2012.

Typically, a linker is included that associates the C1-INH polypeptide or domain with the Fc or albumin domain without substantially affecting or reducing the ability of the C1-INH polypeptide or domain to bind to any of its cognate ligands (e.g., C1s, etc.).

### Glycosylation/Glycan Mapping (Profile) of C1-INH Proteins

According to the present invention, a C1-INH protein is conjugated at a sialic acid residue. Thus, a C1-INH protein suitable for conjugation according to the present invention may be characterized with distinct glycan maps, in particular, sialic acid content. In some embodiments, a C1-INH protein has a glycosylation profile similar to that of plasma-derived C1-INH. In some embodiments, a C1-INH protein has a glycosylation profile that is distinct from that of plasma-derived C1-INH.

Without wishing to be bound by any theory, it is thought that glycan map including glycan linkage along with the shape and complexity of the branch structure may impact in vivo clearance, bioavailability, and/or efficacy.

Typically, a glycan map may be determined by enzymatic digestion and subsequent chromatographic analysis. Various enzymes may be used for enzymatic digestion including suitable glycosylases, peptidases (e.g., Endopeptidases, Exopeptidases), proteases, and phosphatases. In some embodiments, a suitable enzyme is alkaline phosphatase. In some embodiments, a suitable enzyme is neuraminidase. Glycans may be detected by chromatographic analysis. For example, glycans may be detected by High Performance Anion Exchange Chromatography with Pulsed Amperometric Detection (HPAE-PAD) or size exclusion High Performance Liquid Chromatography (HPLC). The quantity of glycan represented by each peak on a glycan map may be calculated using a standard curve of glycan according to methods known in the art and disclosed herein.

In some embodiments, C1-INH proteins may be characterized with a glycan map. The relative amount of glycan corresponding to each peak group may be determined based on the peak group area relative to the corresponding peak group area in a predetermined reference standard. Various reference standards for glycan mapping are known in the art and can be used to practice the present invention. In some embodiments, C1-INH proteins may be characterized with a glycan map comprising five or fewer peak groups selected from the peak groups indicative of neutral, mono-sialylated, di-sialylated, tri-sialylated , or tetra-sialylated C1-INH protein.

In some embodiments, C1-INH proteins have a glycosylation profile comprising at least one of the following: neutral glycan species, mono-sialylated species, di-sialylated species, tri-sialylated species and/or tetra-sialylated species. In some embodiments, C1-INH proteins have a glycosylation profile comprising neutral glycan species, mono-sialylated species, di-sialylated species, tri-sialylated species and tetra-sialylated species. In some embodiments, C1-INH proteins have a glycosylation profile comprising no more than about 50%, 45%, 40%, 35 %, 30%, 25%, 20%, 15 %, 10%, or 5% neutral glycan species. In some embodiments, C1-INH proteins have a glycosylation profile comprising between about 5% and about 30% neutral glycan species. In some embodiments, C1-INH proteins have a glycosylation profile comprising between about 5% and about 25% neutral glycan species. In some embodiments, C1-INH proteins have a glycosylation profile comprising between about 10% and about 20% neutral glycan species. In some embodiments, C1-INH proteins comprises, on average, at least about 80% charged glycans per molecule (e.g., greater than about 85%, 90%, 95% or 99% glycans per molecule). In some embodiments, C1-INH proteins have a glycosylation profile comprising between about 10% and about 30% mono-sialylated species. In some embodiments, C1-INH proteins have a glycosylation profile comprising between about 30% and about 50% di-sialylated species. In some embodiments, C1-INH proteins have a glycosylation profile comprising between about 15% and about 35% tri-sialylated species. In some embodiments, C1-INH proteins have a glycosylation profile comprising between about 5% and about 15% tetra-sialylated species. In some embodiments, C1-INH proteins have a glycosylation profile comprising no more than 30% neutral glycan species, between about 20% and about 30% mono-sialylated glycan species, between about 30% and about 40% di-sialylated glycan species, between about 10% and about 20% tri-sialylated glycan species, and between about 5% and about 10% tetra-sialylated glycan species.

In some embodiments, C1-INH proteins have a sialylation profile similar to that of plasma-derived C1-INH. In some embodiments, C1-INH proteins have a sialylation profile distinct than that of plasma-derived C1-INH. In some embodiments, C1-INH proteins have a sialylation profile that renders a half-life similar to or longer than that of plasma-derived C1-INH. In some embodiments, C1-INH proteins comprise, on average, at least about 10, 11, 12, 13, or 14 sialylated glycan residues per molecule. In some embodiments, C1-INH proteins comprise, on average, at least about 15, 16, 17, 18, 19, 20, 21, 22, 23, 24, 25, 26, 27, 28, or 29 sialylated glycan residues per molecule. In some embodiments, C1-INH proteins comprise, on average, at least about 30, 31, 32, 33, 34, 35, 36, 37, 38, 39, or 40 sialylated glycan residues per molecule.

In some embodiments, C1-INH proteins contain less than about 20%, 15%, 10%, or 5% of one or more of mannose, α-galactose, N-glycolylneuraminic acid (NGNA), or oligomannose-type glycosylation. In some embodiments, C1-INH proteins contain no more than about 20%, 15%, 10%, or 5% of one or more of mannose, α-galactose, N-glycolylneuraminic acid (NGNA), or oligomannose-type glycosylation.

In some embodiments, C1-INH proteins have a glycosylation profile that is not immunogenic. In some embodiments, C1-INH proteins have a glycosylation profile that does not increase serum clearance rate when compared with plasma-derived human C1-INH. In some embodiments, C1-INH proteins have a glycosylation profile that decreases serum clearance rate when compared with plasma-derived human C1-INH. In some embodiments, C1-INH proteins have a glycosylation profile that decreases serum clearance rate when compared with conestat alfa.

Various methods of manipulating the glycosylation profile of proteins are known in the art. These methods as well as others yet to be discovered are contemplated by the instant invention. Methods of manipulating the glycosylation profile of C1-INH proteins and polypeptides of the invention include in vitro, in situ, and in vivo methods. In some embodiments the glycosylation profile of expressed proteins or polypeptides is altered through post-expression chemical modification of the expressed protein or polypeptide. In some embodiments the cell culture conditions are manipulated to achieve expression of proteins having a desired glycosylation profile. These cell culture conditions include control of the production and culture process including length of culture, additives to culture medium, and/or co-expression of genes to enhance glycosylation. Selection of host cells and specific clones of transfected host cells may also be used to enhance glycosylation. Some methods of enhancing glycosylation include purification processes to enrich for proteins or polypeptides having the desired glycosylation profile.

In some embodiments, cells engineered to express C1-INH proteins can also be engineered to modify glycosylation, in particular, increase sialylation of the expressed C1-INH. For example, cells may be engineered to express a heterologous enzyme in the glycosylation pathway (wild-type or mutated) to achieve desired glycosylation, e.g., to increase sialylation. In some embodiments, cells may also be engineered to overexpress an endogenous enzyme to achieve desired glycosylation, e.g., to increase sialylation. In some embodiments, cells are engineered to reduce or prevent expression of endogenous enzymes that reduce, inhibit, or degrade sialylation (e.g., with an antisense construct).

The various glycosylation patterns/glycan maps and in particular, sialylation profiles or levels, described herein may be applicable to a C1-INH domain or polypeptide alone or in a fusion protein context (e.g., a C1-INH-Fc or C1-INH-albumin fusion protein). C1-INH proteins with glycosylation patterns/glycan maps and in particular, sialylation profiles or levels, described herein may be conjugated or unconjugated. It is contemplated that a desired glycosylation pattern/glycan map including a desired sialylation profile or level may extend in vivo half-life of C1-INH protein. In particular, a desired glycosylation pattern/glycan map including a desired sialylation profile or level, in combination with Fc or albumin fusion, may achieve desired in vivo half-life of CL-INH protein described in this application even without conjugation. Conjugation (e.g., PEGylation) however further extends in vivo half-life of C1-INH proteins including those with desired glycosylation pattern or sialylation level.

### PEGylation

According to the present invention, a chemical or biological moiety can be conjugated, directly or indirectly, to a C1-INH protein described herein, as defined in the claims. In particular, such a moiety is a polyethylene glycol (PEG) moiety including mono- or poly-(e.g., 2-4) PEG moieties. As used herein, a process of conjugating a PEG moiety, directly or indirectly, to a protein is referred to as PEGylation. PEGylation can result in increased half-life of C1-INH, as described herein.

PEGylation can be carried out by any suitable reaction known in the art. Methods for preparing a PEGylated protein can generally include (a) reacting a polypeptide with polyethylene glycol (such as a reactive ester or aldehyde derivative of PEG) under conditions whereby the polypeptide becomes attached to one or more PEG groups; and (b) obtaining the reaction product(s). In general, the conditions for the reactions can be determined case by case based on known parameters and the desired result.

There are a number of PEG attachment methods available to those skilled in the art and described in, for example, EP 0 401 384; Malik et al., Exp. Hematol., 20:1028-1035 (1992); EP 0 154 316; EP 0 401 384; WO 92/16221; and WO 95/34326. For example, the step of PEGylating a therapeutic molecule described herein can be carried out via an acylation reaction or an alkylation reaction with a reactive polyethylene glycol molecule.

| **Target sites** | **Activated PEGs** |
|---|---|
| N-terminal amino group | PEG-NHS, PEG-Aldehyde, PEG-p-Nitrophenyloxycarbonyl |
| -NH₂ of Lysine | PEG-NHS, PEG-Aldehyde, PEG-p-Nitrophenyloxycarbonyl |
| carboxylic group | PEG-NH2 |
| Thiol/cysteine | PEG-Maleimide, PEG-Iodoacetamide |
| Glycan/aldehyde (sialic acid and terminal galatose) | PEG-Aminoxy, PEG-Hydrazide |

In some embodiments, a PEG moiety for conjugation is an activated PEG. For example, a suitable PEG moiety may include an aminoxy functional group.

In some embodiments, a PEG moiety may have linear or branched structures. For example, a PEG moiety may include 2, 3, 4, or 5 arm branches. A suitable PEG-NHS moiety may include linear PEG-NHS 1K, linear PEG-NHS 2K, linear PEG-NHS 5K, branched PEG-NHS 5K, branched PEG-NHS 20K, or branched PEG-NHS 40K. As a further example, a PEG-aminoxy moiety may include linear or branched PEG-aminoxy 2K, PEG-aminoxy 5K, PEG-aminoxy 5K, PEG-aminoxy 10K, PEG-aminoxy 20K, or PEG-aminoxy 40K.

The PEG is conjugated to C1-INH via one or more sialic acid residues of the C1-INH protein. In some embodiments one or more of the sialic acid residues of the C1-INH protein are oxidized before the PEG is conjugated to the sialic acid residues.

The PEG is conjugated to oxidized sialic acid via an oxime linkage.

A C1-INH protein may be PEGylated at various levels according to the present invention. For example, the molar ratio of PEG to C1-INH may range between about 5:1 and 100:1; between about 10:1 and 100:1; between about 15:1 and 100:1; between about 20:1 and 100:1; between about 25:1 and 100:1; between about 30:1 and 100:1; between about 40:1 and 100:1; between about 50:1 and 100:1; between about 10:1 and 90:1; between about 10:1 and 80:1; between about 10:1 and 70:1; between about 10:1 and 60:1; between about 10:1 and 50:1; between about 10:1 and 40:1; between about 15:1 and 35:1; or between about 20:1 and 30:1. In some embodiments, the molar ratio of PEG to C1-INH may be at least about 1:1, at least about 5:1, at least about 10:1; at least about 15: 1; at least about 20:1; at least about 25:1; at least about 30:1; at least about 35:1; at least about 40:1; at least about 45:1; or at least about 50:1.

In some embodiments, the molar ratio of PEG to sialic acid is at least about 1:1, at least about 1:5, at least about 1:10, at least about 1:15, at least about 1:20, at least about 1:25, at least about 1:30, at least about 1:35, at least about 1:40 at least about 1:45, at least about 1:50. In some embodiments, the molar ratio of PEG to sialic acid is between about 1:1 and about 1:50, between about 1:1 and about 1:45, between about 1:1 and about 1:40, between about 1:1 and about 1:35, between about 1:1 and about 1:30, between about 1:1 and about 1:25, between about 1:1 and about 1:20, between about 1:1 and about 1:15, between about 1:1 and about 1:10, or between about 1:1 and about 1:5.

### Polysialic Acid Conjugation

Polysialic acid (PSA), also referred to as colominic acid (CA), is a naturally occurring polysaccharide. It is a homopolymer of N-acetylneuraminic acid with α(2→8) ketosidic linkage and contains vicinal diol groups at its non-reducing end. It is negatively charged and a natural constituent of the human body.

PSAs consist of polymers (generally homopolymers) of N-acetylneuraminic acid. The secondary amino group normally bears an acetyl group, but it may instead bear a glycolyl group. Possible substituents on the hydroxyl groups include acetyl, lactyl, ethyl, sulfate, and phosphate groups.

PSAs and modified PSAs (mPSAs) generally comprise linear polymers consisting essentially of N-acetylneuraminic acid moieties linked by 2,8- or 2,9- glycosidic linkages or combinations of these (e.g. alternating 2,8- and 2,9- linkages). In some embodiments, the glycosidic linkages of PSAs and mPSAs, are α-2,8. Such PSAs and mPSAs are derived from colominic acids. Typical PSAs and mPSAs comprise at least 2, preferably at least 5, more preferably at least 10 and most preferably at least 20 N-acetylneuraminic acid moieties. Thus, they may comprise from 2 to 300 N-acetylneuraminic acid moieties, preferably from 5 to 200 N-acetylneuraminic acid moieties, or most preferably from 10 to 100 N-acetylneuraminic acid moieties. PSAs and CAs preferably are essentially free of sugar moieties other than N-acetylneuraminic acid. In some embodiments, PSAs comprise at least 90%, at least 95% and or at least 98% N-acetylneuraminic acid moieties.

Where PSAs comprise moieties other than N-acetylneuraminic acid (as, for example in mPSAs) these are preferably located at one or both of the ends of the polymer chain. Such "other" moieties may, for example, be moieties derived from terminal N-acetylneuraminic acid moieties by oxidation or reduction.

For example, WO 2001/087922 describes mPSAs in which the non-reducing terminal N-acetylneuraminic acid unit is converted to an aldehyde group by reaction with sodium periodate. Additionally, WO 2005/016974 describes mPSAs in which the reducing terminal N-acetylneuraminic acid unit is subjected to reduction to reductively open the ring at the reducing terminal N-acetylneuraminic acid unit, whereby a vicinal diol group is formed, followed by oxidation to convert the vicinal diol group to an aldehyde group.

Different PSA derivatives can be prepared from oxidized PSA containing a single aldehyde group at the non-reducing end. The preparation of aminooxy PSA is described, for example, in WO2012/166622. PSA-NH2 containing a terminal amino group can be prepared by reductive amination with NH₄Cl and PSA-SH containing a terminal sulfhydryl group by reaction of PSA-NH2 with 2-iminothiolane (Traut's reagent), both procedures are described in US 7,645,860 B2. PSA hydrazine can be prepared by reaction of oxidized PSA with hydrazine according to US 7,875,708 B2. PSA hydrazide can be prepared by reaction of oxidized PSA with adipic acid dihydrazide (WO 2011/012850 A2).

Colominic acids (a sub-class of PSAs) are homopolymers of N-acetylneuraminic acid (NANA) with α (2→8) ketosidic linkage, and are produced, inter alia, by particular strains of Escherichia coli possessing K1 antigen. Colominic acids have many physiological functions. They are important as a raw material for drugs and cosmetics.

As used herein, "sialic acid moieties" includes sialic acid monomers or polymers ("polysaccharides") which are soluble in an aqueous solution or suspension and have little or no negative impact, such as side effects, to mammals upon administration of the PSA-blood coagulation protein conjugate in a pharmaceutically effective amount. The polymers are characterized, in one aspect, as having 1, 2, 3, 4, 5, 10, 20, 30, 40, 50, 60, 70, 80, 90, 100, 200, 300, 400, or 500 sialic acid units. In certain aspects, different sialic acid units are combined in a chain.

In some embodiments, the sialic acid portion of the polysaccharide compound is highly hydrophilic, and in another embodiment the entire compound is highly hydrophilic. Hydrophilicity is conferred primarily by the pendant carboxyl groups of the sialic acid units, as well as the hydroxyl groups. The saccharide unit may contain other functional groups, such as, amine, hydroxyl or sulphate groups, or combinations thereof. These groups may be present on naturally-occurring saccharide compounds, or introduced into derivative polysaccharide compounds.

The naturally occurring polymer PSA is available as a polydisperse preparation showing a broad size distribution (e.g. Sigma C-5762) and high polydispersity (PD). Because the polysaccharides are usually produced in bacteria carrying the inherent risk of copurifying endotoxins, the purification of long sialic acid polymer chains may raise the probability of increased endotoxin content. Short PSA molecules with 1-4 sialic acid units can also be synthetically prepared (Kang SH et al., Chem Commun. 2000;227-8; Ress DK and Linhardt RJ, Current Organic Synthesis. 2004;1:31-46), thus minimizing the risk of high endotoxin levels. However PSA preparations with a narrow size distribution and low polydispersity, which are also endotoxin-free, can now be manufactured. Polysaccharide compounds of particular use for the present disclosure are, in one aspect, those produced by bacteria. Some of these naturally-occurring polysaccharides are known as glycolipids. In some embodiments, the polysaccharide compounds are substantially free of terminal galactose units.

The PSA is conjugated to C1-INH via one or more sialic acid residues of the C1-INH protein. In some embodiments one or more of the sialic acid residues of the C1-INH protein are oxidized before the PSA is conjugated to the sialic acid residues.

The PSA is conjugated to oxidized sialic acid via an oxime linkage.

A C1-INH protein may be conjugated with PSA at various levels according to the present invention. For example, the molar ratio of PSA to C1-INH may range between about 5:1 and 100:1; between about 10:1 and 100:1; between about 15:1 and 100:1; between about 20:1 and 100:1; between about 25:1 and 100:1; between about 30:1 and 100:1; between about 40:1 and 100:1; between about 50:1 and 100:1; between about 10:1 and 90:1; between about 10:1 and 80:1; between about 10:1 and 70:1; between about 10:1 and 60:1; between about 10:1 and 50:1; between about 10:1 and 40:1; between about 15:1 and 35:1; or between about 20:1 and 30:1. In some embodiments, the molar ratio of PSA to C1-INH may be at least about 1:1, at least about 5:1, at least about 10:1; at least about 15:1; at least about 20:1; at least about 25:1; at least about 30:1; at least about 35:1; at least about 40:1; at least about 45:1; or at least about 50:1.

In some embodiments, the molar ratio of PSA to sialic acid is at least about 1:1, at least about 1:5, at least about 1:10, at least about 1:15, at least about 1:20, at least about 1:25, at least about 1:30, at least about 1:35, at least about 1:40 at least about 1:45, at least about 1:50. In some embodiments, the molar ratio of PSA to sialic acid is between about 1:1 and about 1:50, between about 1:1 and about 1:45, between about 1:1 and about 1:40, between about 1:1 and about 1:35, between about 1:1 and about 1:30, between about 1:1 and about 1:25, between about 1:1 and about 1:20, between about 1:1 and about 1:15, between about 1:1 and about 1:10, or between about 1:1 and about 1:5.

### Extended half-life

According to the present invention, conjugation (e.g., PEGylation or PSA conjugated) extends in vivo half-life of C1-INH. Typically, conjugated (e.g., PEGylated or PSA conjugated) C1-INH has a half-life longer than the unconjugated (e.g., un-PEGylated or non-PSA conjugated) C1-INH. In some embodiments, conjugated (e.g., PEGylated or PSA conjugated) C1-INH has a half-life comparable to or greater than a plasma-derived human C1-INH protein. In some embodiments, the half-life of the conjugated (e.g., PEGylated or PSA conjugated) C1-INH is in the range of about 80%-500%, 90%-500%, 100%-500%, 110%-500%, 120%-500%, 80%-400%, 90%-300%, 100%-300%, 100%-250%, 100%-200%, or 100%-150% of the half-life of the plasma-derived C1-INH protein.

In some embodiments, the conjugated (e.g., PEGylated or PSA conjugated) C1-INH protein has a half-life of at least about 70, 75, 80, 85, 90, 95, 100, 105, 110, 115, 120, 125, 130, 135, 140, 145, 150, 155, 160, 165, or 170 hours. In some embodiments, conjugated (e.g., PEGylated or PSA conjugated) C1-INH has an *in vivo* half-life of or greater than about 2 days, 2.5 days, 3 days, 3.5 days, 4 days, 4.5 days, 5 days, 5.5 days, 6 days, 6.5 days, 7 days, 7.5 days, 8 days, 8.5 days, 9 days, 9.5 days, 10 days, 11 days, 12 days, 13 days, or 14, days. In some embodiments, a conjugated (e.g., PEGylated or PSA conjugated) C1-INH protein has an *in vivo* half-life ranging between about 0.5 and 14 days, 0.5 and 10 days, between 1 day and 10 days, between 1 day and 9 days, between 1 day and 8 days, between 1 day and 7 days, between 1 day and 6 days, between 1 day and 5 days, between 1 day and 4 days, between 1 day and 3 days, between 2 days and 10 days, between 2 days and 9 days, between 2 days and 8 days, between 2 days and 7 days, between 2 days and 6 days, between 2 days and 5 days, between 2 days and 4 days, between 2 day and 3 days, between 2.5 days and 10 days, between 2.5 days and 9 days, between 2.5 days and 8 days, between 2.5 days and 7 days, between 2.5 days and 6 days, between 2.5 days and 5 days, between 2.5 days and 4 days, between 3 days and 10 days, between 3 days and 9 days, between 3 days and 8 days, between 3 days and 7 days, between 3 days and 6 days, between 3 days and 5 days, between 3 days and 4 days, between 3.5 days and 10 days, between 3.5 days and 9 days, between 3.5 days and 8 days, between 3.5 days and 7 days, between 3.5 days and 6 days, between 3.5 days and 5 days, between 3.5 days and 4 days, between 4 days and 10 days, between 4 days and 9 days, between 4 days and 8 days, between 4 days and 7 days, between 4 days and 6 days, between 4 days and 5 days, between 4.5 days and 10 days, between 4.5 days and 9 days, between 4.5 days and 8 days, between 4.5 days and 7 days, between 4.5 days and 6 days, between 4.5 days and 5 days, between 5 days and 10 days, between 5 days and 9 days, between 5 days and 8 days, between 5 days and 7 days, between 5 days and 6 days, between 5.5 days and 10 days, between 5.5 days and 9 days, between 5.5 days and 8 days, between 5.5 days and 7 days, between 5.5 days and 6 days, between 6 days and 10 days, between 7 days and 10 days, between 8 days and 10 days, between 9 days and 10 days, between 10 days and 11 days, between 11 days and 12 days, between 12 days and 13 days, between 13 days and 14 days.

### Pharmaceutical compositions

The present invention further provides a pharmaceutical composition containing a conjugated C1-INH described herein and a physiologically acceptable carrier, as defined in the claims. The carrier and conjugated C1-INH protein are typically sterile and formulated to suit the mode of administration.

Suitable pharmaceutically acceptable carriers include water, salt solutions (e.g., NaCl), saline, buffered saline, alcohols, glycerol, ethanol, gum arabic, vegetable oils, benzyl alcohols, polyethylene glycols, gelatin, carbohydrates such as lactose, amylose or starch, sugars such as mannitol, sucrose, or others, dextrose, magnesium stearate, talc, silicic acid, viscous paraffin, perfume oil, fatty acid esters, hydroxymethylcellulose, polyvinyl pyrrolidone, *etc.,* as well as combinations thereof. The pharmaceutical preparations can, if desired, be mixed with auxiliary agents (e.g., lubricants, preservatives, stabilizers, wetting agents, emulsifiers, salts for influencing osmotic pressure, buffers, coloring, flavoring and/or aromatic substances and the like) which do not deleteriously react with the active compounds or interference with their activity. In a preferred embodiment, a water-soluble carrier suitable for intravenous administration is used.

A suitable pharmaceutical composition or medicament, if desired, can also contain minor amounts of wetting or emulsifying agents, or pH buffering agents. A composition can be a liquid solution, suspension, emulsion, tablet, pill, capsule, sustained release formulation, or powder. A composition can also be formulated as a suppository, with traditional binders and carriers such as triglycerides. Oral formulations can include standard carriers such as pharmaceutical grades of mannitol, lactose, starch, magnesium stearate, polyvinyl pyrrolidone, sodium saccharine, cellulose, magnesium carbonate, *etc.*

A pharmaceutical composition or medicament can be formulated in accordance with the routine procedures as a pharmaceutical composition adapted for administration to human beings. For example, in some embodiments, a composition for intravenous administration typically is a solution in sterile isotonic aqueous buffer. Where necessary, the composition may also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is to be administered by infusion, it can be dispensed with an infusion bottle containing sterile pharmaceutical grade water, saline or dextrose/water. Where the composition is administered by injection, an ampule of sterile water for injection or saline can be provided so that the ingredients may be mixed prior to administration.

A conjugated C1-INH described herein can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, *etc.*

A preferred formulation comprises 50 mM NaPO4 (pH 7.2), 50 mM Sorbitol, and 150 mM Glycine. The formulation may be liquid, or may be lyophilized and reconstituted prior to administration.

### Routes of Administration

A conjugated C1-INH described herein (or a composition or medicament containing a conjugated C1-INH described herein) is administered by any appropriate route. In some embodiments, a conjugated C1-INH or a pharmaceutical composition containing the same is administered systemically. Systemic administration may be intravenous, intradermal, intracranial, intrathecal, inhalation, transdermal (topical), intraocular, intramuscular, subcutaneous, intramuscular, oral, and/or transmucosal administration. In some embodiments, a conjugated C1-INH or a pharmaceutical composition containing the same is administered subcutaneously. As used herein, the term "subcutaneous tissue", is defined as a layer of loose, irregular connective tissue immediately beneath the skin. For example, the subcutaneous administration may be performed by injecting a composition into areas including the thigh region, abdominal region, gluteal region, or scapular region. In some embodiments, a conjugated C1-INH or a pharmaceutical composition containing the same is administered intravenously. In some embodiments, a conjugated C1-INH or a pharmaceutical composition containing the same is administered orally. In some embodiments, a conjugated C1-INH or a pharmaceutical composition containing the same is administered intracranially. In some embodiments, a conjugated C1-INH or a pharmaceutical composition containing the same is administered intrathecally. More than one route can be used concurrently, if desired.

In some embodiments, a conjugated C1-INH or a pharmaceutical composition containing the same is administered to the subject by subcutaneous (i.e., beneath the skin) administration. For such purposes, the formulation may be injected using a syringe. However, other devices for administration of the formulation are available such as injection devices (e.g., the Inject-ease^{™} and Genject^{™} devices); injector pens (such as the GenPen^{™}); needleless devices (e.g., MediJector^{™} and BioJector^{™}); and subcutaneous patch delivery systems. Thus, the present invention further provides a kit containing a pharmaceutical composition comprising conjugated C1-INH (e.g., in a liquid and lyophilized form) and an injection device such as a syringe. In some embodiments, the syringe is preloaded with the pharmaceutical composition comprising conjugated C1-INH. Wherein the pharmaceutical composition is lyophilized, the kit may further include a reconstitution buffer.

The present invention contemplates single as well as multiple administrations of a therapeutically effective amount of a conjugated C1-INH or a pharmaceutical composition containing the same described herein. A conjugated C1-INH or a pharmaceutical composition containing the same can be administered at regular intervals, depending on the nature, severity and extent of the subject's condition (e.g., hereditary angioedema). In some embodiments, a therapeutically effective amount of a conjugated C1-INH or a pharmaceutical composition containing the same may be administered periodically at regular intervals (e.g., once every year, once every six months, once every five months, once every three months, bimonthly (once every two months), monthly (once every month), biweekly (once every two weeks), weekly, daily or continuously).

In some embodiments, administration results only in a localized effect in an individual, while in other embodiments, administration results in effects throughout multiple portions of an individual, for example, systemic effects. Typically, administration results in delivery of a conjugated C1-INH to one or more target tissues. In some embodiments, the conjugated C1-INH is delivered to one or more target tissues including heart, brain, skin, blood, spinal cord, striated muscle (e.g., skeletal muscle), smooth muscle, kidney, liver, lung, and/or spleen. In some embodiments, the conjugated C1-INH is delivered to the heart. In some embodiments, the conjugated C1-INH is delivered to the central nervous system, particularly the brain and/or spinal cord. In some embodiments, the conjugated C1-INH is delivered to triceps, tibialis anterior, soleus, gastrocnemius, biceps, trapezius, deltoids, quadriceps, and/or diaphragm.

### Dosage Forms and Dosing Regimen

In some embodiments, a composition is administered in a therapeutically effective amount and/or according to a dosing regimen that is correlated with a particular desired outcome (e.g., with prophylaxis of a complement-mediated chronic disease, such as HAE).

Particular doses or amounts to be administered in accordance with the present invention may vary, for example, depending on the nature and/or extent of the desired outcome, on particulars of route and/or timing of administration, and/or on one or more characteristics (e.g., weight, age, personal history, genetic characteristic, lifestyle parameter, severity of cardiac defect and/or level of risk of cardiac defect, etc., or combinations thereof). Such doses or amounts can be determined by those of ordinary skill. In some embodiments, an appropriate dose or amount is determined in accordance with standard clinical techniques. Alternatively or additionally, in some embodiments, an appropriate dose or amount is determined through use of one or more *in vitro* or *in vivo* assays to help identify desirable or optimal dosage ranges or amounts to be administered.

In various embodiments, a conjugated C1-INH is administered at a therapeutically effective amount. Generally, a therapeutically effective amount is sufficient to achieve a meaningful benefit to the subject (e.g., prophylaxis, treating, modulating, curing, preventing and/or ameliorating the underlying disease or condition). Generally, the amount of a therapeutic agent (e.g., a conjugated C1-INH) administered to a subject in need thereof will depend upon the characteristics of the subject. Such characteristics include the condition, disease severity, general health, age, sex and body weight of the subject. One of ordinary skill in the art will be readily able to determine appropriate dosages depending on these and other related factors. In addition, both objective and subjective assays may optionally be employed to identify optimal dosage ranges. In some particular embodiments, appropriate doses or amounts to be administered may be extrapolated from dose-response curves derived from *in vitro* or animal model test systems.

In some embodiments, a composition is provided as a pharmaceutical formulation. In some embodiments, a pharmaceutical formulation is or comprises a unit dose amount for administration in accordance with a dosing regimen correlated with achievement of the reduced incidence or risk of an HAE attack.

In some embodiments, a formulation comprising a conjugated C1-INH described herein administered as a single dose. In some embodiments, a formulation comprising a conjugated C1-INH described herein is administered at regular intervals. Administration at an "interval," as used herein, indicates that the therapeutically effective amount is administered periodically (as distinguished from a one-time dose). The interval can be determined by standard clinical techniques. In some embodiments, a formulation comprising a conjugated C1-INH described herein is administered bimonthly, monthly, twice monthly, triweekly, biweekly, weekly, twice weekly, thrice weekly, daily, twice daily, or every six hours. The administration interval for a single individual need not be a fixed interval, but can be varied over time, depending on the needs of the individual.

A therapeutically effective amount is commonly administered in a dosing regimen that may comprise multiple unit doses. For any particular therapeutic protein, a therapeutically effective amount (and/or an appropriate unit dose within an effective dosing regimen) may vary, for example, depending on route of administration, on combination with other pharmaceutical agents. Also, the specific therapeutically effective amount (and/or unit dose) for any particular patient may depend upon a variety of factors including the disorder being treated and the severity of the disorder; the activity of the specific pharmaceutical agent employed; the specific composition employed; the age, body weight, general health, sex and diet of the patient; the time of administration, route of administration, and/or rate of excretion or metabolism of the specific C1-INH employed; the duration of the treatment; and like factors as is well known in the medical arts.

As used herein, the term "bimonthly" means administration once per two months (*i.e.,* once every two months); the term "monthly" means administration once per month; the term "triweekly" means administration once per three weeks (i.e., once every three weeks); the term "biweekly" means administration once per two weeks (*i.e.,* once every two weeks); the term "weekly" means administration once per week; and the term "daily" means administration once per day.

In some embodiments, a formulation comprising a conjugated C1-INH described herein is administered at regular intervals indefinitely. In some embodiments, a formulation comprising a conjugated C1-INH described herein is administered at regular intervals for a defined period.

It is to be further understood that for any particular subject, specific dosage regimens should be adjusted over time according to the individual need and the professional judgment of the person administering or supervising the administration of the enzyme replacement therapy and that dosage ranges set forth herein are exemplary only.

### Combination Therapy

In some embodiments, a conjugated C1-INH is administered in combination with one or more known therapeutic agents (e.g., corticosteroids) currently used for treatment of a complement-mediated disease. In some embodiments, the known therapeutic agent(s) is/are administered according to its standard or approved dosing regimen and/or schedule. In some embodiments, the known therapeutic agent(s) is/are administered according to a regimen that is altered as compared with its standard or approved dosing regimen and/or schedule. In some embodiments, such an altered regimen differs from the standard or approved dosing regimen in that one or more unit doses is altered (e.g., reduced or increased) in amount, and/or in that dosing is altered in frequency (e.g., in that one or more intervals between unit doses is expanded, resulting in lower frequency, or is reduced, resulting in higher frequency).

### Complement-mediated Disorders

Conjugated C1-INH and pharmaceutical composition containing the same may be used to treat HAE and various other complement-mediated disorders.

In some embodiments, the conjugated proteins provided by the invention are suitable for use in methods of treating acute attacks associated with complement-mediated disorders, e.g., NMOSD AMR, and HAE events. These attacks may be long or short. In some embodiments, the disease or disorder is chronic. In some embodiments the compositions and methods of the invention are used prophylactically. Exemplary methods that the compositions may be used in include methods of treating a complement mediated disease including hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis, multifocal motor neuropathy.

### EXAMPLES

Other features, objects, and advantages of the present invention are apparent in the examples that follow.. Various changes and modifications within the scope of the invention will become apparent to those skilled in the art from the examples.

### Example 1. PEGylation of C1-INH

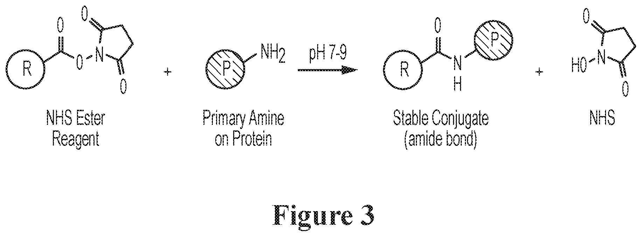

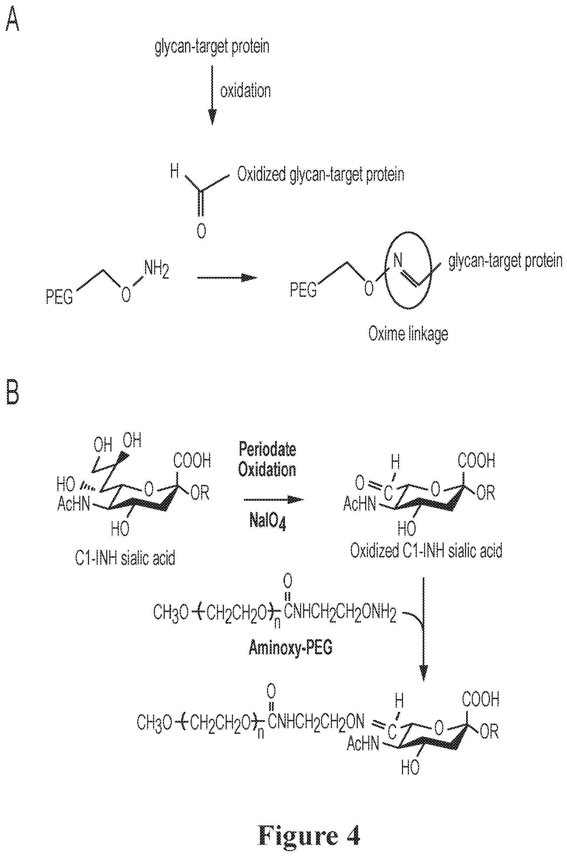

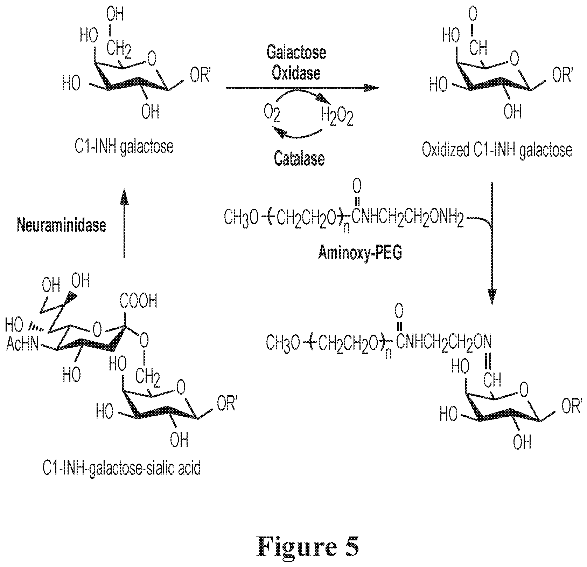

This example illustrates exemplary methods suitable for PEGylation of C1-INH proteins. Three different PEGylation strategies were explored. Exemplary PEGylation scheme are shown in Figures 3-5. These were conjugation of PEG to sialic acid residues (sialic acid mediated [SAM] chemistry), conjugation of PEG to galactose acid residues (galactose mediated [GAM] chemistry), and amine mediated conjugation of PEG.

Aminoxy-PEGs were utilized in order to form a more stable oxime linkage. PEGylation was performed utilizing techniques developed based on methods described in Park et al., Carbohydrate-Mediated Polyethylene Glycol Conjugation of TSH Improves Its Pharmacological Properties. Endocrinology, March 2013, 154(3): 1373-1383.

Exposed sialic acid residues on a glycosylated protein typically result in increased half-life compared to a protein with fewer or no sialic acid residues while terminal galactose residues on carbohydrate chains are known to cause receptor mediated clearance and decrease the serum half-life of proteins. Accordingly, initial efforts focused on GAM PEG conjugation in order to block receptor mediated clearance of C1 INH. While all three approaches appeared to be promising, amine and SAM PEGylation were surprisingly found to yield the greatest degree of C1-INH PEGylation with minimal and acceptable loss in potency. GAM PEGylation was less efficient and more heterogeneous in comparison.

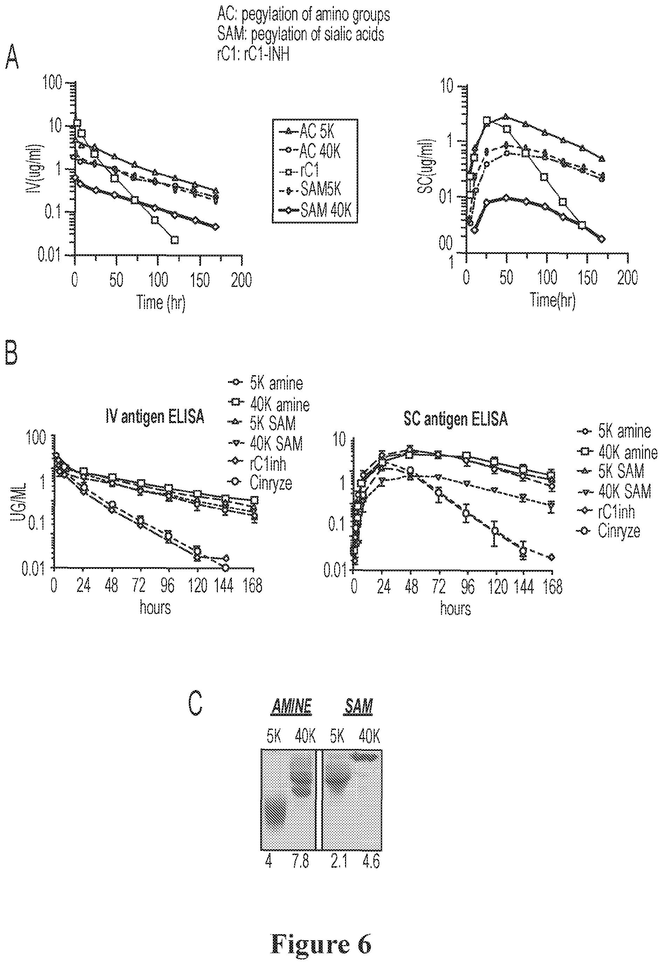

Initial in vivo PK study was conducted to evaluate PEGylated C1-INH. Specifically, SAM 5 KDa and 40 KDa PEGylated C1-INH was compared with amino PEGylated C1-INH in a rat PK study. See Figure 6, panels A-C. PEGylated C1-INH was quantified using an antigen assaying using a C1-INH to prepare the standard curve. The samples were also analyzed by Western blot to check for potential degradation. Doses of 1 mg/kg IV and 3 mg/kg were in the range of Cinryze^{®} in humans (2-3 mg/kg). These studies demonstrated that the PEGylated proteins had a 3-4 fold increase in half-life, likely due to a decrease in clearance.

Further pharmacokinetic studies were performed with C1-INH-PEG using 1 mg/kg intravenous administration to male SD rats. These data are presented in Table 1 below.

**Table 1. Pharmacokinetic parameters of C1-INH-PEG intravenously administered to male Sprague Dawley (SD) rats.**

| | | **C1-INH** | | | | | | | | | | | |
|---|---|---|---|---|---|---|---|---|---|---|---|---|---|
| | | **PEG-NHS** | | | | | | **PEG-Aminoxy; Sialic Acid Mediated (SAM)** | | | | | |
| **PK parameters** | **Unit** | **1K** | **2K** | **5K*** | **5K**** | **20K** | **40K** | **2K** | **' 5K*** | **5K**** | **10K** | **20K** | **40K** |
| CL | mL/da y/kg | 102 | 166 | 65.9 | 158 | 88.2 | 119 | 226 | 135 | 84.5 | 116 | 129 | 76.2 |
| Vss | mL/kg | 148 | 162 | 115 | 162 | 167 | 166 | 292 | 250 | 131 | 160 | 150 | 118 |
| Terminal t_{1/2} | day | 1.18 | 1.0 5 | 1.51 | 1.26 | 1.67 | 1.59 | 1.11 | 1.33 | 1.21 | 1.17 | 1.09 | 1.04 |
| AUCₗₐₛₜ | day^{∗}n g/ml | 9690 | 598 7 | 1485 7 | 6297 | 1090 8 | 8251 | 4375 | 7250 | 1179 5 | 8705 | 7760 | 1298 8 |
| AUC_{INF} | day^{∗}n g/ml | 9842 | 606 7 | 1533 4 | 6382 | 1141 4 | 8453 | 4430 | 7434 | 1201 3 | 8821 | 7816 | 1313 9 |
| MRT_{INF} | day | 1.46 | 0.9 75 | 1.74 | 1.02 | 1.90 | 1.40 | 1.29 | 1.85 | 1.56 | 1.38 | 1.15 | 1.54 |

| | | | | | | | | | | | | | |
|---|---|---|---|---|---|---|---|---|---|---|---|---|---|
| ^{∗}PEG is linear. ^{∗∗} PEG is branched | | | | | | | | | | | | | |

In addition, in NHP studies, subcutaneous bioavailability was observed to be about 30-40%, which was an unexpected improvement over an unconjugated recombinant C1-INH protein.

Therefore, PEGylated C1-INH appears to have increased half-life and sufficient bioavailability suitable for therapeutic use.

### Example 2. Exemplary PEGylation Protocols

### Process A

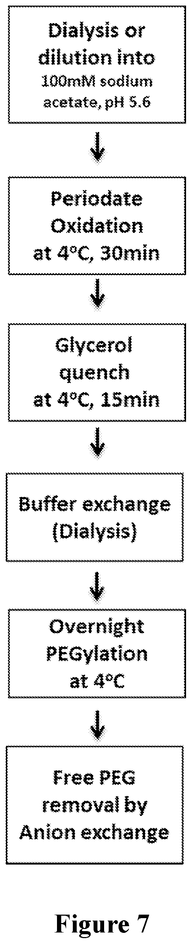

Purified C1-INH was dialyzed into 100 mM sodium acetate at pH 5.6. Periodate oxidation was carried out for 30 minutes at 4°C. The reaction was quenched with glycerol for 15 minutes at 4°C. The oxidized C1-INH was dialyzed into acetate buffer. The material was then PEGylated overnight at 4°C, followed by a glycine quench. Free PEG was removed by anion exchange. An exemplary schematic of Process A is provided in Figure 7.

C1-INH-PEG 40 KDa prepared by Process A, was further purified using the following method.

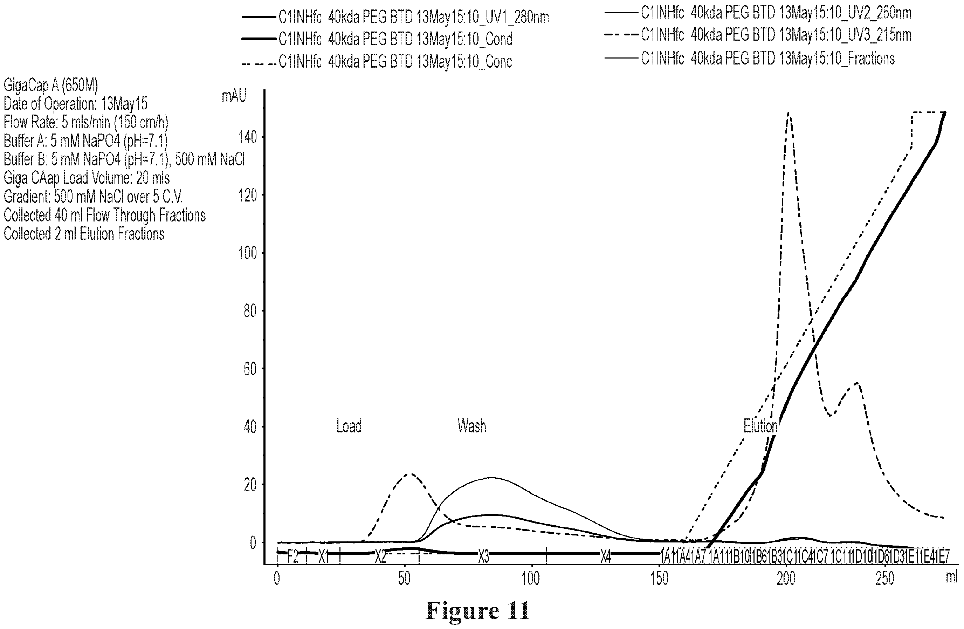

About 1 mg of 40 KDa PEG amine conjugated to C1-INH was diluted 20 fold with sample dilution buffer (5 mM NaPO4 at pH 7.00). The resulting solution exhibited a conductivity of 0.716 mS/cm. The sample was loaded onto a 10 mL GigaCap Q (650) column XK16. A flow rate was 150 cm/h for the entire process. The column was washed extensively with sample dilution buffer and the protein was eluted with a 10 column volume gradient to 500 mM NaCl. 2 mL fractions were collected and the samples analyzed by SDS-PAGE. The chromatography results are depicted in Figure 11. Peak fractions were then pooled and dialized into formulation buffer (50 mM Phosphate (pH=7.1), 150 mM Glycine, 50 mM Sorbitol), concentrated to ≥1.0 mg/ml, and quantitated by 280 nm absorbance (Nano-drop).

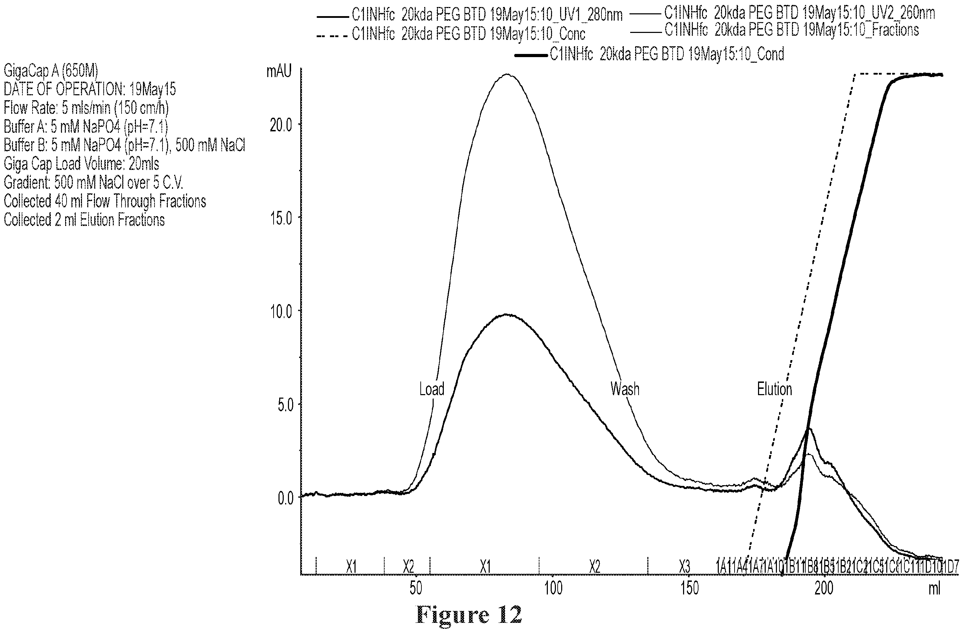

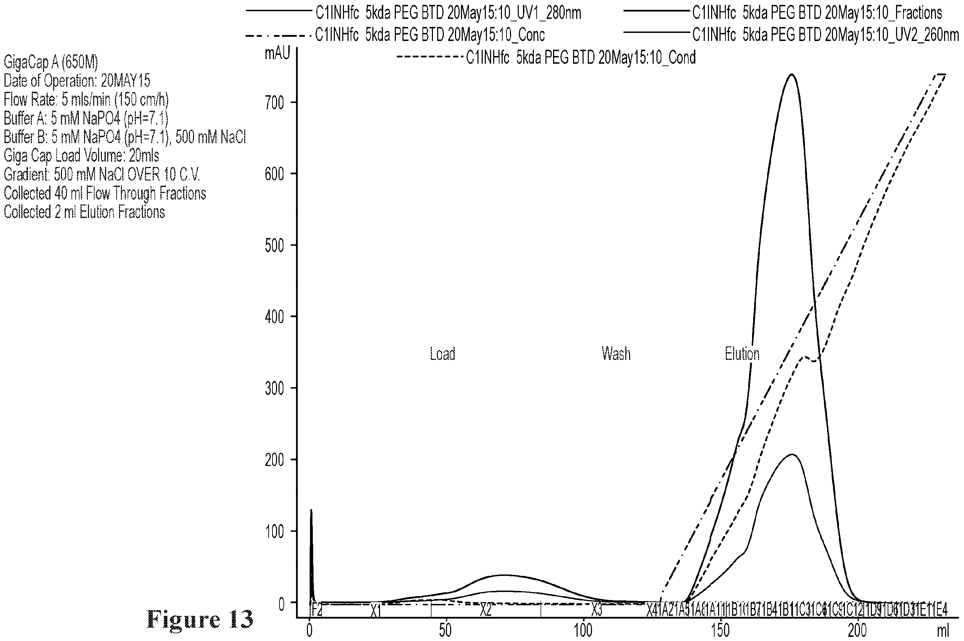

A similar purification was performed on a C1-INH-PEG 20 KDa preparation, depicted in Figure 12 and a C1-INH-PEG 5 KDa preparation, depicted in Figure 13.

Quantitation of all of the samples was performed on a nano-drop instrument using the extinction coefficient and molecular weight derived from the protein's amino acid sequence. The results are shown below in Table 2:

**Table 2: Quantitation of C1-INH PEGylation process samples.**

| | **Conc. (mg/ml)** | **Volume (ml)** | **Total Protein (mg)** | **Total Protein at Start (mg)** | **% Recovery** |
|---|---|---|---|---|---|
| **C1-INH - 40 kDa PEG** | 0.56 | 0.4 | 0.224 | 0.75 | 30 |
| **C1-INH - 20 kDa PEG** | 1 | 0.2 | 0.2 | 0.75 | 27 |
| **C1-INH** - **5 kDa PEG** | 2.2 | 0.15 | 0.33 | 0.75 | 44 |

### Process B

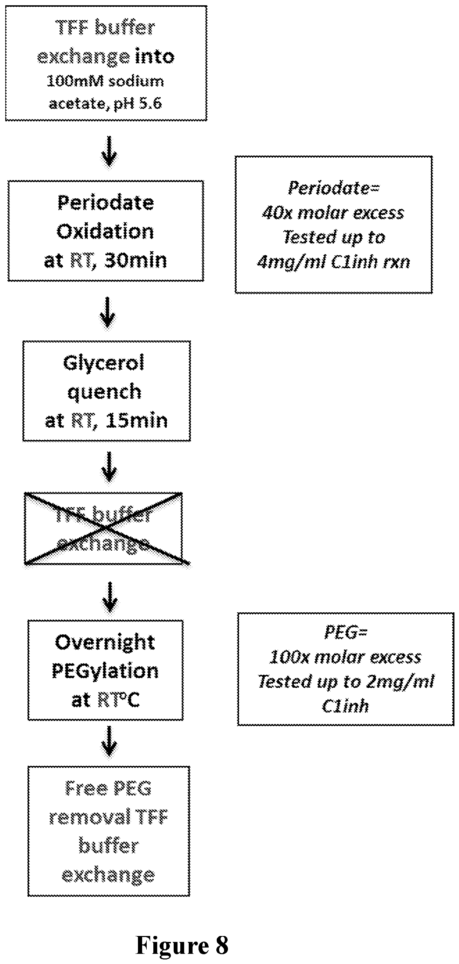

Purified C1-INH was exchanged into 100 mM sodium acetate at pH 5.6 via TFF buffer exchange. Periodate oxidation was carried out for 30 minutes at room temperature. Periodate was provided at 40x molar excess. Up to 4 mg/mL C1-INH was present in the reaction. The reaction was quenched with glycerol for 15 minutes at room temperature. The material was then PEGylated overnight at room temperature. PEG was provided at 100x molar excess. Up to 2 mg/mL C1-INH was present in the reaction. Free PEG was removed by TFF buffer exchange. An exemplary schematic of Process B is provided in Figure 8.

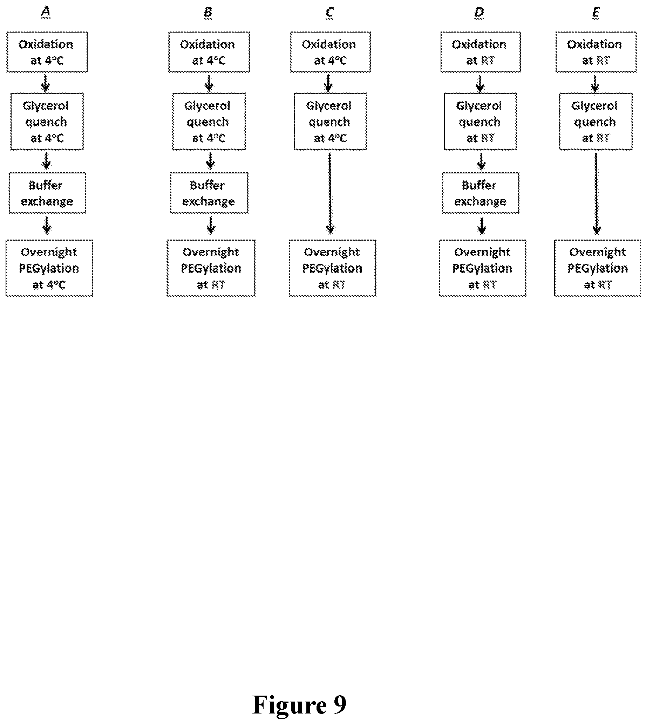

Other exemplary PEGylation protocols suitable for PEGylating C1-INH are summarized in Figures 9A-E.

### SAM Process PEG 5K

In this process, about 200 mL of octyl load material (∼0.9 mg/ml C1-INH in Tris/ammonium sulphate solution) was buffer exchanged into 100 mM sodium acetate, pH5.6 using Pellicon XL, Biomax, 30kDa (PES) TFF cassette with 10x diavolume exchange. 40 µM C1-INH (3.7 mg/ml) was treated with 1.6 mM sodium periodate (40x) for 30 minutes at room temperature with gentle stirring (50 ml reaction, in 100 mM sodium acetate, pH 5.6). The reaction was quenched with 1.5 % glycerol for 15 minutes at room temp.

21.6 uM C1-INH (2mg/ml) was treated with 2.16 mM 5kDa-PEG (100x) gently stirring overnight at room temp (92.5 ml reaction, in 100mM sodium acetate, pH 5.6). The reaction was then quenched with 2.16 mM glycine (100x) for 1 hour at room temperature.

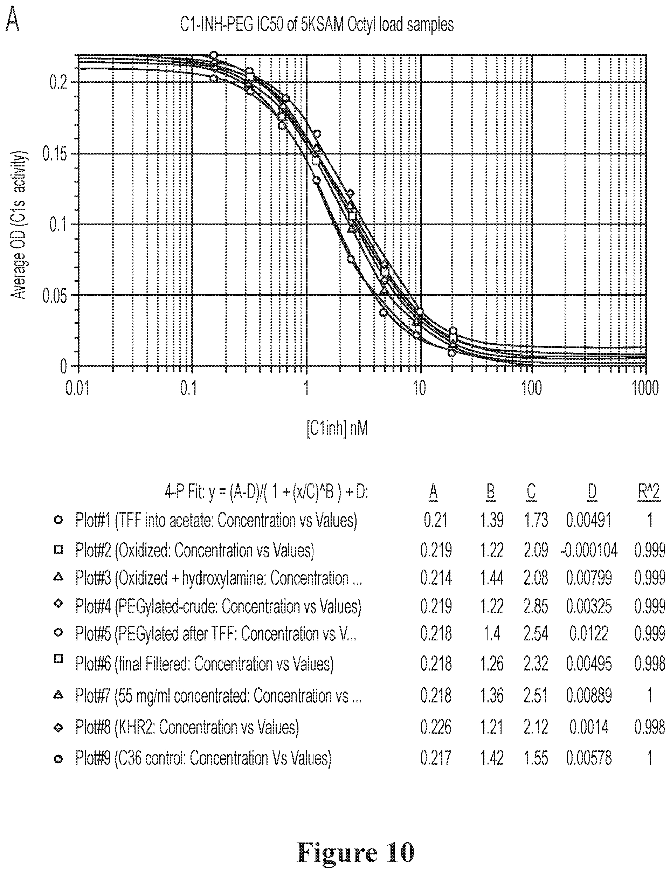

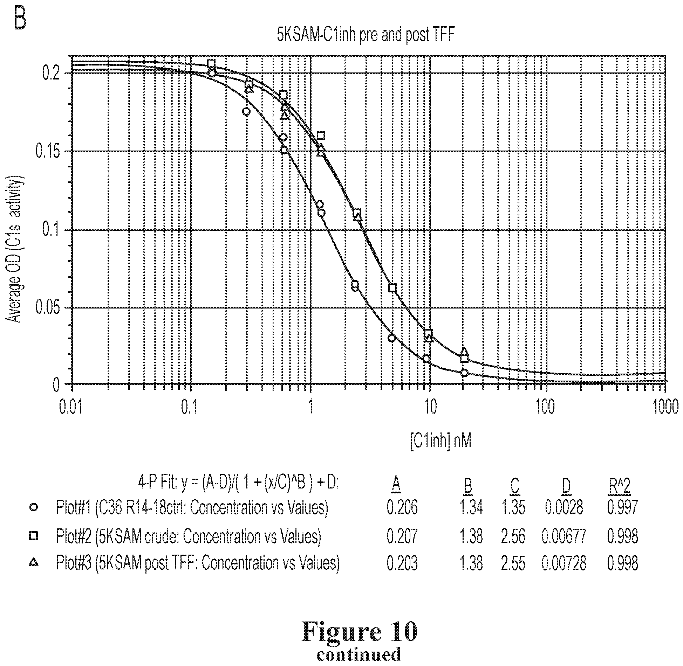

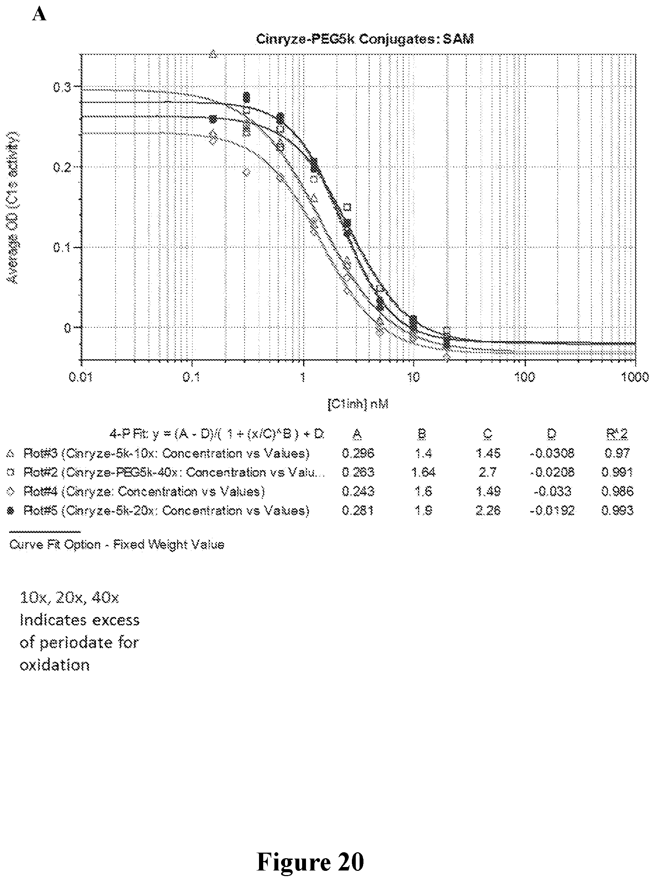

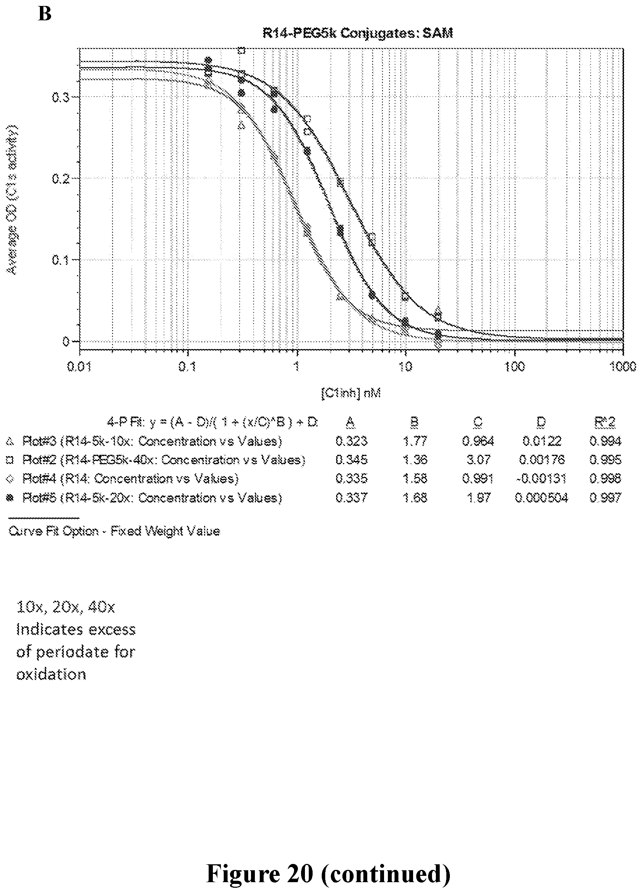

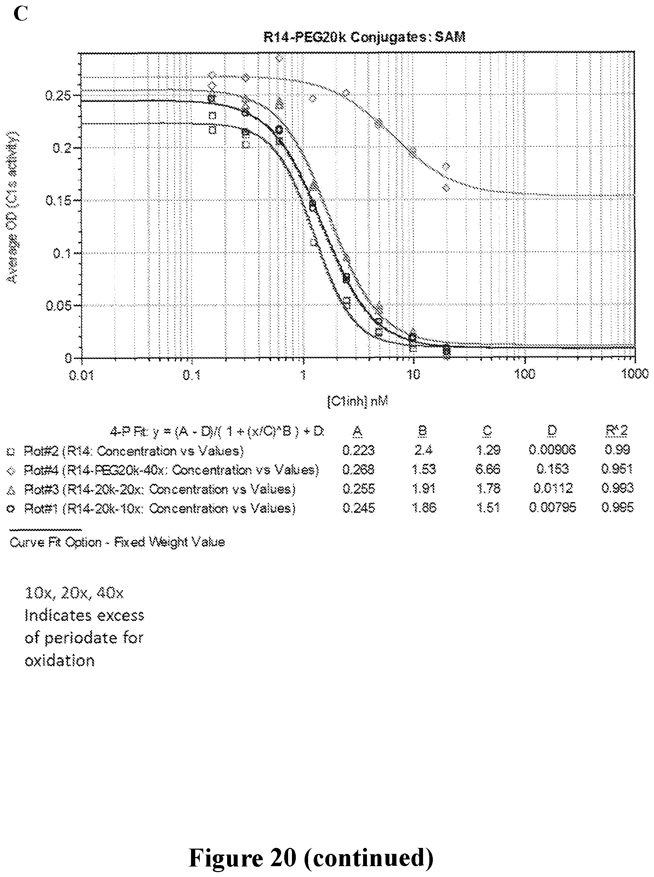

TFF diafiltration removal of free PEG was done using a Pellicon XL, Biomax 100kDa MWCO (PES) TFF cassette with 10x diavolume exchange into 50 mM sodium phosphate, 150 mM glycine, and 50 mM sorbitol, at pH 7.1. The product was then filter sterilized using a .22 uM, PES, Millipore steriflip filter. The IC50 of the PEGylated samples are shown in Figure 10, panels A and B, and in Figure 20, panels A-B.

The yield after each process step is presented below in Table 3:

**Table 3: Octyl load PEGylation step yields.**

| **Step** | **volume (ml)** | **concentration (mg/ml)** | **total (mg)** | **recovery (%)** |
|---|---|---|---|---|
| **Octvl load** | 200 | .93 | 186 | - |
| **TFF into acetate** | 44 | 4.2 | 184.8 | 99.0 |
| **Oxidation** | 50 | 3.6 | 180 | 97.2 |
| **PEGvlation** | 92.5 | 2 | 185 | 100 |
| **TFF to storage buffer** | 20.8 | 8.25 | 171.6 | 93.1 |
| **82.5 mg, sterile filtration** | 9.7 | 8 | 77.6 | 94.0 |
| | | | | **84** |

### SAM Process PEG linear 2K, 5K, branched 5K, 10K, 20K, 40K

The SAM process was also used to prepare C1-INH-PEG with the following kinds of PEG: linear 2K, linear 5K, branched 5K, branched 10K, branched 20K, and branched 40K.

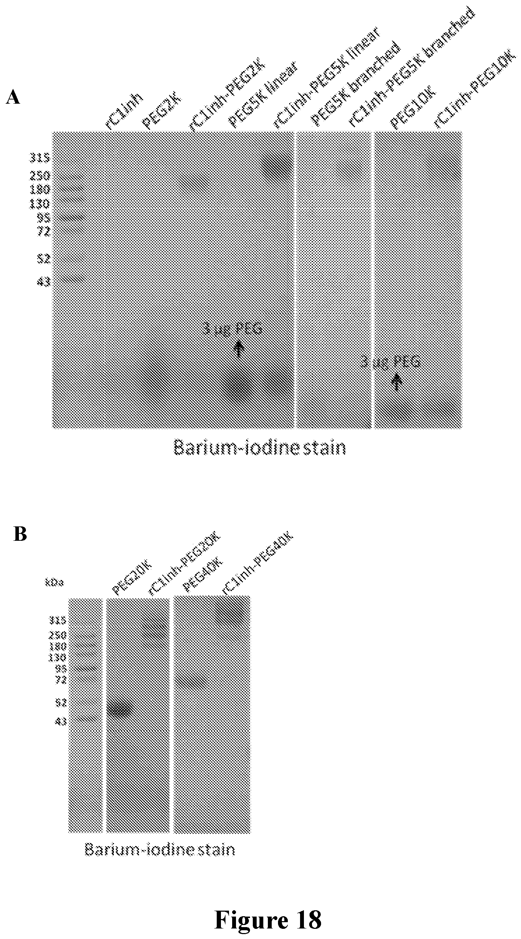

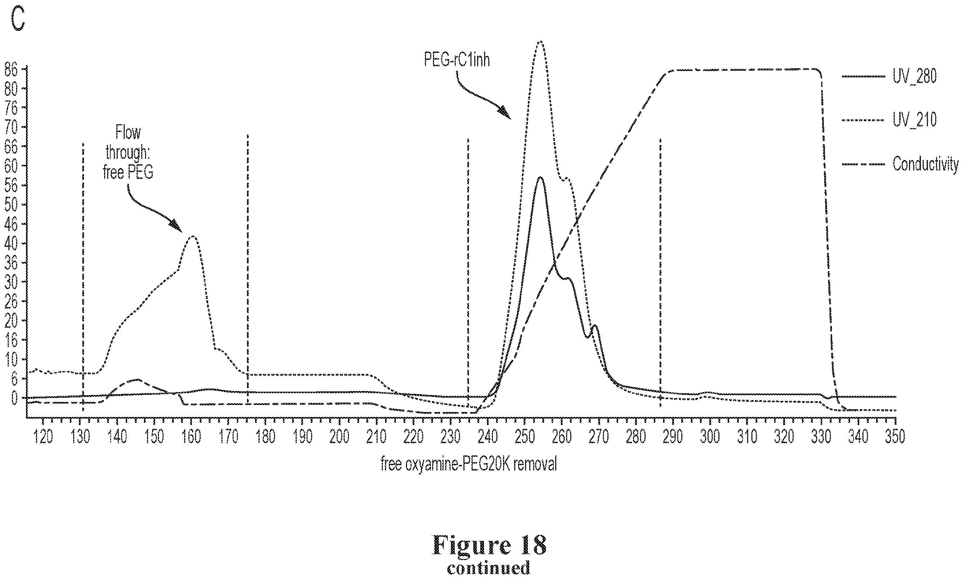

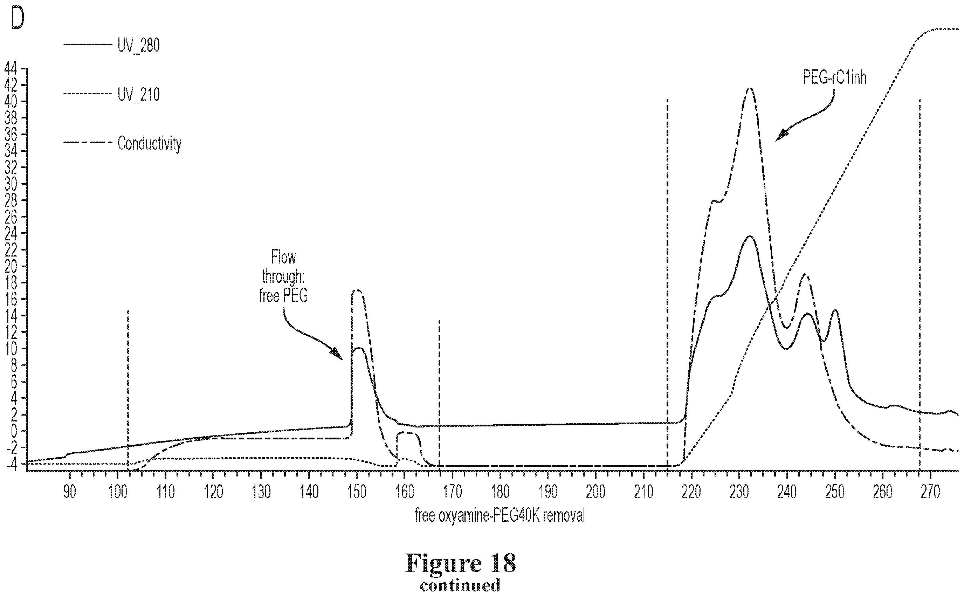

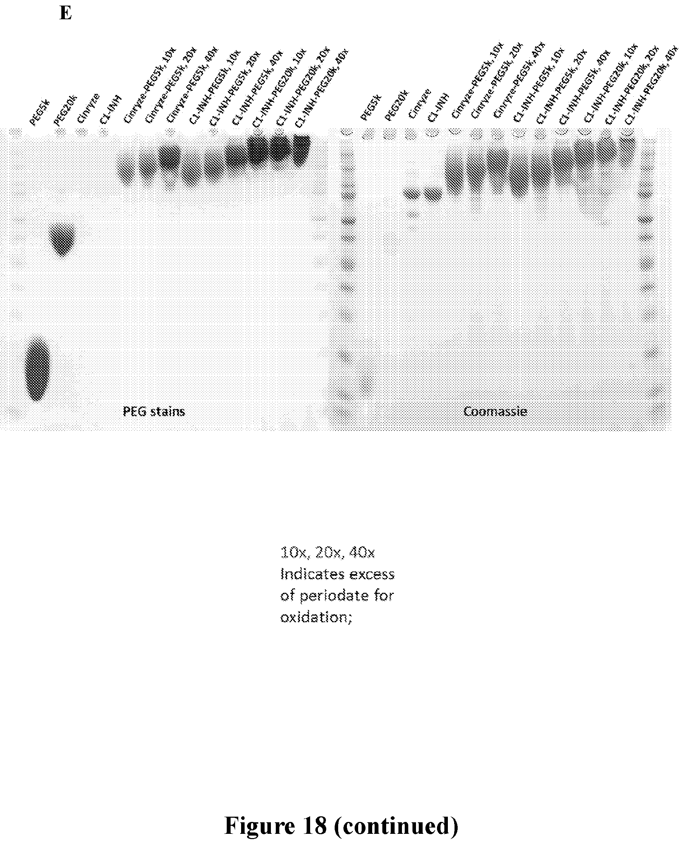

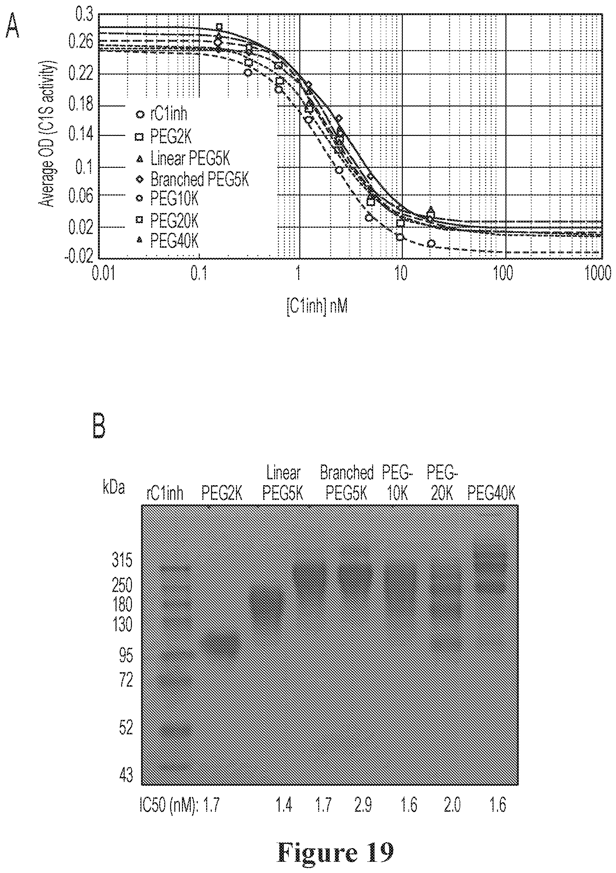

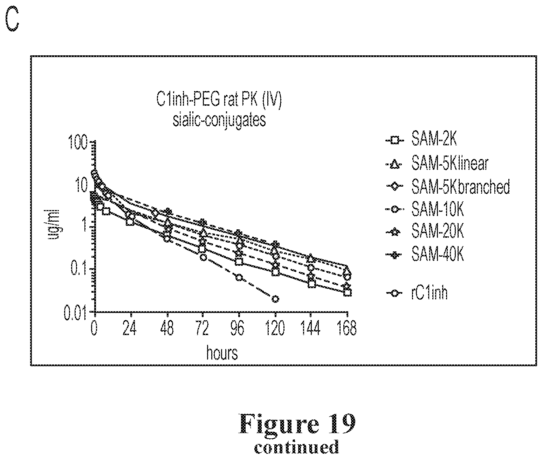

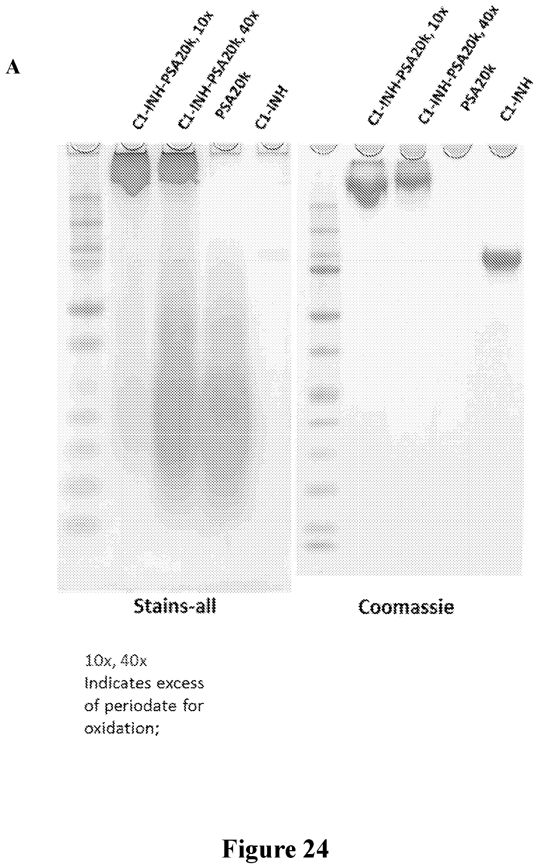

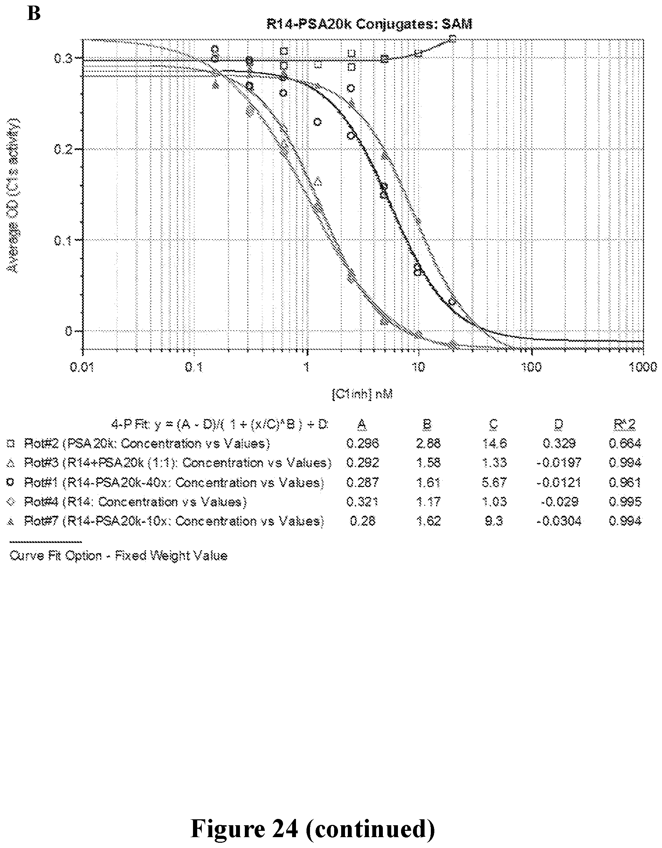

C1-INH PEGylated with PEG 2K, 5K and 10 K were purified with Amicon centrifugal filter (cut-off 30K). C1-INH PEGylated with PEG-aminoxy 20K or 40K was purified by AKTA system for free PEG removal. Characterization of the C1-INH is shown in Figure 18, panels A-E. C1-INH-PEG produced by the SAM process was assayed for purity and potency, and PK was evaluated in rat models. These data are presented in Figure 19, panels A-C. Additional characterization and IC50 values of the PEGylated samples are shown in Figure 24, panels A and B.

The SAM PEGylation conditions for C1-INH-PEG generation is shown in Table 4 below.

**Table 4. SAM PEGylation conditions for C1-INH-PEG generation.**

| **PEG Mw** | **Oxidation step** | | **Conjugation step** | |
|---|---|---|---|---|
| | **rClinh Conc. (mg/ml)** | **NaIO₄ equivalent** | **Protein conc. (mg/mL)** | **PEG equivalent** |
| Linear 2K | 5 | 20 | 5 | 100 |
| Linear 5K | 5 | 20 | 2 | 100 |
| Branched 5K | 5 | 20 | 2 | 100 |
| Branched 10K | 5 | 10 | 3.5 | 100 |
| Branched 20K | 5 | 5 | 2 | 100 |
| Branched 40K | 5 | 5 | 2 | 100 |

### PEGylation via Amine Coupling Process

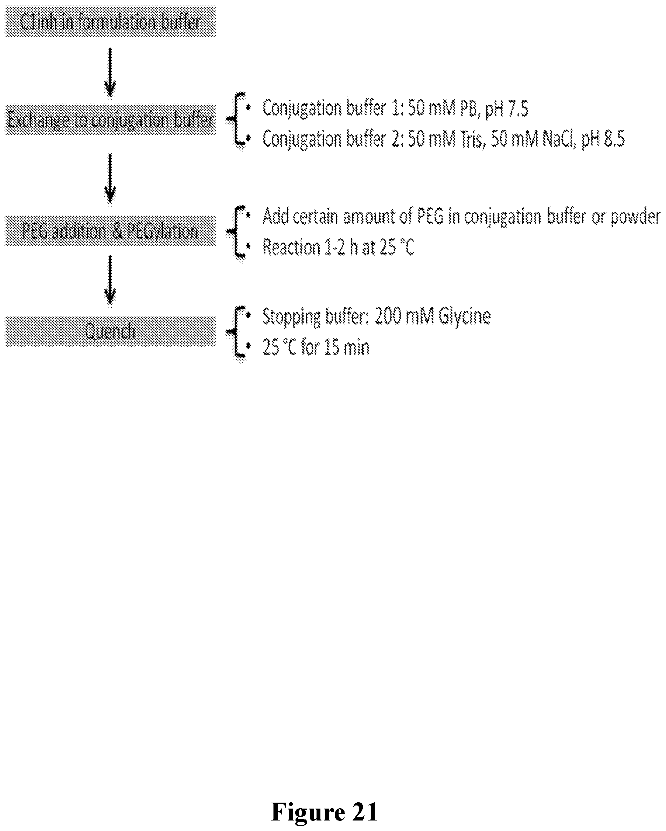

C1-INH-PEG was also prepared with an amine coupling process. A schematic representation of an exemplary PEGylation via amino coupling process is depicted in Figure 21.

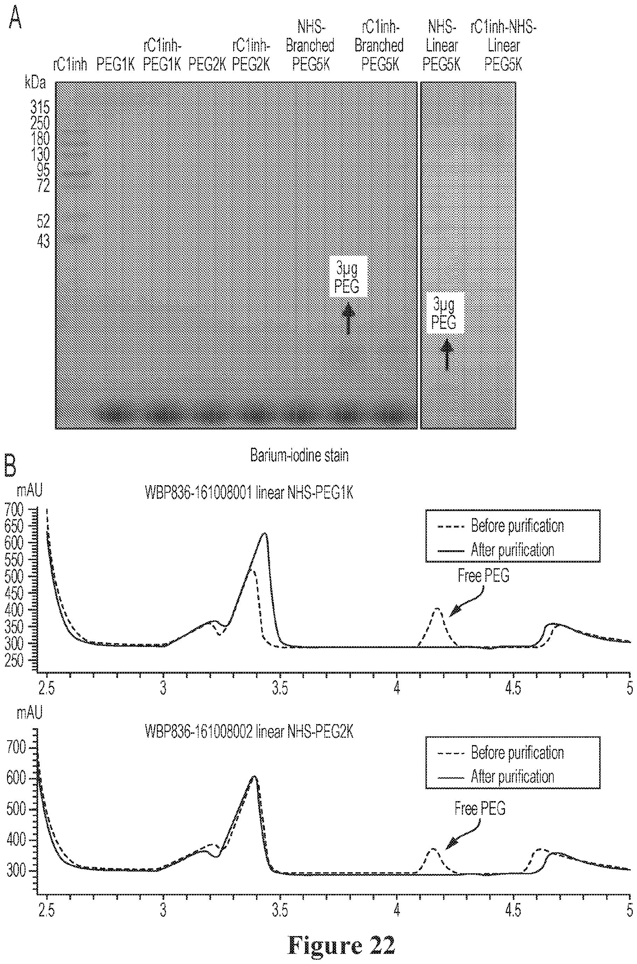

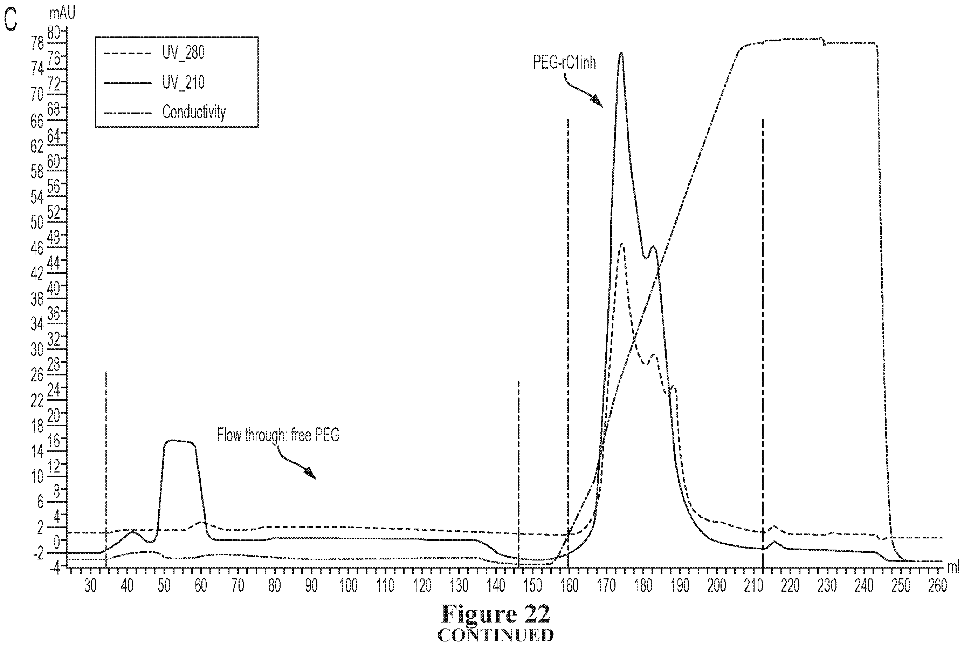

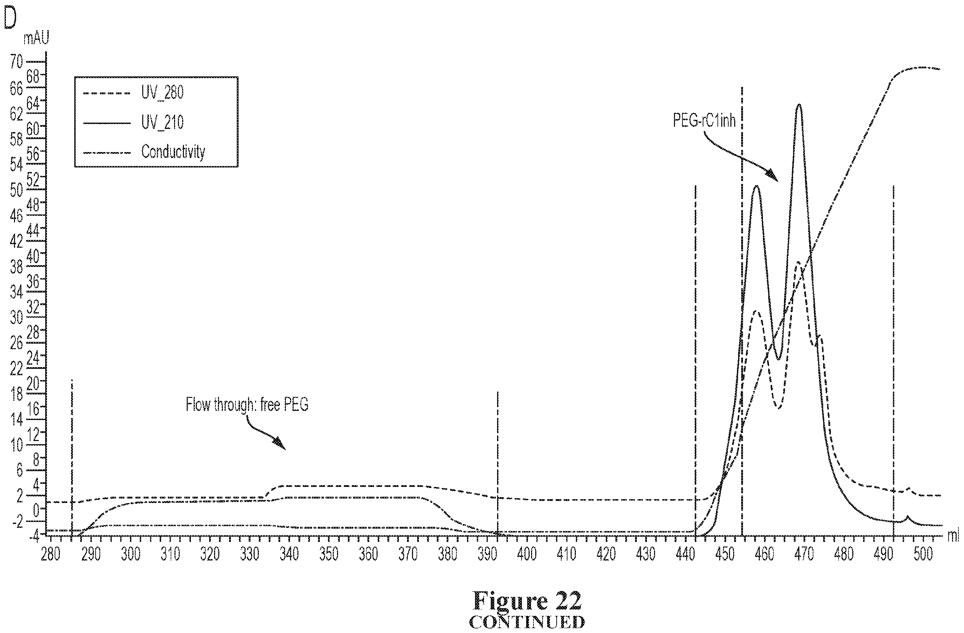

C1-INH PEGylated with PEG1K, linear 5K and branched 5K were purified by Amicon centrifugal filter (cut-off 30K). A barium-iodine stain was used to detect free PEG for PEG5K moieties, and RP-HPLC was utilized to detect free PEG1K and 2K. C1-INH PEGylated with NHS-PEG20K and 40K were purified by the AKTA pure chromatography system. Characterization of the PEGylated C1-INH is shown in Figure 22, panels A-D.

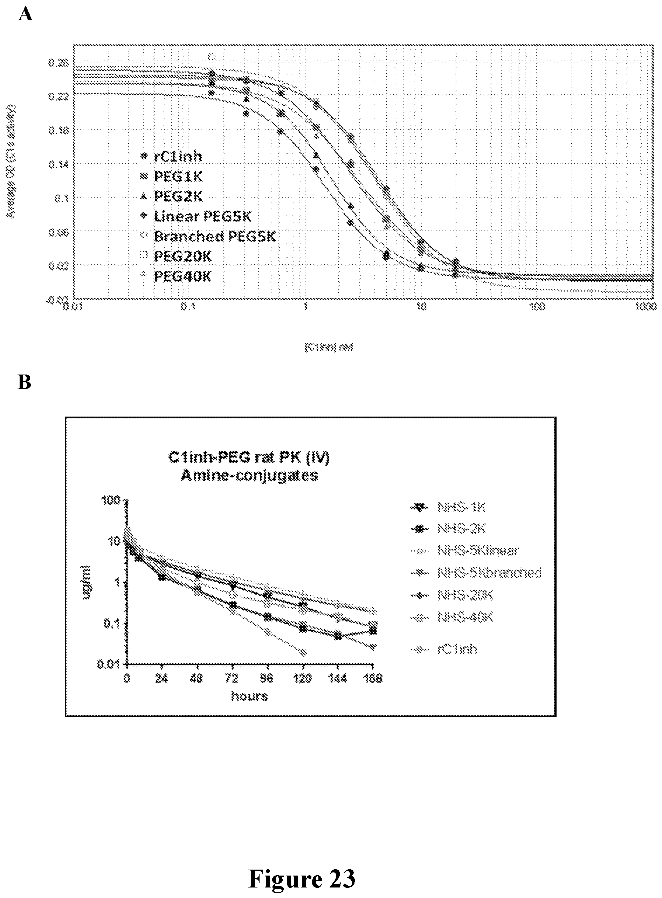

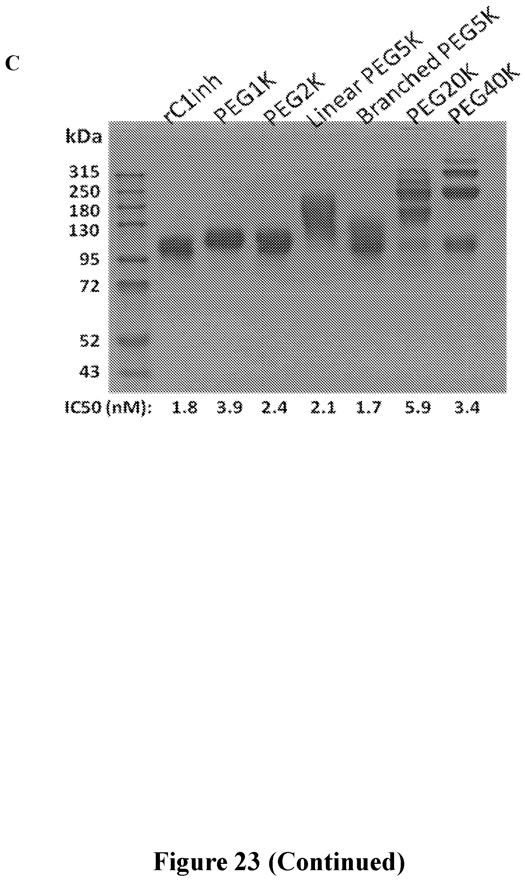

C1-INH-PEG produced by the amine coupling process was assayed for purity, potency, and PK was evaluated in a rat model. These data are presented in Figure 23, panels - C.

The PEGylation conditions for C1-INH-PEG generation via the amine coupling process is shown in Table 5 below.

**Table 5. PEGylation conditions for C1-INH-PEG generation via the amine coupling process.^{∗}PEGylation with 100 x PEG20K had a low conversion ratio and was reprocessed with another 40X PEG20K.**

| **PEG MW** | **Protein conc. (mg/mL)** | **PEG equivalent** | **pH** | **Temp. (°C)** | **Time (h)** |
|---|---|---|---|---|---|
| Linear 1K | 5 | 10 | 7.5 | 25 | 1 |
| Linear 2K | 5 | 5 | 7.5 | 25 | 1 |
| Linear 5K | 5 | 10 | 7.5 | 25 | 1 |
| Branched 5K | 5 | 150 | 8.5 | 25 | 1 |
| Branched 20K | 5 | 100+40* | 8.5 | 25 | 2+1 * |
| Branched 40K | 2 | 100 | 8.5 | 25 | 2 |

### Example 3: Non-Human Primate PK Study of IV Administered PEGylated C1-INH

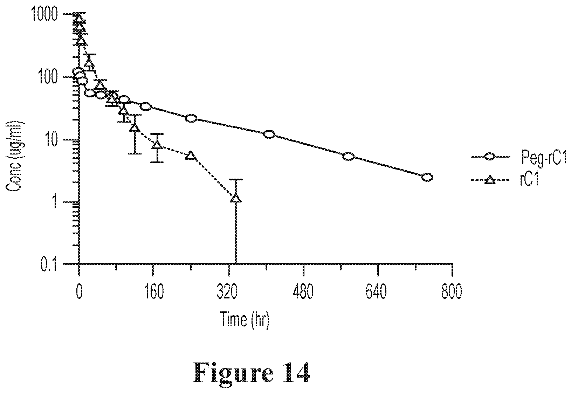

Non-human primates (NHP) (cynomolgus monkeys) were divided into two groups and intravenously dosed with recombinant human C1-INH (rhC1-INH) at 30 mg/kg or PEGylated rhC1-INH at 5 mg/kg. Exemplary results of the study are summarized in Figure 14 and Table 6.

In NHP, PEGylated rhC1-INH displayed 6-fold lower clearance and 3-fold longer terminal half-life compared to rhC1-INH. A similar trend was also observed in rat studies, which showed a 4-fold decrease in clearance and a 4-fold increase in half-life.

**Table 6: NHP PK Study of PEGylated rhC1 INH v. rhC1 INH results**

| **NHP, IV** | **Dose (mg/kg)** | **n** | **CL (mL/hr/kg)** | **Vz (mL/kg)** | **T_{1/2} (hr)** |
|---|---|---|---|---|---|
| hrC 1-inh | 30 | 3 | 1.9 | 143 | 54 |
| Peg-hrC 1-inh | 5 | a 2 | 0.3 | 75 | 161 |

| | | | | | |
|---|---|---|---|---|---|
| ^{a}one of the three monkeys in the study showed increased elimination rate after 408 hr and was not included in PK calculation | | | | | |

### Influence of PEG Load on PK of NHP Administered IV C1-INH

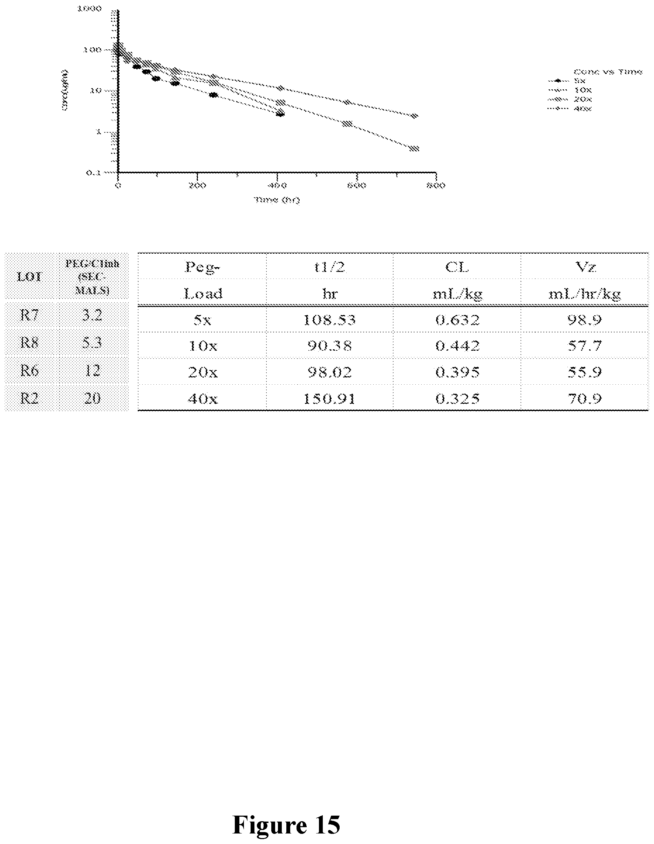

Further PK studies were conducted with NHP. NHP received IV administered C1-INH-PEG at 5x, 10x, 20x and 40x loads. Exemplary results are shown in Figure 15.

### Example 4: NHP IV v. SC PK of PEGylated C1-INH

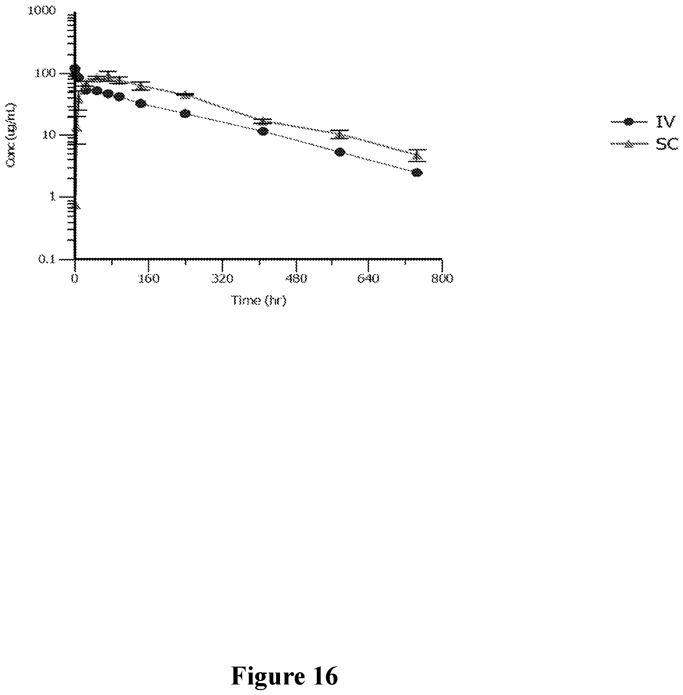

NHP were divided into two groups and intravenously dosed with PEGylated C1-INH at 5 mg/kg or subcutaneously (SC) dosed with PEGylated C1-INH at 10 mg/kg. The results of the study are summarized in Figure 16 and Table 7. Functional activity (SA = 4.8 U/mg) of the PEGylated C1-INH was maintained over the time course of the study.

Significantly and unexpectedly, in NHP, PEGylated C1-INH exhibited a bioavailability of 85%, with half-life comparable to that of IV administration. The preclinical data collected thus far supports potential for once weekly or even less frequent dosing.

**Table 7: IV v. SC dosing of PEGylated rhC1-INH in NHP**

| | **Dose (mg/kg)** | **n** | **Cmax (ug/mL)** | **Tmax (hr)** | **AUCinf (ug/mL-hr)** | **F (%)** |
|---|---|---|---|---|---|---|
| IV | 5 | 2 | - | - | 15144 | - |
| SC | 10 | 3 | 94 | 72 | 25599 | 85 |

| | | | | | | |
|---|---|---|---|---|---|---|
| F = 58% for hrC1-inh in NHP following SC dosing | | | | | | |

### Example 5: Oxidation/titration to test minimal PEG to maximized PK profile

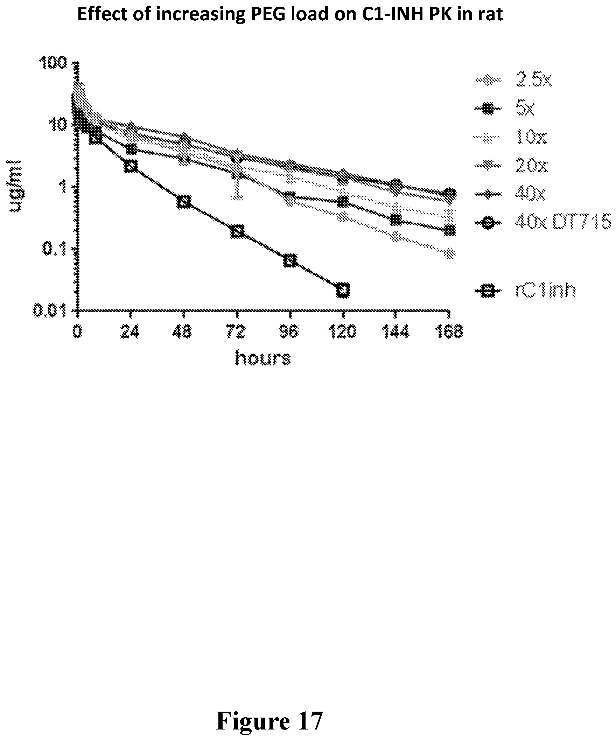

The DT-1215 titer assay used was an ELISA based method which captures PEG-rC1-INH protein from serum samples with an anti-PEG antibody. The protein was then detected with a labeled anti-C1-INH protein. PEG-rC1-INH was used to prepare the standard curve. Figures 17 depicts the results of a DT-1215 titer analysis and sample specific activity. Tables 8 and 9 provide further data.

**Table 8: The change in specific activity observed at different levels of periodate treatment. As seen in Table 9 (below) the change in the periodate level resulted in a different ratio of PEG to C1 INH.**

| **group** | **lot** | **sample** | **IC50 (nM)** | **% relative potentcy (vs parent)** | **specific activity (U/mg)** |
|---|---|---|---|---|---|
| A | KHR3 | **2.5x** | 1.52 | 92.11 | **6.54** |
| B | KHR3 | **5x** | 1.61 | 86.96 | **6.17** |
| C | KHR3 | **10x** | 1.61 | 86.96 | **6.17** |
| D | KHR3 | **20x** | 1.77 | 79.10 | **5.62** |
| E | KHR2 | **40x** | 2.05 | 68.29 | **4.85** |
| | | | | | |
| | C36R14-18 | parent | 1.4 | 100 | 7.1 |

**Table 9: The half-life achieved at different levels of PEG compared to the unconjugated C1 INH.**

| **Sample** | **t_{1/2} (hr)** | **% of Longest t_{1/2}** | **%rC1 Activity** | **PEG/rC1 mol/mol** |
|---|---|---|---|---|
| C1-INH | 13 | 33 | 100 | NA |
| 2.5x | 25.5 | 64.5 | 92 | 2 |
| 5x | 29.5 | 74.7 | 87 | 3 |
| 10x | 32.0 | 81.0 | 87 | 8 |
| 20x | 39.5 | 100 | 79 | 14 |
| 40x | 38.9 | 98.5 | 68 | 20 |

### Example 6: Physical Characterization of PEGylated C1-INH

Purity of PEGylated preparation was analyzed using SEC and SEC-MALs. CD spectra of 0.1 mg/ml PEG-C1-INH proteins were measured at 25°C. CD data were processed by AVIV and CDNN softwares. No significant change is observed when proteins are PEGylated based on the CD spectra and secondary structure analysis. According to the C-terminal crystal structure of C1-INH (20AY), 27 % helical and 30% beta-sheet.

**Table 10: Data demonstrates that PEGylation Does Not Alter C1-INH Secondary Structure.**

| | **C1-INH** | **A 5K Amine PEG-C1-INH** | **B 40K AMINE PEG-C1-INH** | **C 5K SAM PEG-C1-INH** | **D 40K SAM PEG-C1-INH** | **E 5K SAM PEG-C1-INH** |
|---|---|---|---|---|---|---|
| Helix | 31.30% | 29.60% | 33.10% | 29.60% | 29.60% | 32.20% |
| Antiparallel | 10.00% | 11.20% | 8.20% | 11.50% | 11.50% | 9.10% |
| Parallel | 9.00% | 9.60% | 8.80% | 9.50% | 9.50% | 8.90% |
| Beta-Turn | 17.30% | 17.50% | 16.90% | 17.60% | 17.60% | 17.10% |
| Rndm. Coil | 34.00% | 35.90% | 33.50% | 35.50% | 35.50% | 33.80% |
| Total Sum | 101.60% | 103.70% | 100.50% | 103.60% | 103.60% | 101.00% |

The melting temperature (Tm) of PEGylated C1-INH was measured by nanoDSF. PEGylation was found not to dramatically change C1-INH thermal stability. The Tm of 40 KDa amino-PEGylated C-INH was measured to be 2°C higher than the other conjugates tested. The data are presented in Table 11.

**Table 11: Tm analysis of PEGylated C1-INH.**

| **Sample ID** | **Sample Description** | **Sample Lot#** | **Inflection Point #1 for Ratio (Unfolding)** |
|---|---|---|---|
| A1 | 5K Amine PEG C1-INH | CS19875 | 57.7°C |
| B1 | 40K Amine PEG C1-INH | CS19876 | 59.5°C |
| C1 | 5K SAM PEG C1-INH | CS19877 | 57.0°C |
| D1 | 40K SAM PEG C1-INH | CS19878 | 57.4°C |
| E1 | 5K SAM PEG C1-INH | SK-SAM-C 1-INH-KH-R1 | 56.5°C |
| C1-INH1 | C1 INH | SHIRE DT615 | 57.3°C |

Nuclear magnetic resonance (NMR) was used to characterize the PEGylation level. PEGylation on amine was low, about 3 PEG moeities per C1-INH. Sialic acid can be heavily PEGylated to reach saturation for the 5K PEG reactant. 40K PEGylated on sialic acid reaches ∼9 PEGs per molecule. PEGylated level was quantified at different periodate concentration. The data is presented in Table 12.

**Table 12: NMR characterization of PEGylated C1-INH preparations.**

| **Sample name** | **PEG/C1-INH Ratio** | **PEG-C1-INH MW*** | **Comments** |
|---|---|---|---|
| A | 3.2 | 101 | 5K Amine PEG |
| B | 3.2 | 213 | 40K Amine PEG |
| C | 28.3 | 226.5 | 5K SAM PEG |
| D | 9.3 | 457 | 40K SAM PEG |
| R1 | 25.3 | 211.5 | 5K SAM PEG |
| R2 | 21.2 | 191 | 5K SAM PEG |
| R3A | 2.5 | 97.5 | 2.5X Periodate |
| R3B | 5 | 110 | 5X Periodate |
| R3C | 11.5 | 142.5 | 10X Periodate |
| R3D | 19.5 | 182.5 | 20X Periodate |
| R4 | 21.2 | 191 | TFF process |

### Example 7: Characterization of C1-INH-PSA

C1-INH was conjugated with polysialic acid (PSA) via the sialic acid mediated (SAM) process. Characterization of the C1-INH-PSA produced by the SAM process was assayed for purity and potency. These data are presented in Figure 24, panels A and B. The data indicate that while free PSA does not interfere with the potency assay itself, CL-INH potency was reduced by ~4-7 fold under the PSA:C1INH conditions tested here.

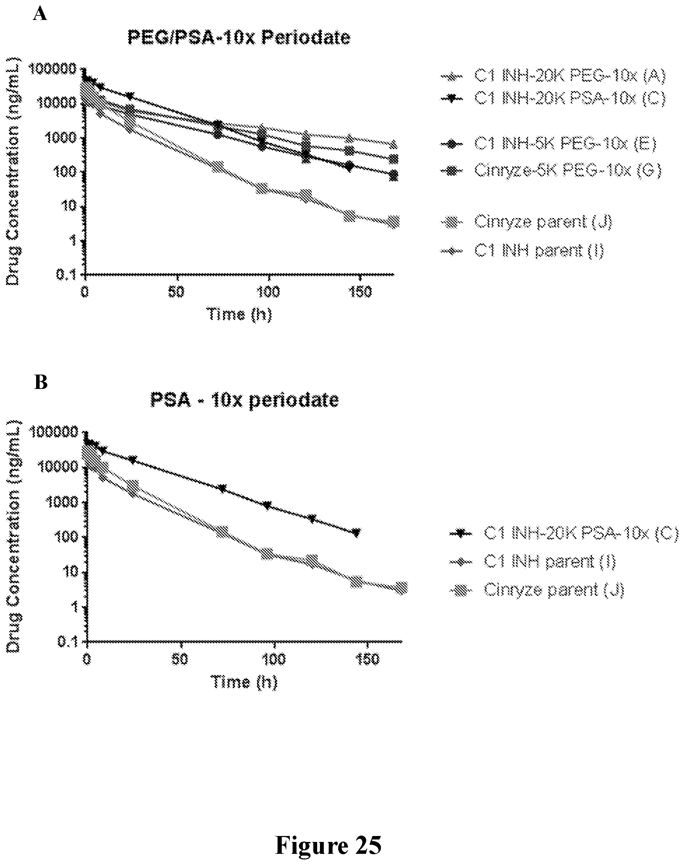

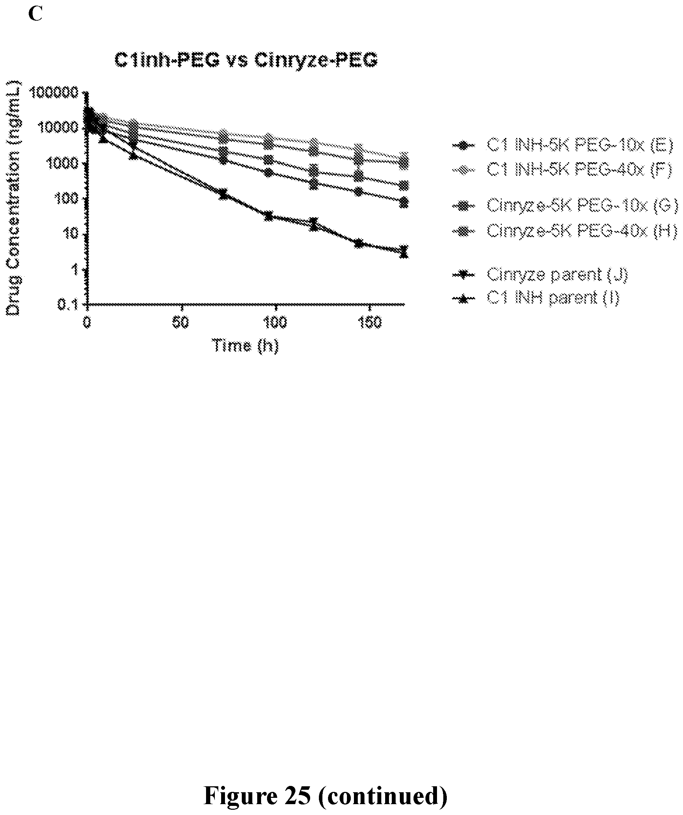

PK studies were performed in rat using C1-INH-PSA, C1-INH-PEG, and Cinryze^{®}-PEG. The data are presented in Figure 25, panels A-C.

## Claims

1. A composition comprising a conjugated C1 esterase inhibitor (C1-INH) comprising:
(i) a C1-INH protein comprising at least one polyethylene glycol (PEG) moiety; and wherein the at least one PEG moiety is covalently linked to the C1-INH protein via an oxime linkage, wherein the oxime linkage is between the PEG moiety and a glycan residue of C1-INH, wherein the glycan residue is a sialic acid residue, and wherein the conjugated C1-INH has an extended *in vivo* half-life compared to unconjugated C1-INH; or
(ii) a C1-INH protein comprising at least one glycan residue; and
at least one polysialic acid (PSA) moiety,
wherein the at least one polysialic acid (PSA) moiety is covalently linked to the C1-INH protein via an oxime linkage, wherein the oxime linkage is between the PSA moiety and a glycan residue of C1-INH, wherein the glycan residue is a sialic acid residue, and wherein the conjugated C1-INH has an extended *in vivo* half-life compared to unconjugated C1-INH.

2. The composition of claim 1(i), wherein:
(i) the C1-INH protein is recombinantly produced or plasma derived;
(ii) the C1-INH protein comprises a C1-INH domain having an amino acid sequence of SEQ ID NO: 1, SEQ ID NO:2, SEQ ID NO:37, or SEQ ID NO:38; and/or
(iii) the C1-INH protein is a fusion protein, wherein the fusion protein optionally comprises (a) an Fc domain directly or indirectly fused to a C1-INH domain, such as wherein the Fc domain is derived from IgG1 and/or the Fc domain comprises amino acid substitutions corresponding to L234A and L235A according to EU numbering; or (b) an albumin domain directly or indirectly fused to a C1-INH domain.

3. The composition of claim 1(i) or claim 2, wherein:
(i) the C1-INH protein has a glycosylation profile comprising no more than 50%, 45%, 40%, 35 %, 30%, 25%, 20%, 15 %, 10%, or 5% neutral glycan species, prior to PEGylation;
(ii) the C1-INH protein has a glycosylation profile comprising between 5% and 25% neutral glycan species, prior to PEGylation;
(iii) the C1-INH protein comprises, on average, at least 30%, 35%, 40%, 45%, 50%, 55%, 60%, 65%, 70%, 75%, 80%, 85%, 90%, 95%, 96%, 97%, 98%, 99%, or 100% charged glycans per molecule;
(iv) the C1-INH protein contains less than 20%, 15%, 10%, or 5% of one or more of mannose, α-galactose, NGNA, or oligomannose-type glycosylation, prior to PEGylation;
(v) prior to PEGylation, the C1-INH protein has a glycosylation profile comprising one or more of the following:
between 5% and 30% neutral glycan species;
between 10% and 30% mono-sialylated glycan species;
between 30% and 50% di-sialylated glycan species;
between 15% and 35% tri-sialylated glycan species; or
between 5% and 15% tetra-sialylated glycan species;
(vi) prior to PEGylation, the C1-INH protein has a glycosylation profile comprising:
no more than 30% neutral glycan species;
between 20% and 30% mono-sialylated glycan species;
between 30% and 40% di-sialylated glycan species;
between 10% and 20% tri-sialylated glycan species; and
between 5% and 10% tetra-sialylated glycan species;
(vii) the C1-INH protein comprises, on average, at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 sialylated glycan residues per molecule; and/or
(viii) the C1-INH protein comprises, on average, at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 mole sialic acid per mole of protein.

4. The composition of any one of claims 1(i), 2 or 3, wherein:
(i) the PEG has a molecular weight between 1 KDa and 50 KDa, between 1 KDa and 40 KDa, between 5 KDa and 40 KDa, between 1 KDa and 30 KDa, between 1 KDa and 25 KDa, between 1 KDa and 20 KDa, between 1 KDa and 15 KDa, between 1 KDa and 10 KDa, or between 1 KDa and 5 KDa;
(ii) the PEG has a molecular weight of 1 KDa, 5 KDa, 10 KDa, 15 KDa, 20 KDa, 25 KDa, 30 KDa, 35 KDa, 40 KDa, 45 KDa, or 50 KDa;
(iii) the conjugated C1-INH has a PEG/C1-INH ratio of between 1 to 25, between 1 to 20, between 1 to 15, between 1 to 10, or between 1 to 5;
(iv) the conjugated C1-INH has a half-life comparable or greater that than a plasma derived human C1-INH;
(v) the conjugated C1-INH has a half-life in the range of 100%-500% of the half-life of the plasma derived C1-INH;
(vi) the conjugated C1-INH has a half-life of at least 70, 75, 80, 85, 90, 95, 100, 105, 110, 115, 120, 125, 130, 135, 140, 145, 150, 155, 160, 165, or 170 hours;
(vii) the conjugated C1-INH has a half-life of at least 3, 4, 5, 6, 7, 8, 9, 10, 11, 12, 13, or 14 days; and/or
(viii) the conjugated C1-INH has a specific activity in the range of 50%-150% of the specific activity of plasma derived human C1-INH.

5. The composition of claim 1(ii), wherein:
(i) the C1-INH protein is recombinantly produced or plasma derived;
(ii) the C1-INH protein comprises a C1-INH domain having an amino acid sequence of SEQ ID NO:1, SEQ ID NO:2, SEQ ID NO:37, or SEQ ID NO:38; and/or
(iv) the C1-INH protein is a fusion protein, optionally wherein the fusion protein comprises (a) an Fc domain directly or indirectly fused to a C1-INH domain, such as wherein the Fc domain is derived from IgG1, and/or wherein the Fc domain comprises amino acid substitutions corresponding to L234A and L235A according to EU numbering; or (b) an albumin domain directly or indirectly fused to a C1-INH domain.

6. The composition of claim 1(ii) or claim 5, wherein:
(i) the C1-INH protein has a glycosylation profile comprising no more than 50%, 45%, 40%, 35 %, 30%, 25%, 20%, 15 %, 10%, or 5% neutral glycan species, prior to PEGylation; and/or
(ii) the C1-INH protein has a glycosylation profile comprising between 5% and 25% neutral glycan species, prior to PEGylation.

7. The composition of any one of claims 1(ii), 5, or 6, wherein:
(i) the C1-INH protein comprises, on average, at least 30%, 35%, 40%, 45%, 50%, 55%, 60%, 65%, 70%, 75%, 80%, 85%, 90%, 95%, 96%, 97%, 98%, 99%, or 100% charged glycans per molecule;
(ii) the C1-INH protein contains less than 20%, 15%, 10%, or 5% of one or more of mannose, α-galactose, NGNA, or oligomannose-type glycosylation, prior to conjugation with PSA;
(iii) prior to conjugation with PSA, the C1-INH protein has a glycosylation profile comprising one or more of the following:
between 5% and 30% neutral glycan species;
between 10% and 30% mono-sialylated glycan species;
between 30% and 50% di-sialylated glycan species;
between 15% and 35% tri-sialylated glycan species; or
between 5% and 15% tetra-sialylated glycan species;
(iv) prior to conjugation with PSA, the C1-INH protein has a glycosylation profile comprising:
no more than 30% neutral glycan species;
between 20% and 30% mono-sialylated glycan species;
between 30% and 40% di-sialylated glycan species;
between 10% and 20% tri-sialylated glycan species; and
between 5% and 10% tetra-sialylated glycan species;
(vii) the C1-INH protein comprises, on average, at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 sialylated glycan residues per molecule; and/or
(v) the C1-INH protein comprises, on average, at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 mole sialic acid per mole of protein.

8. The composition of any one of claims 1(ii), 5 6, or 7, wherein:
(i) the PSA has a molecular weight between 1 KDa and 50 KDa, between 1 KDa and 40 KDa, between 5 KDa and 40 KDa, between 1 KDa and 30 KDa, between 1 KDa and 25 KDa, between 1 KDa and 20 KDa, between 1 KDa and 15 KDa, between 1 KDa and 10 KDa, or between 1 KDa and 5 KDa;
(ii) the PSA has a molecular weight of 1 KDa, 5 KDa, 10 KDa, 15 KDa, 20 KDa, 25 KDa, 30 KDa, 35 KDa, 40 KDa, 45 KDa, or 50 KDa;
(iii) the conjugated C1-INH has a PSA/C1-INH ratio of between 1 to 25, between 1 to 20, between 1 to 15, between 1 to 10, or between 1 to 5;
(iv) the conjugated C1-INH has a half-life comparable or greater that than a plasma derived human C1-INH;
(v) the conjugated C1-INH has a half-life in the range of 100%-500% of the half-life of the plasma derived C1-INH;
(vi) the conjugated C1-INH has a half-life of at least 70, 75, 80, 85, 90, 95, 100, 105, 110, 115, 120, 125, 130, 135, 140, 145, 150, 155, 160, 165, or 170 hours;
(vii) the conjugated C1-INH has a half-life of at least 3, 4, 5, 6, 7, 8, 9, 10, 11, 12, 13, or 14 days; and/or
(viii) the conjugated C1-INH has a specific activity in the range of 50%-150% of the specific activity of plasma derived human C-INH.

9. A method of producing a conjugated C1 esterase inhibitor (C1-INH), said method comprising steps of:
(i) providing a C1-INH protein comprising at least one glycan residue; and providing a PEG moiety under conditions that permit the PEG moiety reacts with the at least one glycan residue to form an oxime linkage, thereby producing the conjugated C1-INH, wherein the at least one glycan residue is a sialic acid residue, and wherein the conjugated C1-INH has an extended *in vivo* half-life compared to unconjugated C1-INH; or
(ii) providing a C1-INH protein comprising at least one glycan residue; and providing a polysialic acid (PSA) moiety under conditions that permit the PSA moiety to react with the at least one glycan residue to form an oxime linkage, thereby producing the conjugated C1-INH,
wherein the at least one glycan residue is a sialic acid residue, and wherein the conjugated C1-INH has an extended *in vivo* half-life compared to unconjugated C1-INH.

10. The method of claim 9(i), wherein:
(i) the PEG moiety comprises PEG-CH₂-O-NH₂;
(ii) the method further comprises a step of oxidizing the at least one sialic acid residue prior to reacting with the PEG moiety, wherein the oxidizing step optionally comprises periodate oxidation, optionally wherein the periodate oxidation is carried out with a molar ratio of periodate to C1-INH at between 20:1 to 50:1, optionally wherein the molar ratio of periodate to PEG is between 2.5 to 40;
(iii) the molar ratio of PEG to C1-INH is between 25:1 and 100:1; and/or
(iv) the method further comprises a step of purifying the conjugated C1-INH, optionally wherein the purifying step comprises one or more of anion exchange, tangential flow filtration diafiltration, and dialysis.

11. The method of claim 9(ii), wherein:
(i) the method further comprises a step of oxidizing the at least one sialic acid residue prior to reacting with the PSA moiety, optionally wherein the oxidizing step comprises periodate oxidation, such as wherein the periodate oxidation is carried out with a molar ratio of periodate to C1-INH at between 20:1 to 50:1, for example wherein the molar ratio of periodate to PSA is between 2.5 to 40;
(ii) the molar ratio of PSA to C1-INH is between 25:1 and 100:1; and/or
(iii) the method further comprises a step of purifying the conjugated C1-INH, optionally wherein the purifying step comprises one or more of anion exchange, tangential flow filtration diafiltration, and dialysis.

12. A conjugated C1 esterase inhibitor (C1-INH) produced by a method of any one of claims 9-11.

13. A pharmaceutical composition comprising a conjugated C1 esterase inhibitor (C1-INH) of any one of claims 1-8 or 12, and a pharmaceutically acceptable carrier, optionally wherein the composition is liquid or lyophilized.

14. A kit comprising a pharmaceutical composition of claim 13, and
a syringe, optionally wherein:
(i) the syringe is preloaded with the pharmaceutical composition; or
(ii) the pharmaceutical composition is lyophilized and the kit further comprises a reconstitution buffer.

15. The pharmaceutical composition of claim 13 or the kit of claim 14 for use in a method of treating a complement-mediated disorder, optionally wherein the complement-mediated disorder is selected from hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis, multifocal motor neuropathy.

## Patentansprüche

1. Zusammensetzung, die einen konjugierten C1-EsteraseInhibitor (C1-INH) umfasst, umfassend:
(i) ein C1-INH-Protein, das mindestens eine Polyethylenglykol(PEG)-Einheit umfasst;
und wobei die mindestens eine PEG-Einheit über eine Oximbindung kovalent mit dem C1-INH-Protein verbunden ist, wobei die Oximbindung zwischen der PEG-Einheit und einem Glykanrest von C1-INH besteht, wobei der Glykanrest ein Sialinsäurerest ist, und wobei das konjugierte C1-INH im Vergleich zu unkonjugiertem C1-INH eine verlängerte In-vivo-Halbwertszeit aufweist; oder
(ii) ein C1-INH-Protein, das mindestens einen Glykanrest umfasst; und
mindestens eine Polysialinsäure(PSA)-Einheit,
wobei die mindestens eine Polysialinsäure(PSA)-Einheit über eine Oximbindung kovalent mit dem C1-INH-Protein verbunden ist, wobei die Oximbindung zwischen der PSA-Einheit und einem Glykanrest von C1-INH besteht, wobei der Glykanrest ein Sialinsäurerest ist, und wobei das konjugierte C1-INH im Vergleich zu unkonjugiertem C1-INH eine verlängerte *In-vivo-*Halbwertszeit aufweist.

2. Zusammensetzung nach Anspruch 1(i), wobei:
(i) das C1-INH-Protein rekombinant hergestellt wird oder aus Plasma stammt;
(ii) das C1-INH-Protein eine C1-INH-Domäne mit einer Aminosäuresequenz von SEQ ID NO:1, SEQ ID NO:2, SEQ ID NO:37 oder SEQ ID NO:38 umfasst; und/oder
(iii) das C1-INH-Protein ein Fusionsprotein ist, wobei das Fusionsprotein optional (a) eine Fc-Domäne, die direkt oder indirekt mit einer C1-INH-Domäne fusioniert ist, wie etwa wenn die Fc-Domäne von IgG1 stammt und/oder die Fc-Domäne Aminosäuresubstitutionen umfasst, die gemäß EU-Nummerierung L234A und L235A entsprechen; oder (b) eine Albumindomäne, die direkt oder indirekt mit einer C1-INH-Domäne fusioniert ist, umfasst.

3. Zusammensetzung nach Anspruch 1(i) oder Anspruch 2, wobei:
(i) das C1-INH-Protein vor der PEGylierung ein Glykosylierungsprofil aufweist, das nicht mehr als 50 %, 45 %, 40 %, 35 %, 30 %, 25 %, 20 %, 15 %, 10 % oder 5 % neutrale Glykanspezies umfasst;
(ii) das C1-INH-Protein vor der PEGylierung ein Glykosylierungsprofil aufweist, das zwischen 5 % und 25 % neutrale Glykanspezies umfasst;
(iii) das C1-INH-Protein im Durchschnitt mindestens 30 %, 35 %, 40 %, 45 %, 50 %, 55 %, 60 %, 65 %, 70 %, 75 %, 80 %, 85 %, 90 %, 95 %, 96 %, 97 %, 98 %, 99 % oder 100 % geladene Glykane pro Molekül umfasst;
(iv) das C1-INH-Protein vor der PEGylierung weniger als 20 %, 15 %, 10 % oder 5 % einer oder mehrerer Glykosylierungen vom Mannose-, α-Galactose-, NGNA- oder Oligomannose-Typ enthält;
(v) das C1-INH-Protein vor der PEGylierung ein Glykosylierungsprofil aufweist, das eines oder mehrere von Folgendem umfasst:
zwischen 5 % und 30 % neutrale Glykanspezies;
zwischen 10 % und 30 % monosialylierte Glykanspezies;
zwischen 30 % und 50 % disialylierte Glykanspezies;
zwischen 15 % und 35 % trisialylierte Glykanspezies; oder
zwischen 5 % und 15 % tetrasialylierte Glykanspezies;
(vi) das C1-INH-Protein vor der PEGylierung ein Glykosylierungsprofil aufweist, das Folgendes umfasst:
nicht mehr als 30 % neutrale Glykanspezies;
zwischen 20 % und 30 % monosialylierte Glykanspezies;
zwischen 30 % und 40 % disialylierte Glykanspezies;
zwischen 10 % und 20 % trisialylierte Glykanspezies; und
zwischen 5 % und 10 % tetrasialylierte Glykanspezies;
(vii) das C1-INH-Protein im Durchschnitt mindestens 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 oder 40 sialylierte Glykanreste pro Molekül umfasst; und/oder
(viii) das C1-INH-Protein im Durchschnitt mindestens 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 oder 40 Mol Sialinsäure pro Mol Protein umfasst.

4. Zusammensetzung nach einem der Ansprüche 1(i), 2 oder 3, wobei:
(i) das PEG ein Molekulargewicht zwischen 1 kDa und 50 kDa, zwischen 1 kDa und 40 kDa, zwischen 5 kDa und 40 kDa, zwischen 1 kDa und 30 kDa, zwischen 1 kDa und 25 kDa, zwischen 1 kDa und 20 kDa, zwischen 1 kDa und 15 kDa, zwischen 1 kDa und 10 kDa oder zwischen 1 kDa und 5 kDa aufweist;
(ii) das PEG ein Molekulargewicht von 1 kDa, 5 kDa, 10 kDa, 15 kDa, 20 kDa, 25 kDa, 30 kDa, 35 kDa, 40 kDa, 45 kDa oder 50 kDa aufweist;
(iii) das konjugierte C1-INH ein PEG/C1-INH-Verhältnis zwischen 1 und 25, zwischen 1 und 20, zwischen 1 und 15, zwischen 1 und 10 oder zwischen 1 und 5 aufweist;
(iv) das konjugierte C1-INH eine Halbwertszeit aufweist, die mit der eines aus Plasma stammenden humanen C1-INH vergleichbar oder größer als eine solche ist;
(v) das konjugierte C1-INH eine Halbwertszeit im Bereich von 100-500 % der Halbwertszeit des aus Plasma stammenden C1-INH aufweist;
(vi) das konjugierte C1-INH eine Halbwertszeit von mindestens 70, 75, 80, 85, 90, 95, 100, 105, 110, 115, 120, 125, 130, 135, 140, 145, 150, 155, 160, 165 oder 170 Stunden aufweist;
(vii) das konjugierte C1-INH eine Halbwertszeit von mindestens 3, 4, 5, 6, 7, 8, 9, 10, 11, 12, 13 oder 14 Tagen aufweist; und/oder
(viii) das konjugierte C1-INH eine spezifische Aktivität im Bereich von 50-150 % der spezifischen Aktivität von aus Plasma stammendem humanem C1-INH aufweist.

5. Zusammensetzung nach Anspruch 1(ii), wobei:
(i) das C1-INH-Protein rekombinant hergestellt wird oder aus Plasma stammt;
(ii) das C1-INH-Protein eine C1-INH-Domäne mit einer Aminosäuresequenz von SEQ ID NO:1, SEQ ID NO:2, SEQ ID NO:37 oder SEQ ID NO:38 umfasst; und/oder
(iv) das C1-INH-Protein ein Fusionsprotein ist, wobei das Fusionsprotein optional (a) eine Fc-Domäne, die direkt oder indirekt mit einer C1-INH-Domäne fusioniert ist, wie etwa wenn die Fc-Domäne von IgG1 stammt, und/oder die Fc-Domäne Aminosäuresubstitutionen umfasst, die gemäß EU-Nummerierung L234A und L235A entsprechen; oder (b) eine Albumindomäne, die direkt oder indirekt mit einer C1-INH-Domäne fusioniert ist, umfasst.

6. Zusammensetzung nach Anspruch 1(ii) oder Anspruch 5, wobei:
(i) das C1-INH-Protein vor der PEGylierung ein Glykosylierungsprofil aufweist, das nicht mehr als 50 %, 45 %, 40 %, 35 %, 30 %, 25 %, 20 %, 15 %, 10 % oder 5 % neutrale Glykanspezies umfasst; und/oder
(ii) das C1-INH-Protein vor der PEGylierung ein Glykosylierungsprofil aufweist, das zwischen 5 % und 25 % neutrale Glykanspezies umfasst.

7. Zusammensetzung nach einem der Ansprüche 1(ii), 5 oder 6, wobei:
(i) das C1-INH-Protein im Durchschnitt mindestens 30 %, 35 %, 40 %, 45 %, 50 %, 55 %, 60 %, 65 %, 70 %, 75 %, 80 %, 85 %, 90 %, 95 %, 96 %, 97 %, 98 %, 99 % oder 100 % geladene Glykane pro Molekül umfasst;
(ii) das C1-INH-Protein vor der Konjugation mit PSA weniger als 20 %, 15 %, 10 % oder 5 % einer oder mehrerer Glykosylierungen vom Mannose-, α-Galactose-, NGNA- oder Oligomannose-Typ enthält;
(iii) das C1-INH-Protein vor der Konjugation mit PSA ein Glykosylierungsprofil aufweist, das eines oder mehrere von Folgendem umfasst:
zwischen 5 % und 30 % neutrale Glykanspezies;
zwischen 10 % und 30 % monosialylierte Glykanspezies;
zwischen 30 % und 50 % disialylierte Glykanspezies;
zwischen 15 % und 35 % trisialylierte Glykanspezies; oder
zwischen 5 % und 15 % tetrasialylierte Glykanspezies;
(iv) das C1-INH-Protein vor der Konjugation mit PSA ein Glykosylierungsprofil aufweist, das Folgendes umfasst:
nicht mehr als 30 % neutrale Glykanspezies;
zwischen 20 % und 30 % monosialylierte Glykanspezies;
zwischen 30 % und 40 % disialylierte Glykanspezies;
zwischen 10 % und 20 % trisialylierte Glykanspezies; und
zwischen 5 % und 10 % tetrasialylierte Glykanspezies;
(vii) das C1-INH-Protein im Durchschnitt mindestens 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 oder 40 sialylierte Glykanreste pro Molekül umfasst; und/oder
(v) das C1-INH-Protein im Durchschnitt mindestens 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 oder 40 Mol Sialinsäure pro Mol Protein umfasst.

8. Zusammensetzung nach einem der Ansprüche 1(ii), 5, 6 oder 7, wobei:
(i) das PSA ein Molekulargewicht zwischen 1 kDa und 50 kDa, zwischen 1 kDa und 40 kDa, zwischen 5 kDa und 40 kDa, zwischen 1 kDa und 30 kDa, zwischen 1 kDa und 25 kDa, zwischen 1 kDa und 20 kDa, zwischen 1 kDa und 15 kDa, zwischen 1 kDa und 10 kDa oder zwischen 1 kDa und 5 kDa aufweist;
(ii) das PSA ein Molekulargewicht von 1 kDa, 5 kDa, 10 kDa, 15 kDa, 20 kDa, 25 kDa, 30 kDa, 35 kDa, 40 kDa, 45 kDa oder 50 kDa aufweist;
(iii) das konjugierte C1-INH ein PSA/C1-INH-Verhältnis zwischen 1 und 25, zwischen 1 und 20, zwischen 1 und 15, zwischen 1 und 10 oder zwischen 1 und 5 aufweist;
(iv) das konjugierte C1-INH eine Halbwertszeit aufweist, die mit der eines aus Plasma stammenden humanen C1-INH vergleichbar oder größer als eine solche ist;
(v) das konjugierte C1-INH eine Halbwertszeit im Bereich von 100-500 % der Halbwertszeit des aus Plasma stammenden C1-INH aufweist;
(vi) das konjugierte C1-INH eine Halbwertszeit von mindestens 70, 75, 80, 85, 90, 95, 100, 105, 110, 115, 120, 125, 130, 135, 140, 145, 150, 155, 160, 165 oder 170 Stunden aufweist;
(vii) das konjugierte C1-INH eine Halbwertszeit von mindestens 3, 4, 5, 6, 7, 8, 9, 10, 11, 12, 13 oder 14 Tagen aufweist; und/oder
(viii) das konjugierte C1-INH eine spezifische Aktivität im Bereich von 50-150 % der spezifischen Aktivität von aus Plasma stammendem humanem C-INH aufweist.

9. Verfahren zum Herstellen eines konjugierten C1-Esterase-Inhibitors (C1-INH), wobei das Verfahren die folgenden Schritte umfasst:
(i) Bereitstellen eines C1-INH-Proteins, das mindestens einen Glykanrest umfasst; und
Bereitstellen einer PEG-Einheit unter Bedingungen, die es der PEG-Einheit ermöglichen, mit dem mindestens einen Glykanrest zu reagieren, um eine Oximbindung zu bilden, wodurch das konjugierte C1-INH hergestellt wird, wobei der mindestens eine Glykanrest ein Sialinsäurerest ist und wobei das konjugierte C1-INH hat im Vergleich zu unkonjugiertem C1-INH eine verlängerte In-vivo-Halbwertszeit aufweist; oder
(ii) Bereitstellen eines C1-INH-Proteins, das mindestens einen Glykanrest umfasst; und
Bereitstellen einer Polysialinsäure(PSA)-Einheit unter Bedingungen, die es der PSA-Einheit ermöglichen, mit dem mindestens einen Glykanrest zu reagieren, um eine Oximbindung zu bilden, wodurch das konjugierte C1-INH hergestellt wird,
wobei der mindestens eine Glykanrest ein Sialinsäurerest ist und wobei das konjugierte C1-INH im Vergleich zu unkonjugiertem C1-INH eine verlängerte In-vivo-Halbwertszeit aufweist.

10. Verfahren nach Anspruch 9(i), wobei:
(i) die PEG-Einheit PEG-CH₂-O-NH₂ umfasst;
(ii) das Verfahren ferner einen Schritt des Oxidierens des mindestens einen Sialinsäurerests vor der Reaktion mit der PEG-Einheit umfasst, wobei der Oxidationsschritt optional eine Periodatoxidation umfasst, wobei die Periodatoxidation optional mit einem Molverhältnis von Periodat zu C1-INH zwischen 20:1 und 50:1 durchgeführt wird, wobei das Molverhältnis von Periodat zu PEG optional zwischen 2,5 und 40 liegt;
(iii) das Molverhältnis von PEG zu C1-INH zwischen 25:1 und 100:1 liegt; und/oder
(iv) das Verfahren ferner einen Schritt des Reinigens des konjugierten C1-INH umfasst, wobei der Reinigungsschritt optional eines oder mehrere von Anionenaustausch, Tangentialflussfiltration, Diafiltration und Dialyse umfasst.

11. Verfahren nach Anspruch 9(ii), wobei:
(i) das Verfahren ferner einen Schritt des Oxidierens des mindestens einen Sialinsäurerests vor dem Umsetzen mit der PSA-Einheit umfasst, wobei der Oxidationsschritt optional eine Periodatoxidation umfasst, wie etwa wobei die Periodatoxidation mit einem Molverhältnis von Periodat zu C1-INH zwischen 20:1 und 50:1 durchgeführt wird, wobei beispielsweise das Molverhältnis von Periodat zu PSA zwischen 2,5 und 40 liegt;
(ii) das Molverhältnis von PSA zu C1-INH zwischen 25:1 und 100:1 liegt; und/oder
(iii) das Verfahren ferner einen Schritt des Reinigens des konjugierten C1-INH umfasst, wobei der Reinigungsschritt optional einen oder mehrere von Anionenaustausch, Tangentialflussfiltration, Diafiltration und Dialyse umfasst.

12. Konjugierter C1-Esterase-Inhibitor (C1-INH), hergestellt durch ein Verfahren nach einem der Ansprüche 9-11.

13. Pharmazeutische Zusammensetzung, umfassend einen konjugierten C1-Esterase-Inhibitor (C1-INH) nach einem der Ansprüche 1-8 oder 12 und einen pharmazeutisch verträglichen Träger, wobei die Zusammensetzung optional flüssig oder lyophilisiert ist.

14. Kit, umfassend eine pharmazeutische Zusammensetzung nach Anspruch 13 und
eine Spritze, wobei optional:
(i) die Spritze mit der pharmazeutischen Zusammensetzung vorgefüllt ist; oder
(ii) die pharmazeutische Zusammensetzung lyophilisiert ist und das Kit ferner einen Rekonstitutionspuffer umfasst.

15. Pharmazeutische Zusammensetzung nach Anspruch 13 oder Kit nach Anspruch 14 zur Verwendung in einem Verfahren zum Behandeln einer komplementvermittelten Störung, wobei die komplementvermittelte Störung optional aus hereditärem Angioödem, antikörpervermittelter Abstoßung, Neuromyelitis-Optica-Spektrum-Störungen, traumatischer Hirnverletzung, Rückenmarksverletzung, ischämischer Hirnverletzung, Brandverletzung, toxischer epidermaler Nekrolyse, multipler Sklerose, amyotropher Lateralsklerose (ALS), Parkinson-Krankheit, Schlaganfall, chronisch entzündlicher demyelinisierender Polyneuropathie (CIDP), Myasthenia gravis, multifokaler motorischer Neuropathie ausgewählt ist.

## Revendications

1. Composition comprenant un inhibiteur conjugué de C1 estérase (C1-INH) comprenant :
(i) une protéine C1-INH comprenant au moins un groupement de polyéthylène glycol (PEG) ; et dans laquelle l'au moins un groupement de PEG est lié de manière covalente à la protéine C1-INH par l'intermédiaire d'une liaison oxime, dans laquelle la liaison oxime se situe entre le groupement de PEG et un résidu de glycane de C1-INH, dans laquelle le résidu de glycane est un résidu d'acide sialique, et dans laquelle le C1-INH conjugué a une demi-vie *in vivo* prolongée par rapport au C1-INH non conjugué ; ou
(ii) une protéine C1-INH comprenant au moins un résidu de glycane ; et
au moins un groupement de poly(acide sialique) (PSA),
dans laquelle l'au moins un groupement de poly(acide sialique) (PSA) est lié de manière covalente à la protéine C1-INH par l'intermédiaire d'une liaison oxime, dans laquelle la liaison oxime se situe entre le groupement de PSA et un résidu de glycane de C1-INH, dans laquelle le résidu de glycane est un résidu d'acide sialique, et dans laquelle le C1-INH conjugué a une demi-vie *in vivo* prolongée par rapport au C1-INH non conjugué.

2. Composition selon la revendication 1(i), dans laquelle :
(i) la protéine C1-INH est produite par recombinaison ou dérivée du plasma ;
(ii) la protéine C1-INH comprend un domaine de C1-INH ayant une séquence d'acides aminés de SEQ ID NO : 1, SEQ ID NO : 2, SEQ ID NO : 37 ou SEQ ID NO : 38 ; et/ou
(iii) la protéine C1-INH est une protéine de fusion, dans laquelle la protéine de fusion comprend éventuellement (a) un domaine de Fc fusionné directement ou indirectement à un domaine de C1-INH, par exemple dans laquelle le domaine de Fc est dérivé d'IgG1 et/ou le domaine de Fc comprend des substitutions d'acide aminé correspondant à L234A et L235A selon la numérotation EU ; ou (b) un domaine d'albumine fusionné directement ou indirectement à un domaine de C1-INH.

3. Composition selon la revendication 1(i) ou la revendication 2, dans laquelle :
(i) la protéine C1-INH a un profil de glycosylation ne comprenant pas plus de 50 %, 45 %, 40 %, 35 %, 30 %, 25 %, 20 %, 15 %, 10 % ou 5 % d'espèces de glycanes neutres, avant PEGylation ;
(ii) la protéine C1-INH a un profil de glycosylation comprenant entre 5 % et 25 % d'espèces de glycanes neutres, avant PEGylation ;
(iii) la protéine C1-INH comprend, en moyenne, au moins 30 %, 35 %, 40 %, 45 %, 50 %, 55 %, 60 %, 65 %, 70 %, 75 %, 80 %, 85 %, 90 %, 95 %, 96 %, 97 %, 98 %, 99 % ou 100 % de glycanes chargés par molécule ;
(iv) la protéine C1-INH contient moins de 20 %, 15 %, 10 % ou 5 % d'un ou plusieurs parmi le mannose, l'α-galactose, le NGNA ou une glycosylation de type oligomannose, avant PEGylation ;
(v) avant PEGylation, la protéine C1-INH a un profil de glycosylation comprenant un ou plusieurs de ce qui suit :
entre 5 % et 30 % d'espèces de glycanes neutres ;
entre 10 % et 30 % d'espèces de glycanes monosialylés ;
entre 30 % et 50 % d'espèces de glycanes di-sialylés ;
entre 15 % et 35 % d'espèces de glycanes trisialylés ; ou
entre 5 % et 15 % d'espèces de glycanes tétra-sialylés ;
(vi) avant PEGylation, la protéine C1-INH a un profil de glycosylation comprenant :
pas plus de 30 % d'espèces de glycanes neutres ;
entre 20 % et 30 % d'espèces de glycanes monosialylés ;
entre 30 % et 40 % d'espèces de glycanes di-sialylés ;
entre 10 % et 20 % d'espèces de glycanes trisialylés ; et
entre 5 % et 10 % d'espèces de glycanes tétra-sialylés ;
(vii) la protéine C1-INH comprend, en moyenne, au moins 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 ou 40 résidus de glycanes sialylés par molécule ; et/ou
(viii) la protéine C1-INH comprend, en moyenne, au moins 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 ou 40 moles d'acide sialique par mole de protéine.

4. Composition selon l'une quelconque des revendications 1(i), 2 ou 3, dans laquelle :
(i) le PEG a un poids moléculaire compris entre 1 KDa et 50 KDa, entre 1 KDa et 40 KDa, entre 5 KDa et 40 KDa, entre 1 KDa et 30 KDa, entre 1 KDa et 25 KDa, entre 1 KDa et 20 KDa, entre 1 KDa et 15 KDa, entre 1 KDa et 10 KDa, ou entre 1 KDa et 5 KDa ;
(ii) le PEG a un poids moléculaire de 1 KDa, 5 KDa, 10 KDa, 15 KDa, 20 KDa, 25 KDa, 30 KDa, 35 KDa, 40 KDa, 45 KDa ou 50 KDa ;
(iii)le C1-INH conjugué a un rapport PEG/C1-INH compris entre 1 et 25, entre 1 et 20, entre 1 et 15, entre 1 et 10 ou entre 1 et 5 ;
(iv) le C1-INH conjugué a une demi-vie comparable ou supérieure à celle d'un C1-INH humain dérivé du plasma ;
(v) le C1-INH conjugué a une demi-vie dans la plage de 100 % à 500 % de la demi-vie du C1-INH dérivé du plasma ;
(vi) le C1-INH conjugué a une demi-vie d'au moins 70, 75, 80, 85, 90, 95, 100, 105, 110, 115, 120, 125, 130, 135, 140, 145, 150, 155, 160, 165 ou 170 heures ;
(vii) le C1-INH conjugué a une demi-vie d'au moins 3, 4, 5, 6, 7, 8, 9, 10, 11, 12, 13 ou 14 jours ; et/ou
(viii) le C1-INH conjugué a une activité spécifique dans la plage de 50 % à 150 % de l'activité spécifique du C1-INH humain dérivé du plasma.

5. Composition selon la revendication 1(ii), dans laquelle :
(i) la protéine C1-INH est produite par recombinaison ou dérivée du plasma ;
(ii) la protéine C1-INH comprend un domaine de C1-INH ayant une séquence d'acides aminés de SEQ ID NO : 1, SEQ ID NO : 2, SEQ ID NO : 37 ou SEQ ID NO : 38 ; et/ou
(iv) la protéine C1-INH est une protéine de fusion, éventuellement dans laquelle la protéine de fusion comprend (a) un domaine de Fc fusionné directement ou indirectement à un domaine de C1-INH, par exemple dans laquelle le domaine de Fc est dérivé d'IgG1, et/ou dans laquelle le domaine de Fc comprend des substitutions d'acide aminé correspondant à L234A et L235A selon la numérotation EU ; ou (b) un domaine d'albumine fusionné directement ou indirectement à un domaine de C1-INH.

6. Composition selon la revendication 1(ii) ou la revendication 5, dans laquelle :
(i) la protéine C1-INH a un profil de glycosylation ne comprenant pas plus de 50 %, 45 %, 40 %, 35 %, 30 %, 25 %, 20 %, 15 %, 10 % ou 5 % d'espèces de glycanes neutres, avant PEGylation ; et/ou
(ii) la protéine C1-INH a un profil de glycosylation comprenant entre 5 % et 25 % d'espèces de glycanes neutres, avant PEGylation.

7. Composition selon l'une quelconque des revendications 1(ii), 5 ou 6, dans laquelle :
(i) la protéine C1-INH comprend, en moyenne, au moins 30 %, 35 %, 40 %, 45 %, 50 %, 55 %, 60 %, 65 %, 70 %, 75 %, 80 %, 85 %, 90 %, 95 %, 96 %, 97 %, 98 %, 99 % ou 100 % de glycanes chargés par molécule ;
(ii) la protéine C1-INH contient moins de 20 %, 15 %, 10 % ou 5 % d'un ou plusieurs parmi le mannose, l'α-galactose, le NGNA ou une glycosylation de type oligomannose, avant conjugaison avec le PSA ;
(iii) avant conjugaison avec le PSA, la protéine C1-INH a un profil de glycosylation comprenant un ou plusieurs de ce qui suit :
entre 5 % et 30 % d'espèces de glycanes neutres ;
entre 10 % et 30 % d'espèces de glycanes monosialylés ;
entre 30 % et 50 % d'espèces de glycanes di-sialylés ;
entre 15 % et 35 % d'espèces de glycanes trisialylés ; ou
entre 5 % et 15 % d'espèces de glycanes tétra-sialylés ;
(iv) avant conjugaison avec le PSA, la protéine C1-INH a un profil de glycosylation comprenant :
pas plus de 30 % d'espèces de glycanes neutres ;
entre 20 % et 30 % d'espèces de glycanes monosialylés ;
entre 30 % et 40 % d'espèces de glycanes di-sialylés ;
entre 10 % et 20 % d'espèces de glycanes trisialylés ; et
entre 5 % et 10 % d'espèces de glycanes tétra-sialylés ;
(vii) la protéine C1-INH comprend, en moyenne, au moins 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 ou 40 résidus de glycanes sialylés par molécule ; et/ou
(v) la protéine C1-INH comprend, en moyenne, au moins 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 ou 40 moles d'acide sialique par mole de protéine.

8. Composition selon l'une quelconque des revendications 1(ii), 5, 6 ou 7, dans laquelle :
(i) le PSA a un poids moléculaire compris entre 1 KDa et 50 KDa, entre 1 KDa et 40 KDa, entre 5 KDa et 40 KDa, entre 1 KDa et 30 KDa, entre 1 KDa et 25 KDa, entre 1 KDa et 20 KDa, entre 1 KDa et 15 KDa, entre 1 KDa et 10 KDa, ou entre 1 KDa et 5 KDa ;
(ii) le PSA a un poids moléculaire de 1 KDa, 5 KDa, 10 KDa, 15 KDa, 20 KDa, 25 KDa, 30 KDa, 35 KDa, 40 KDa, 45 KDa ou 50 KDa ;
(iii)le C1-INH conjugué a un rapport PSA/C1-INH compris entre 1 et 25, entre 1 et 20, entre 1 et 15, entre 1 et 10 ou entre 1 et 5 ;
(iv) le C1-INH conjugué a une demi-vie comparable ou supérieure à celle d'un C1-INH humain dérivé du plasma ;
(v) le C1-INH conjugué a une demi-vie dans la plage de 100 % à 500 % de la demi-vie du C1-INH dérivé du plasma ;
(vi) le C1-INH conjugué a une demi-vie d'au moins 70, 75, 80, 85, 90, 95, 100, 105, 110, 115, 120, 125, 130, 135, 140, 145, 150, 155, 160, 165 ou 170 heures ;
(vii) le C1-INH conjugué a une demi-vie d'au moins 3, 4, 5, 6, 7, 8, 9, 10, 11, 12, 13 ou 14 jours ; et/ou
(viii) le C1-INH conjugué a une activité spécifique dans la plage de 50 % à 150 % de l'activité spécifique du C-INH humain dérivé du plasma.

9. Procédé de production d'un inhibiteur conjugué de C1 estérase (C1-INH), ledit procédé comprenant les étapes de :
(i) fourniture d'une protéine C1-INH comprenant au moins un résidu de glycane ; et fourniture d'un groupement de PEG dans des conditions qui permettent au groupement de PEG de réagir avec l'au moins un résidu de glycane pour former une liaison oxime, produisant ainsi le C1-INH conjugué, dans lequel l'au moins un résidu de glycane est un résidu d'acide sialique, et dans lequel le C1-INH conjugué a une demi-vie *in vivo* prolongée par rapport au C1-INH non conjugué ; ou
(ii) fourniture d'une protéine C1-INH comprenant au moins un résidu de glycane ; et
fourniture d'un groupement de poly(acide sialique) (PSA) dans des conditions qui permettent au groupement de PSA de réagir avec l'au moins un résidu de glycane pour former une liaison oxime, produisant ainsi le C1-INH conjugué,
dans lequel l'au moins un résidu de glycane est un résidu d'acide sialique, et dans lequel le C1-INH conjugué a une demi-vie *in vivo* prolongée par rapport au C1-INH non conjugué.

10. Procédé selon la revendication 9(i), dans lequel :
(i) le groupement de PEG comprend PEG-CH₂-O-NH₂ ;
(ii) le procédé comprend en outre une étape d'oxydation de l'au moins un résidu d'acide sialique avant de réagir avec le groupement de PEG, dans lequel l'étape d'oxydation comprend éventuellement une oxydation au periodate, éventuellement dans lequel l'oxydation au periodate est effectuée avec un rapport molaire du periodate au C1-INH compris entre 20:1 et 50:1, éventuellement dans lequel le rapport molaire du periodate au PEG est compris entre 2,5 et 40 ;
(iii)le rapport molaire du PEG au C1-INH est compris entre 25:1 et 100:1 ; et/ou
(iv) le procédé comprend en outre une étape de purification du C1-INH conjugué, éventuellement dans lequel l'étape de purification comprend un ou plusieurs parmi un échange d'anions, une diafiltration par filtration à flux tangentiel et une dialyse.

11. Procédé selon la revendication 9(ii), dans lequel :
(i) le procédé comprend en outre une étape d'oxydation de l'au moins un résidu d'acide sialique avant de réagir avec le groupement de PSA, éventuellement dans lequel l'étape d'oxydation comprend une oxydation au periodate, par exemple dans lequel l'oxydation au periodate est effectuée avec un rapport molaire du periodate au C1-INH compris entre 20:1 et 50:1, par exemple dans lequel le rapport molaire du periodate au PSA est compris entre 2,5 et 40 ;
(ii) le rapport molaire du PSA au C1-INH est compris entre 25:1 et 100:1 ; et/ou
(iii)le procédé comprend en outre une étape de purification du C1-INH conjugué, éventuellement dans lequel l'étape de purification comprend un ou plusieurs parmi un échange d'anions, une diafiltration par filtration à flux tangentiel et une dialyse.

12. Inhibiteur conjugué de C1 estérase (C1-INH) produit par un procédé selon l'une quelconque des revendications 9 à 11.

13. Composition pharmaceutique comprenant un inhibiteur conjugué de C1 estérase (C1-INH) selon l'une quelconque des revendications 1 à 8 ou 12, et un support pharmaceutiquement acceptable, éventuellement dans laquelle la composition est liquide ou lyophilisée.

14. Kit comprenant une composition pharmaceutique selon la revendication 13, et
une seringue, éventuellement dans lequel :
(i) la seringue est préremplie de la composition pharmaceutique ; ou
(ii) la composition pharmaceutique est lyophilisée et le kit comprend en outre un tampon de reconstitution.

15. Composition pharmaceutique selon la revendication 13 ou kit selon la revendication 14 pour une utilisation dans un procédé de traitement d'un trouble médié par le complément, éventuellement dans laquelle le trouble médié par le complément est choisi parmi l'angio-œdème héréditaire, le rejet médié par des anticorps, les troubles du spectre de la neuromyélite optique, une lésion cérébrale traumatique, une lésion de la moelle épinière, une lésion cérébrale ischémique, une brûlure, la nécrolyse épidermique toxique, la sclérose en plaques, la sclérose latérale amyotrophique (SLA), la maladie de Parkinson, un accident vasculaire cérébral, la polyneuropathie démyélinisante inflammatoire chronique (PDIC), la myasthénie grave, la neuropathie motrice multifocale.
